# EUROPEAN PATENT SPECIFICATION

(11) **EP 3 362 436 B1**
(45) Date of publication and mention of the grant of the patent: **30.06.2021**
(21) Application number: 16775090.0
(22) Date of filing: 14.09.2016
(51) Int. Cl.: C07D 213/80, C07C 67/20, C07D 213/81, C07D 213/90

(54) **PROCESS FOR THE CATALYTIC DIRECTED CLEAVAGE OF AMIDE-CONTAINING COMPOUNDS**
VERFAHREN ZUR KATALYTISCHEN GERICHTETEN SPALTUNG VON AMIDHALTIGEN VERBINDUNGEN
PROCÉDÉ POUR LE CLIVAGE CATALYTIQUE DIRIGÉ DE COMPOSÉS CONTENANT UN AMIDE

(30) Priority: 14.09.2015 EP 15185070
(43) Date of publication of application: 22.08.2018
(73) Proprietor: Universiteit Antwerpen, 2000 Antwerpen (BE)
(72) Inventor: MAES, Bert, 2610 Wilrijk (BE); WYBON, Clarence, 8650 Houthulst (BE); CHEN, Chen, 2020 Antwerpen (BE); BHEETER, Charles Beromeo, 1090 GS Amsterdam (NL); SERGUEEV, Serguei, 1140 Evere (BE)
(74) Representative: Arnold & Siedsma
(86) International application number: PCT/EP2016/071650
(87) International publication number: WO 2017/046133

(56) References cited:
- EP-A1- 1 314 712
- US-A1- 2009 156 646
- XUE CUIHUA ET AL: "TRANSFORMATION OF AMIDES INTO ESTERS BY THE USE OF CHLOROTRIMETHYLSILANE", JOURNAL OF THE CHINESE CHEMICAL SOCIETY, vol. 51, no. 2, 1 January 2004 (2004-01-01), pages 359-362, XP008077858, CHINESE ELECTRONIC PERIODICAL SERVICES, CHINA ISSN: 0009-4536 cited in the application
- KOI ARAKI ET AL: "Preferential formation of amino aicd esters in aquueous alcohol solutions: solvolysis of 6,6'-Bis(aminoacylamino)-2,2'-bipyridine by metal coordination", JOURNAL OF THE CHEMICAL SOCIETY, CHEMICAL COMMUNICATIONS, 1992, XP002764369, GBCHEMICAL SOCIETY. LETCHWORTH. ISSN: 0300-922X
- LIANA HIE ET AL: "Conversion of amides to esters by the nickel-catalysed activation of amides C-N bonds.", NATURE, vol. 524, 6 August 2015 (2015-08-06), pages 79-83, XP002754615, Nature Publishing Group ISSN: 0028-0836, DOI: 10.1038/nature14615
- COBO J ET AL: "Reactivity of 6-aminopyrimidin-4-(3H)-ones towards dimethyl acetylenedicarboxylate (DMAD). Tandem Diels-Alder/retro Diels-Alder (DA/RDA) reaction in the synthesis of 2-aminopyridines", TETRAHEDRON, vol. 50, no. 34, 1 January 1994 (1994-01-01), pages 10345-10358, XP001155939, ELSEVIER SCIENCE PUBLISHERS, AMSTERDAM, NL ISSN: 0040-4020, DOI: 10.1016/S0040-4020(01)81767-1
- MARIA L. PELLIZZARO ET AL: "Design synthesis and binding studies of a novel quadruple ADDA hydrogen-bonf array", ORGANIC AND BIOMOLECULAR CHEMISTRY, vol. 10, no. 25, 2012, pages 4899-4906, XP002754614,
- SARAH M. CRAWFORD ET AL: "BippyPhos: a single ligand with unprecedented scope in the Buchwald-Hartwig amination of (hetero)aryl chlorides", CHEMISTRY - A EUROPEAN JOURNAL., vol. 19, 2013, pages 16760-16771, XP002754616, USVCH PUBLISHERS. ISSN: 0947-6539
- Y. KITA ET AL: "Zinc-catalyzed amide cleavage and esterficiation of beta-hydroxyethylamides", ANGEWANDTE CHEMIE. INTERNATIONAL EDITION., vol. 51, 2012, pages 5723-5726, XP002754617, DEVCH VERLAG, WEINHEIM. ISSN: 0570-0833 cited in the application

## Description

### FIELD OF THE APPLICATION

The present application relates to a catalytic method for the conversion of amide-containing compouds by means of a build-in directing group and upon the action of a heteronucleophilic compound *(in se* an amine (RNH₂ or RNHR') or an alcohol (ROH) or a thiol (RSH)) in the presence of a metal catalyst to respectively esters, thioesters, carbonates, thiocarbonates and to what is defined as amide-containing compounds (such as carboxamides, urea, carbamates, thiocarbamates). The present application also relates to these amide-containing compounds having a build-in directing group (DG), as well as the use of such directing groups in the catalytic directed cleavage of *N*-DG amides upon the action of heteronucleophiles *(in se* an amine (RNH₂ or RNHR') or an alcohol (ROH) or thiol (RSH)).

### BACKGROUND TO THE APPLICATION

The -NH-CO- structural moiety is ubiquitous in natural products, agrochemicals, industrial polymers and modern pharmaceuticals. Amides for example are crucial to sustain life, since these entities make up the peptide bonds in vital proteins such as receptors and enzymes, whereas (poly)urea and (poly)carbamates have numerous industrial applications. Without doubt, this functional -NH-CO- entity can be considered as one of the most important functional groups in contemporary organic chemistry. Although this synthon appears widely in nature and organic chemistry, transition metal-catalysed transformations of this functional group by attack of heteronucleophiles to the carbonyl moiety, under non-stringent conditions and with the use of non-toxic reagents, while minimizing waste, remain largely unstudied, whereas these reactions have great potential in the synthesis of many industrially interesting compound classes, such as amides, esters, thioesters, urea, carbamates, thiocarbamates, carbonates or thiocarbonates. Indeed, in view of the prevalence of this functional group, it is remarkable that the further synthetic transformation of this omnipresent structural entity is less researched than the formation process *an sich.* Whereas for example amides are (relatively) easy formed in the biological metabolic cycle, the cleavage (hydrolysis) of amide bonds in nature is ATP-dependent and requires enzymes (peptidases). The half-life time of 350-600 years for spontaneous hydrolysis at neutral pH and room temperature clearly indicates this. The reason for this can be found in the chemical inertness of the -NH-CO- structural entity. The chemical transformation of -NH-CO- groups by nucleophilic attack of alcohols or thiols, is thermodynamically unfavorable. The fact that this reaction is significantly thermodynamically up-hill can be explained by the high stability - and thus low reactivity - of the carbonyl of -NH-CO- groups for nucleophilic attack, due to the low electronegativity of an amide nitrogen compared to oxygen in an ester or carboxylic acid. For transamidation (attack of an amine), the stability of substrate and product amide is generally not sufficiently different (thermodynamically neutral reaction). Therefore, performing such reactions is particularly challenging. Indeed, the need for extreme conditions using for example high temperature or toxic, wasteful and expensive activation strategies, has limited the role of such transformations in industrial processes up to now. Nowadays, the cleavage of a wide variance of -NH-CO- moieties under fairly mild conditions and using a single generally applicable protocol, is still not an accessible transformation in classic organic chemistry, whereas this synthetic transformation obviously has a huge potential.

As mentioned, some strategies to circumvent this problem have been developed. Reported approaches for single -NH-CO- cleavage with the use of alcohols or thiols as nucleophiles however typically use stoichiometric activation reagents for carbonyl,¹ leaving group activation,^{2,3} or (super)stoichiometric (Lewis) acid/base.⁴⁻⁶ In addition to the necessity of using stoichiometric amounts of reagents, a pre-activation strategy (leaving group activation) mostly requires dangerous and expensive reagents that suffer from limited functional group compatibility and thus limit the scope of the heteronucleophile or the amide substrate. More specific techniques use intramolecular activation strategies via *N*-O-,⁷ or *N*-S-,⁸ acyl transfer or twisted amides for minimising amide resonance,⁹ in particular. To the best of our knowledge, no generally applicable transition metal-catalysed amide cleavage method under fairly mild, non-air sensitive and neutral conditions which allows the use of both a huge variance of primary amide-containing compounds, such as peptides, urea, aromatic and aliphatic primary amides and carbamates and a huge variance of heteronucleophiles (amine, thiols and alcohols) in non-solvolytic amounts, while also including complex nucleophiles, such as sugars, sterols and amino acid-derived nucleophiles, has been published. The Mashima group recently reported a combined catalytic system of Sc(OTf)₂ and a boronic ester for catalytic amide bond cleavage.¹⁰ However, only solvolytic cleavage with the use of EtOH or PrOH under reflux conditions, was possible, and limited scope of amide-contaning compounds was shown. Subsequently, Atkinson reported the Sc(OTf)₂-catalysed cleavage of primary amides with the use of stoichiometric amounts of primary alcohols at 100 °C or secondary alcohols at 125 °C.¹¹ In other work, CeO₂ was described as the first reusable heterogenous catalytic system for non-solvolytic amide cleavage with the use of alcohols at 165 °C.¹² While these methods offer an alternative for the use of pre-ativation strategies for amide cleavage, the need for mild amide cleavage strategies, using much lower temperatures and preferentially ambient temperature, remains. Indeed, because of their high reaction temperature, these protocols only enable limited amide and alcohol scope. Therefore, new strategies to activate stable amide moieties for nucleophilic attack are still needed to enable cleavage methods at reasonable temperatures.

When amines are used as nucleophiles (transamidation/transureation/transcarbamation), the same lack of good and general protocols can be observed. For transamidation, mixtures are expected in some cases, as the stability of substrate and product amide are not always sufficiently different (thermodynamically neutral). Synthetically useful transamidations are therefore often limited to amides that generate a volatile amine as leaving group (e.g. ammonia), creating a shift in the equilibrium, but generally not applicable. These known methods for transamidation typically use elevated temperatures with,^{13,14} or without metal-^{15,16} or organocatalysts or small molecule assistance.¹⁷⁻¹⁹ However, pre-activation strategies succeed in the cleavage of amides at lower temperatures (up to ambient temperature).^{20,21} These methods have the same disadvantages as mentioned herein above for the alcoholysis of amides via leaving group activation, i.e. they require dangerous and expensive reagents that suffer from limited functional group compatibility.

When double cleavage, *in se* of a -NH-CO-NH- moiety is studied, the state-of-the-art is even more limited. Likewise, the transformation of urea into a carbamate and carbonate requires ammonia to act as a leaving group. This poses an additional challenge since an amine is generally a better nucleophile than an alcohol (except in specific cases e.g. (hetero)aromatic amines). For reactions of urea and carbamates with amines, transamidations (*in se* transureations and transcarbamations), mixtures are expected as the stability of substrate and product is generally not sufficiently different (thermoneutral reaction). For (thio)alcoholysis and transamidation of urea, direct transformation of urea in carbamates or carbonates in the presence of a metal catalyst is known, but requires high temperatures. The transformation of urea into carbamates and carbonates has mainly been studied with simple aliphatic alcohols and especially in the context of dimethyl carbonate (DMC) synthesis using methanol. Due to the unfavorable equilibrium no full conversion can be achieved and mixtures of carbamate and carbonate are obtained. Although catalysts are applied, the state-of-the-art requires a very high reaction temperature, which causes decomposition of the carbonate, and a high methanol to urea ratio.²² Transamidation ("transureation") on urea is known but to the best of our knowledge not on carbamates. The transamidations on urea require a high temperature, even in the presence of a catalyst, and even when very concentrated reaction conditions are used (neat).²³

Cobo J. et Al, 1994, disclose a method of tandem Diels-Alder/Retro Diels-Alder reaction for the synthesis of 2-aminopyridines. Therein, are disclosed nicotinate compounds structurally similar to those disclosed in the present patent application²⁹.

Also US2009156646 and EP1314712 disclose nicotinate compounds structurally similar to those hereby described. EP1314712 discloses pharmaceutical compounds having NF-Kappa-B inhibitory activity.

Liana Hie et al, 2015, describe a method for the conversion of amides to esters by the nickel-catalysed activation of amides C-N bonds. More specifically, it describes a reagent system using a nitrogen containing heterocycle and a metal catalyst for the conversion of amides to esters. For the present method, no reaction between the additive/heterocyclic part takes place³⁰.

Sarah M. Crawford et al, describe a BippyPhos ligand for Buchwald-Hartwig amination of (hetero)aryl chlorides³¹.

Kita et al, 2012, describe the zinc-catalyzed amide cleavage and esterification of betahydroxyethylamides. For said reactions, additives such as benzaldehyde are used. For the present method, no reaction between the additive/heterocyclic part takes place⁷.

Xue Cuihua et al, 2004, describe a process for the conversion of primary amides to esters by the use of chlorotrimethylsilane⁵.

In view of the disadvantages associated with the prior art methods as described herein above, it was an object of the present application to provide a new method for transformation of compounds containing an amide group (-CONH-) via nucleophilic attack of amines (RNH₂, RNHR'), thiols (RSH) and alcohols (ROH), which is in particular applicable at significant lower temperatures and pressures than the state-of-the-art. This milder and chemoselective amide cleavage method should use neutral conditions and a catalyst, rather than potentially toxic and stoichiometric amide activating reagents. Indeed, lower temperature processes enable potential application for production of fine and bulk chemicals in a sustainable manner as they reduce energy consumption. Furthermore, it is generally preferable to use catalytic amounts of additives to perform the reactions. We stress that various approaches have been reported for the cleavage of amide-containing compounds, but surprisingly enough homogeneous transition metal catalysis as being the subject of this application, was not successfully applied to achieve the goal of cleavage under mild and neutral conditions, while equally introducing a general amide-cleaving method for various -NH-CO- containing moieties and enabling the use of different classes of heteronucleophiles.

### SUMMARY OF THE APPLICATION

### DEFINITIONS

By amide group we mean a group having the structure -NHCO-, and within the scope of our definition of amide group we include groups in which the structural unit -NHCO- is part of a larger group or structural entity, such as, but not limited to: urea, oxamide, carbamate, thiocarbamate. By amide group we do not mean thioamide, imide, fosfonamide, sulphonamide, sulfenamide or a metal derivative of ammonia and amines in which a cation replaces a hydrogen atom on nitrogen: what is defined by IUPAC as a carboxamide group should at least be present, but this carboxamide unity can be part of a larger functional group, such as, but not limited to: urea, oxamide, carbamate, thiocarbamate.

Following this definition, an amide can be depicted according to the following formula (I): 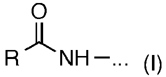 wherein R is a hydrogen, aryl, heteroaryl, heteroarylalkyl, arylalkyl, alkyl, alkenyl, alkynyl, arylamino, heteroarylamino, alkoxy, alkylthio, alkylamino, dialkylamino, alkyl aryl amino, alkyl heteroaryl amino, heteroaryl arylamino, diheteroarylamino, diarylamino, arylthio, heteroarylthio, aryloxy, heteroaryloxy, hydroxyl, amino, thio wherein said aryl, heteroaryl, alkoxy or alkyl is optionally substituted with one to ten substituents independently selected from the group consisting of halogen, aryl, heteroaryl, alkyl, trifluoromethyl, alkoxy, alkoxycarbonyl, trifluoromethoxy, alkylthio, cyano, alkylamino, nitro, hydroxyl, carbonyl, amino, oxime, imino, azido, hydrazine, acyl, alkylamino, dialkylamino, carboxylic acid, acylamino, alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino; said R-group can be a polymer. Likewise, a tautomer, isomer, salt or solvate of R-CO-NH-... is also considered to be an amide.

Furthermore, a directing group (DG) is defined as an easily introducible and removable group that enables achieving efficient reaction control in organometallic chemistry.^{24,25}

Herein, an amide-cleaving directing group (ACDG) is used to identify a substituted or unsubstituted azaheterocyclic ring containing at least one unsaturated bond; this unsaturated bond is part of an endocyclic imino function, in which the free electron pair of the nitrogen group is available for exocyclic coordination with a metal or for hydrogen bonding via an electrostatic interaction with a hydrogen bond donating group. This directing group can be introduced on what we previously defined as an amide (thus replacing the ... in formula (I) by the DG), on the condition that by it's introduction an amidine structural motive, defined as R-N=C-NH-CO-, is formed. This resulting amidine R-N=C-NH-CO-(ACDG-NH-CO) can either (1) form a bidentate chelate in the presence of a metal: the metal coordinates both the carbonyl of the amide group and the endocyclic nitrogen atom of the ACDG that is part of the imino group, leading to the formation of a six-membered ring system or (2) chelate a metal via the nitrogen group of the -C-NH-CO- moiety should this amide occur in it's imidic acid tautomer, whereas the hydrogen atom of the imidic acid may form a hydrogen bond with the nitrogen atom of the previously defined -R-N=C- motive or (3) chelate a metal via the nitrogen group of the - C-NH-CO- moiety should this amide occur in it's imidic acid tautomer and/or chelate a metal via the nitrogen atom of the previously defined -R-N=C- motive; the latter metal can potentially chelate the oxygen atom of the imidic acid moiety. The azaheterocyclic ring can be substituted, but the substituents don't necessarily enable interactions that play a role in the directing effect. Examples of azaheterocyclic systems that can be ACDG's for amides are - but not limited to-: pyridin-2-yl, pyrazin-2-yl, pyrimidin-2-yl, isoquinonlin-2-yl, quinolin-2-yl, imidazolyl-2-yl, thiazolyl-2-yl, oxazolyl-2-yl, imidazolyl-4-yl, thiazolyl-4-yl, oxazolyl-4-yl, isoxazol-3-yl, thioxazol-3-yl, pyrazol-3-yl, purin-6-yl, pyridazin-6-yl, azecin-2-yl, azocinyl-2-yl, imidazolin-2-yl, thiazolin-2-yl, oxazolin-2-yl.

Furthermore, a hydrogen bond-based amide-cleaving directing group (HBDG) is a directing group that answers to the definition of an amide-cleaving directing group (ACDG), but that also bears an exocyclic substituent in alpha-position of the endocyclic imino function. Said exocyclic substituent contains a heteroatom that is positioned in alpha, beta or gamma position of said endocyclic imino function.

By *N*-ACDG amide we mean a compound having the structure ACDG-NH-CO-, and within the scope of our definition of *N*-ACDG amide we include groups in which the structural unit ACDG-NHCO- is part of a larger group or structural entity, such as, but not limited to: urea, oxamide, carbamate, thiocarbamate. By *N*-ACDG amide we not mean the *N*-substitution of thioamides, imides, fosfonamides, sulphonamides or sulfenamides with a directing group HBDG.

By *N*-HBDG amide we mean a compound having the structure HBDG-NH-CO-, and within the scope of our definition of *N*-HBDG amide we include groups in which the structural unit HBDG-NHCO- is part of a larger group or structural entity, such as, but not limited to: urea, oxamide, carbamate, thiocarbamate. By *N*-HBDG amide we not mean the *N*-substitution of thioamides, imides, fosfonamides, sulphonamides or sulfenamides with a directing group HBDG. Note that an *N*-HBDG amide is always an *N*-ACDG amide, but that an *N*-ACDG is not always an *N*-HBDG amide.

Following the previous definition, by *N,N'*-ACDG₂ amide we mean a compound having the structure ACDG-NH-CO-NH-ACDG or having the structure ACDG-NH-CO-X-CO-NH-ACDG, in which X can consists of any functional group or combination of functional groups.

Similarly, by *N*^{*n*-}ACDGₙ amide, we mean a compound having a structure wherein the structural motive ACDG-NH-CO- can be found n times. When identifying n, the sum of the number of ACDG-NH-CO structural motives, a -CO- moiety can be used one or two times to identify this motive.

Following the previous definition, by *N,N'*-HBDG₂ amide we mean a compound having the structure HBDG-NH-CO-NH-HBDG or having the structure HBDG-NH-CO-X-CO-NH-HBDG, in which X can consists of any functional group or combinations of functional groups.

Similarly, by *Nⁿ*-HBDGₙ amide, we mean a compound having a structure wherein the structural motive HBDG-NH-CO- can be found n times. When identifying n, the sum of the number of HBDG-NH-CO structural motives, a -CO- moiety can be used one or two times to identify this motive.

Note than an *N*-ACDG amide can also be an *Nⁿ*-ACDGₙ amide or an *N,N'*-ACDG₂ amide.

Note than an *N*-HBDG amide can also be an *Nⁿ*-HBDGₙ amide or an *N,N'*-HBDG₂ amide.

An ACDG-precursor is defined as a compound that can be selected from the following list: an amide-containing compound, LG-CO-LG (with LG = suitable leaving group), CO₂, and CO. Said ACDG-precursor compound can be transformed into an ACDG-containing (amide-cleaving directing group-containing) compound, by introducing an amide-cleaving directing group (ACDG).

A leaving group (LG) is defined according to IUPAC as an atom or group (charged or uncharged) that becomes detached from an atom in what is considered to be the residual or main part of the substrate in a specified reaction. Examples of leaving groups are F, Cl, I, Br, OTs, OMs, N(alkyl)₃,...

An ACDG-introducing compound is a compound that can be reacted in sub-, supra- or stoichiometric (but not in catalytic) fashion with an ACDG-precursor in the presence or absence of catalyst(s), ligand(s), reactant(s), reagent(s) and/or solvent(s) to an ACDG-containing (amide-cleaving directing group-containing) compound of the formula ACDG-NH-CO-.

By heteronucleophile we mean a nucleophilic compound in which the atom containing nucleophilic properties is a heteroatom such as nitrogen (N), sulphur (S) or oxygen (O). Herein, the term heteronucleophile (HetNu) indicates an amine (RNH₂ or RNHR') or an alcohol (ROH) or thiol (RSH)). By catalyst, we mean a metal catalyst defined according to the general structure M_{y}Lₓ or a salt, solvate or hydrate thereof in which M is a metal or semi-metal and L represents a suitable organic or inorganic ligand, x = 0-30, y preferably = 1 (1 metal and x ligands) or y > 1 (y metals and x ligands).

### SUMMARY

Although the cleavage of amide-containing compounds with the use of heteronucleophiles such as (*in se* an amine (RNH₂ or RNHR') or an alcohol (ROH) or thiol (RSH)) has been largely studied, there is no method available that combines both broad applicability and relatively mild conditions, defined as a bench-marked maximum cleavage temperature of 60 °C for amide and carbamate cleavage and mono-cleavage of urea and 100 °C for double cleavage of urea, when using steric and electronically suitable heteronucleophiles. It was surprisingly found that reactions with heteronucleophilic moieties at such lower temperatures are possible by using a DG on the nitrogen atom of the amide-containing compound. Indeed, by converting the amide to an *Nⁿ*-ACDGₙ amide (step of amide-cleaving directing group introduction), the *Nⁿ*-ACDGₙ amide (nₘₐₓ = 10) can be cleaved 1 to maximum n times (using a metal-catalyst and with the use of 1 to maximum n HetNu in 1 to maximum n steps using 1 to maximum n different temperatures, resulting in the formation of 1 end-product and 0 to (n-1) isolatable intermediates. Said heteronucleophilic moieties may be selected from amines (RNH₂ or RNHR'), alcohols (ROH) or thiols (RSH)). When applied to the catalytic directed cleavage of an *Nⁿ*-ACDGₙ amide with n > 1, one can use the same heteronucleophile for the n cleavage steps (resulting in a one step process) or select m different HetNu to apply in m cleavage steps, with m = n or m < n. Thus, the present application relates to a method for the conversion of amides by means of a build-in directing group, named an amide-cleaving directing group, and upon the action of a nucleophilic compound (*in se* an amine (RNH₂ or RNHR') or an alcohol (ROH) or a thiol (RSH)) in the presence of a metal catalyst to respectively amides, esters, thioesters, urea, carbamates, thiocarbamates, carbonates or thiocarbonates. The present application also relates to amides and urea having a build-in DG, as well as the use of such DG in the catalytic directed mono-cleavage of *N*-DG amides (DG-NH-CO-R) with the use of nucleophiles (in se an amine (RNH₂ or RNHR') or an alcohol (ROH) or thiol (RSH)).

Briefly, the following reactivity hypothesis is proposed: the introduction of a DG will provide activation of the unreactive carbonyl group of said amide-containing compound through either (1) the simultaneous bidentate complexation of the metal catalyst with the carbonyl function of the amide and a heteroatom of the directing group or through (2) the occurrence of the amide moiety in it's imidic acid tautomeric form and complexation of the metal catalyst with the imino function of the imidic acid and/or hydrogen bond formation of the hydrogen atom of the imidic acid with a heteroatom of the directing group or through (3) the occurrence of the amide moiety in it's imidic acid tautomeric form and complexation of the metal catalyst with the imino function of the imidic acid and/or chelation of a metal with the oxygen atom of the imidic acid moiety and/or with a heteroatom of the directing group with a metal catalyst.

Additionally, the structure of the amide-cleaving directing group (ACDG) can be refined towards a hydrogen bond-based amide-cleaving directing group (HBDG). Said HBDG is an ACDG that also bears an exocyclic substituent in alpha-position of the endocyclic imino function that was defined in the definition of ACDG. Said exocyclic substituent contains a heteroatom that is positioned in alpha, beta or gamma position of said endocyclic imino function. Similarly, the following reactivity hypothesis is proposed: the introduction of the HBDG will provide activation of the unreactive carbonyl group of said amide-containing compound through either (1) the simultaneous bidentate complexation of the metal catalyst with the carbonyl function of the amide and a heteroatom of the directing group and/or hydrogen bonding of the hydrogen atom of the amide moiety and the herebove defined exocyclic substituent containing a heteroatom (intermolecular hydrogen bonding) or through (2) the occurrence of the amide moiety in it's imidic acid tautomeric form and complexation of the metal catalyst with both the imino function of the imidic acid and the herebove defined exocyclic substituent containing a heteroatom (bidentate chelation) and/or hydrogen bond formation of the hydrogen atom of the imidic acid with a heteroatom of the directing group or through (3) the occurrence of the amide moiety in it's imidic acid tautomeric form and complexation of the metal catalyst with both the imino function of the imidic acid and the herebove defined exocyclic substituent containing a heteroatom (bidentate chelation) and/or chelation of a metal with the oxygen atom of the imidic acid moiety and/or with a heteroatom of the directing group with a metal catalyst.

In summary, the introduction of said HBDG will provide activation of the unreactive carbonyl group of said amide-containing compound. Indeed, by converting a primary amide to an *Nⁿ*-HBDGₙ amide (step of HBDG-introduction), the *Nⁿ*-HBDGₙ amide can be cleaved 1 to maximum n times (n = 1 to 10) using a metal-catalyst in catalytic amounts and with the use of 1 to maximum n HetNu in 1 to maximum n steps using 1 to maximum n different temperatures, resulting in the formation of 1 end-product and 0 to (n-1) isolatable intermediates. In the context of the convention, said heteronucleophilic moieties may be selected from amines (RNH₂ or RNHR'), alcohols (ROH) or thiols (RSH)) and said reaction temperatures will be lower than for the cleavage of *Nⁿ*-ACDGₙ amides. When applied to the catalytic directed cleavage of *Nⁿ*-HBDGₙ with n > 1, one can use one and the same heteronucleophile for the n cleavage steps (resulting in a one step process) or select m different HetNu to apply in m cleavage steps, with m = n or m < n. Thus, the present application also relates to a method for the conversion of amides by means of a build-in directing group, named a hydrogen bond-based amide-cleaving directing group, and upon the action of a nucleophilic compound (*in se* an amine (RNH₂ or RNHR') or an alcohol (ROH) or a thiol (RSH)) in the presence of a metal catalyst to respectively amide-containing compounds (such as carboxamides, urea, carbamates thiocarbamates), esters, thioesters, carbonates or thiocarbonates.

### BRIEF DESCRIPTION OF THE DRAWINGS

With specific reference now to the figures, it is stressed that the particulars shown are by way of example and for purposes of illustrative discussion of the different embodiments of the present application only. They are presented in the cause of providing what is believed to be the most useful and readily description of the principles and conceptual aspects of the application. In this regard no attempt is made to show structural details of the application in more detail than is necessary for a fundamental understanding of the application. The description taken with the drawings making apparent to those skilled in the art how the several forms of the application may be embodied in practice.
Figure 1: Concepts for amide activation toward addition-elimination reactions with hetero-nucleophiles.
Figure 2: Design of possible alternative DG's.

### DETAILED DESCRIPTION OF THE APPLICATION

### DEFINITIONS

The present application will now be further described. In the following passages, different aspects of the application are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Unless a context dictates otherwise, asterisks (*) or suspension points (...) are used herein to indicate the point at which a mono- or bivalent radical depicted is connected to the structure to which it relates and of which the radical forms part.

When describing the methods and compounds of the application, the terms used are to be construed in accordance with the following definitions, unless a context dictates otherwise:
The term "alkyl" by itself or as part of another substituent refers to a fully saturated hydrocarbon of Formula CₓH₂ₓ₊₁ wherein x is a number greater than or equal to 1. Generally, alkyl groups of this application comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, *i*-butyl and *t*-butyl); pentyl and its isomers, hexyl and its isomers, heptyl and its isomers, octyl and its isomers, nonyl and its isomers; decyl and its isomers. C₁-C₆ alkyl includes all linear, branched, or cyclic alkyl groups with between 1 and 6 carbon atoms, and thus includes methyl, ethyl, n-propyl, *i*-propyl, butyl and its isomers (e.g. *n*-butyl, *i*-butyl and *t*-butyl); pentyl and its isomers, hexyl and its isomers, cyclopentyl, 2-, 3-, or 4-methylcyclopentyl, cyclopentylmethylene, and cyclohexyl.

The term "optionally substituted alkyl" refers to an alkyl group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

The term "optionally substituted alkoxy" refers to an alkoxy group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

The term "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[*a,d*]cylcoheptenyl, and 1-, 2-, 3-, 4-, or 5-pyrenyl. The aryl ring can optionally be substituted by one or more substituents. An "optionally substituted aryl" refers to an aryl having optionally one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO₂-NH₂, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO₂R^{a}, alkylthio, carboxyl, and the like, wherein R^{a} is alkyl or cycloalkyl.

Where a carbon atom in an aryl group is replaced with a heteroatom, the resultant ring is referred to herein as a heteroaryl ring.

The term "heteroaryl" as used herein by itself or as part of another group refers but is not limited to 5 to 12 carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. As used herein with respect to a substituting group, and unless otherwise stated, the term "heteroaryl" thus refers to a mono- or polycyclic, aromatically unsaturated monovalent hydrocarbon group having from 2 up to 15 carbon atoms and including one or more heteroatoms in one or more rings, each of said rings having from 3 to 10 atoms (and optionally further including one or more heteroatoms attached to one or more carbon atoms of said ring, for instance in the form of a carbonyl or thiocarbonyl or selenocarbonyl group, each of said heteroatoms being independently selected from the group consisting of nitrogen, oxygen and sulfur, also including groups wherein a heterocyclic ring is fused to one or more aromatic hydrocarbon rings for instance in the form of benzo-fused, dibenzo-fused or naphtho-fused heterocyclic groups, and also including groups wherein each carbon atom of each ring may furthermore be independently substituted with a substituent selected from the group consisting of halogen, nitro, C1-4 alkyl (optionally containing, in the main chain or a side chain, one or more atoms or groups such as oxo, hydroxyl, ether, thioether, acetal, amino, or halogen). Within the broad definition hereinabove are included heterocyclic aromatically unsaturated groups such as, but not limited to, diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxazepinyl, benzothiazepinyl, benzodiazepinyl, benzoxazocinyl, benzothiazocinyl, benzodiazocinyl, benzoxathiocinyl, benzoxathiazepinyl, benzoxadiazepinyl, benzothiadiazepinyl, benzotriazepinyl, benzotriazinonyl, benzoxazolinonyl, azetidinonyl, azaspiroundecyl, selenazinyl, selenazolyl, selenophenyl, azahypoxanthinyl, bipyrazinyl, bipyridinyl, oxazolidinyl, diselenopyrimidinyl, benzophenazinyl, benzoquinolizinyl, dibenzocarbazolyl, dibenzoacridinyl, dibenzophenazinyl, dibenzoquinoxalinyl, dibenzothiazepinyl, dibenzisoquinolinyl, tetraazaadamantyl, thiatetraazaadamantyl, oxauracil, oxazinyl, oxazolinyl, oxazolonyl, azaindolyl, azolonyl, thiazolinyl, thiazolonyl, thiazolidinyl, thiazanyl, pyrimidonyl, thiopyrimidonyl, azlactonyl, naphthindazolyl, naphthindolyl, naphthothiazolyl, naphthothioxolyl, naphthoxindolyl, naphthotriazolyl, azabenzimidazolyl, azacycloheptyl, azacyclooctyl, azacyclononyl, azabicyclononyl, dioxindolyl, dioxazinyl, thiourazolyl, thiotriazolyl, quinoleinyl, oxyquinoleinyl, quinuclidinyl, xanthinyl, dihydropyranyl, benzodihydrofuryl, benzothiopyronyl, benzothiopyronyl, benzoxazinyl, benzoxazolyl, benzodioxolyl, benzodioxanyl, benzothiadiazolyl, benzotriazinyl, benzothiazolyl, benzoxazolyl, phenothiazolyl, phenoxazolyl, pyridinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, hydantoinyl, indolyl, indazolyl, quinolyl, quinazolinyl, quinoxalinyl, carbazolyl, phenoxazinyl, phenothiazinyl, xanthenyl, purinyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, benzimidazolyl, uridinyl, thymidinyl, cytidinyl, azirinyl, aziridinyl, diazirinyl, diaziridinyl, oxaziridinyl, azetyl, azetidinyl, diazabicyclooctyl, diazetyl, diaziridinonyl, diaziridinethionyl, benzisothiazolyl, benzocarbazolyl, benzisoalloxazinyl, phenometoxazinyl, phenoparoxazinyl, phentriazinyl, thiodiazinyl, thiodiazolyl, benzodiazinyl (e.g. phtalazinyl), phthalidyl, phthalimidinyl, phtalazonyl, alloxazinyl, isatyl, isopyrazolyl, isopyrazolonyl, urazolyl, urazinyl, uretinyl, uretidinyl, and the like, including all possible isomeric forms thereof.

The term "oxo" as used herein refers to the group =O.

The term "halo" or "halogen" as a group or part of a group is generic for fluoro, chloro, bromo, or iodo. Where groups may be optionally substituted, such groups may be substituted with once or more, and preferably once, twice or thrice. Substituents may be selected from, for example, the group comprising halogen, hydroxyl, oxo, nitro, amido, carboxy, amino, cyano haloalkoxy, and haloalkyl.

As used herein the terms such as "alkyl, aryl, or cycloalkyl, each being optionally substituted with" or "alkyl, aryl, or cycloalkyl, optionally substituted with" refers to optionally substituted alkyl, optionally substituted aryl and optionally substituted cycloalkyl.

It will be clear to the skilled person that the compounds of the application may exist in the form of different isomers and/or tautomers, including but not limited to geometrical isomers, conformational isomers, E/Z-isomers, stereochemical isomers (i.e. enantiomers and diastereoisomers) and isomers that correspond to the presence of the same substituents on different positions of the rings present in the compounds of the application. All such possible isomers, tautomers and mixtures thereof are included within the scope of the application. Furthermore, when stable or radioactive isotopes of atoms are used to build-up the compounds, said resulting compound is also included in the scope of the application. Furthermore, all salts and hydrates and mixtures thereof are included within the scope of the application.

As used in the specification and the appended claims, the singular forms "a", "an", and "the" include plural referents unless the context clearly dictates otherwise. By way of example, "a compound" means one compound or more than one compound.

As used herein and unless otherwise stated, the term "solvate" includes any combination which may be formed by a derivative of this application with a suitable inorganic solvent (e.g. hydrates) or a suitable organic solvent such as, but not limited to, alcohols (thus forming alcoholates), ketones, esters, ethers, nitriles (e.g. acetonitrile) and the like.

The terms described above and others used in the specification are well understood to those skilled in the art.

### APPLICATION

Our application relates to the fact that the chemical inertness of the amide can be overcome by introducing a directing group (DG), *in se* an amide-cleaving directing group (ACDG), on the nitrogen atom of the primary amide (-CO-NH₂). The new approach presented is first of all to introduce n times an amide-cleaving directing group (ACDG) onto the nitrogen atom of the primary amide (NH₂-CO-), resulting in the formation of an *Nⁿ*-ACDGₙ amide (with n = 1 or n >1) to overcome the stability of the - NH₂-C=O- moiety in said compound by means metal chelation and/or hydrogen bonding. Also, it is shown that the steric and electronic properties of this DG will contribute to the increased reactivity. So, secondly, a well-chosen substituent on the amide-cleaving directing group, preferably a hydrogen bond acceptor moiety in beta-position of the carbonyl of the ACDG-NH-C=O moiety, can be introduced to further lower the activation energy either through (1) hydrogen bonding with the hydrogen atom in the resulting ACDG-NH-C=O moiety in the resulting *Nⁿ*-ACDGₙ amide (with n = 1 or n >1) or through (2) chelation of this hydrogen bond acceptor moiety in beta-position of the carbonyl of the ACDG-NH-C=O moiety with a metal. This substituent can be introduced when it is wanted, necessary or convenient to lower the reaction temperature of the directed catalytic cleavage reaction of the resulting *Nⁿ*-ACDGₙ amide with the use of HetNu (*in se* an amine (RNH₂ or RNHR') or an alcohol (ROH) or a thiol (RSH) (when n = 1 or n>1), or a combination thereof (when n > 1 and by using m HetNu in m cleavage steps, with m > 1 and m > or = n). In that case, *Nⁿ*-ACDGₙ amide (n > 0) can be called an *Nⁿ*-HBDGₙ amide. This finding thus implies a new method to cleave primary amides under relatively mild conditions, in a catalytic fashion and a synthetic useful manner. When neglecting existing (synthetic and natural) metal-dependent enzymes such as metalloproteases and ureases for being very substrate specific and thus focusing on mild and broad-scope chemocatalysis methods for amide cleavage rather then biocatalysis, this is the first method that succeeds in the chemoselective, relatively mild cleavage of a broad scope of primary amides with the use HetNu *(in se* an amine (RNH₂ or RNHR') or an alcohol (ROH) or a thiol (RSH)) in solvolytic or stoichiometric amounts and with the use of catalytic amounts of metals under neutral conditions and broad-scope synthetic applicability. Said relatively mild conditions are defined as a bench-marked maximum cleavage temperature of 60 °C when n = 1 or when p, the number of amide moieties to be cleaved, is 1, and 100 °C when n > 1 and p > 1 and p < n or p = n. Since low temperature processes have potential application for the production of fine chemicals in a sustainable manner as they reduce energy consumption and since the use of catalytic processes is preferred over waste-full and/or potentially dangerous pre-activation strategies, this method can be considered truly innovating. The formation of an amine by-product, ACDG-NH₂, is no downside, since it can be valorised separately or used to synthesise the starting compounds for a next cleavage cycle, *in se* the *Nⁿ*-ACDGₙ amides, that can also be *Nⁿ*-HBDGₙ amides.

When n = 2, the *Nⁿ*-ACDGₙ amides can be called *N,N'*-ACDG₂ amides. These *N,N'*-ACDG₂ amides can be cleaved one or two times in the presence of a heteronucleophile *(in se* an amine (RNH₂ or RNHR') or an alcohol (ROH) or a thiol (RSH)) and a metal-catalyst in catalytic amounts. In the case of two cleavage steps, a higher temperature (Tₘₐₓ = 100 °C) than in the case of 1 cleavage step (Tₘₐₓ = 60 °C) is necessary. When a symmetric end-product is desired and thus one and the same heteronucleophile *(in se* an amine (RNH₂ or RNHR') or an alcohol (ROH) or a thiol (RSH)) is used for both amide cleavage steps, the reaction can proceed as a one pot reaction. When an asymmetric end-product is desired, and thus two different HetNu (*in se* an amine (RNH₂ or RNHR') or an alcohol (ROH) or a thiol (RSH)) are used for the two cleavage steps, the reaction proceeds in 2 steps, wherein the second step proceeds at a higher temperature than step 1.

The inventive step of the herein reported method is using the amide-cleaving directing group to synthesise an ACDG-containing amide that can be cleaved upon the reaction with heteronucleophiles in the presence of a metal catalyst. The synthesis of the ACDG-containing amide cannot only be done from an amide-containing compound, but more generally from any ACDG-precursor. Thus, this application equally relies to converting an ACDG-precursor compound into an ACDG-containing (amide-cleaving directing group-containing) compound, by introducing an amide-cleaving directing group (ACDG), resulting in the formation of a *N*-ACDG amide of formula ACDG-NH-CO-, and this by the reaction of said ACDG-precursor with an ACDG-introducing compound in the presence or absence of catalyst(s), ligand(s), reactant(s), reagent(s) and/or solvent(s) to an ACDG-containing (amide-cleaving directing group-containing) compound of the formula ACDG-NH-CO-.

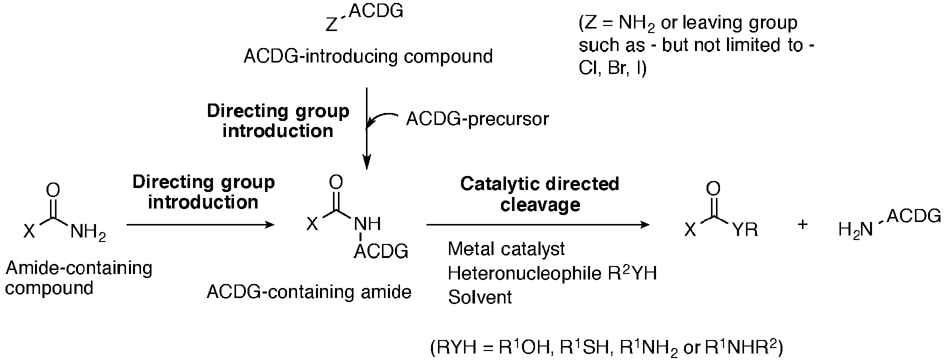

### Scheme 1: General concept of the application: new strategy for metal-catalyzed directed cleavage of amide-containing compounds with an N-ACDG directing group.

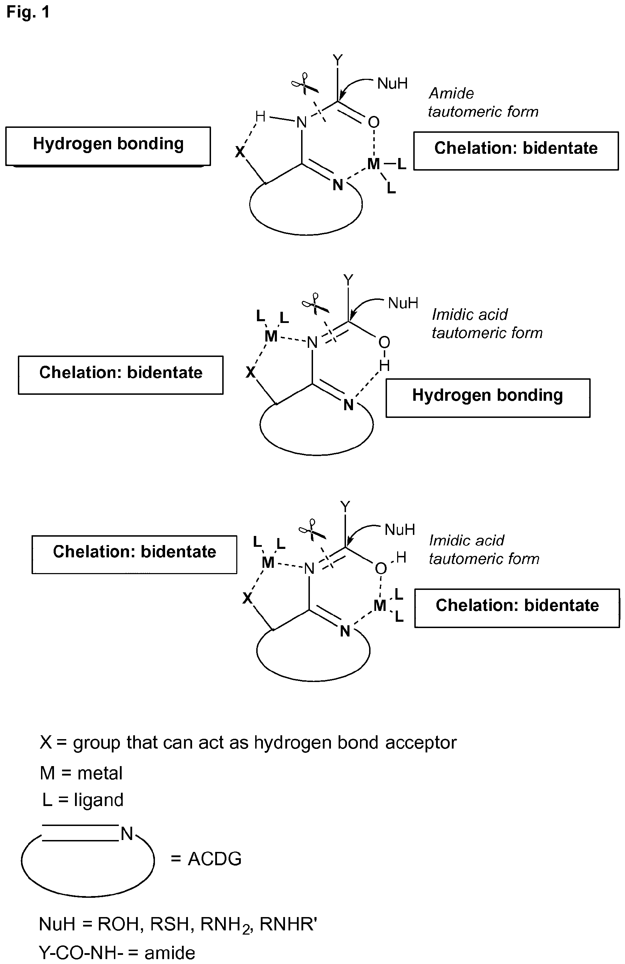

In summary, this application is based on the novel method for the catalytic directed cleavage of amide-containing compounds with heteronucleophiles, like presented in the general embodiment in Scheme **1** and Figure **1****.** Surprisingly, in one embodiment of this method using a pyridine-based DG, it was found that this cleavage may proceed using very mild conditions. For example, kinetic studies show that full Zn-catalyzed (4.0 mol% Zn(OAc)₂.17H₂O) cleavage of *tert*-butyl 2-benzamidonicotinate with the use of primary alcohols such as MeOH (2.5 equiv) is accomplished within 30 minutes at 60 °C. To the best of our knowledge, this is unprecedented by any existing method. Indeed, the hereto known mildest cleavage non-enzymatic of benzamide with the use of stoichiometric amounts of MeOH under neutral conditions without first pre-activating the benzamide to a non-carboxamide containing intermediate, is a process a 165 °C for 22 h (CeO₂-catalysis, 1.0 equiv MeOH, mesitylene as a solvent).¹² On the other hand, when using stoichiometric amounts of these simple primary alcohols 1 at 40 °C, full cleavage of this substrate is also possible at room temperature in 1 day. In the presence of 4.0 mol% catalyst and with the use of 1.5 equiv EtOH, *tert*-butyl 2-benzamidonicotinate can be converted to ethyl benzoate in 83% GC-yield at 40 °C within 24 h.

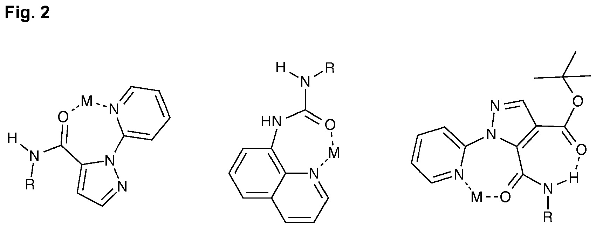

This application is solemnly based on a certain DG scaffold, previously defined as ACDG and graphically represented in the embodiments in Scheme **1** and Figure 1. However, based on the principles proposed in this work, alternative DG can be proposed and designed. Possible DG are depicted in Figure 2. For example, 7-membered metallocycles are proposed as a result of bidentate chelation, although other mechanistic insights in the potential of these directing groups can be proposed, based on the herein proposed reaction hypotheses (*vide supra*)

This application relates to a new strategy for metal-catalyzed directed cleavage of amide-containing compounds. Different implementations and methods of carrying out the application are described in the following embodiments (EM).
EM1. Viewed from one aspect of the embodiments herein, this application provides a method for the catalytic cleavage of primary amide-containing compounds to obtain esters, thioesters, carbonates, thiocarbonates and what is defined as amide-containing compounds (such as carboxamides, urea, carbamates thiocarbamates) ; said method comprising the steps of:
   - providing a primary amide-containing (H₂N-CO-) compound;
   - converting said compound into a ACDG-containing (amide-cleaving directing group-containing) compound by introducing an amide-cleaving directing group, according to the formula (Ia), on the nitrogen atom of said amide, resulting in the formation of an *N*-ACDG amide;
      Said amide-cleaving directing group (ACDG) is a substituted or unsubstituted azaheterocyclic ring containing at least one unsaturated bond; this unsaturated bond is part of an endocyclic imino function, in which the free electron pair of the nitrogen group is available for exocyclic coordination with a metal or for hydrogen bonding via an electrostatic interaction with a hydrogen bond donating group. This directing group can be introduced on what we previously defined as an amide, on the condition that by it's introduction an amidine structural motive, defined as R-N=C-NH-CO-, is formed. This resulting amidine R-N=C-NH-CO- (ACDG-NH-CO) can either (1) form a bidentate chelate in the presence of a metal: the metal coordinates both the carbonyl of the amide group and the endocyclic nitrogen atom of the ACDG that is part of the imino group, leading to the formation of a six-membered ring system or (2) chelate a metal via the nitrogen group of the -C-NH-CO- moiety should this amide occur in it's imidic acid tautomer, whereas the hydrogen atom of the imidic acid may form a hydrogen bond with the nitrogen atom of the previously defined -R-N=C- motive or (3) chelate a metal via the nitrogen group of the -C-NH-CO- moiety should this amide occur in it's imidic acid tautomer and/or chelate a metal via the nitrogen atom of the previously defined -R-N=C- motive; the latter metal can potentially chelate the oxygen atom of the imidic acid moiety. The azaheterocyclic ring can be substituted, but the substituents don't necessarily enable interactions that play a role in the directing effect;
   - cleaving said ACDG-containing compound (namely the *N*-ACDG amide) in the presence of a metal catalyst (defined as a M_{y}Lₓ compound or a salt, solvate or hydrate thereof in which M is a metal or semi-metal like Al, Zn, Cu, Ni, Co, Fe, Mn, Pd, Ru, Rh, Pb, Zr, Sc, Ti, Cr, Ga, Ge, Se, Zr, Nb, Ag, Cd, In, Sn, Sb, Pb, Te, Ir, Re, Os, Pt, Au, W, Eu, La, Yb, Ta, preferably Zn, Mn, Ni or Co, and L represents a suitable organic or inorganic ligand; preferably a carboxylate-based ligand; x = 0-30, y preferably = **1** (**1** metal and x ligands) or y > 1 (y metals and x ligands)) and a nucleophile (preferably a heteronucleophile (HetNu) selected from the list comprising alcohols (ROH), thiols (RSH) and amines (RNH₂ or RNHR')) in stoichiometric (in the presence or the absence of a co-solvent) or solvolytic amounts at room temperature to 140 °C under neutral conditions; said *N*-ACDG amide is the compound having the structure ACDG-NH-CO-, and within the scope of our definition of *N*-ACDG amide we include groups in which the structural unit ACDG-NHCO- is part of a larger group or structural entity, namely: urea, oxamide, carbamate, thiocarbamate. 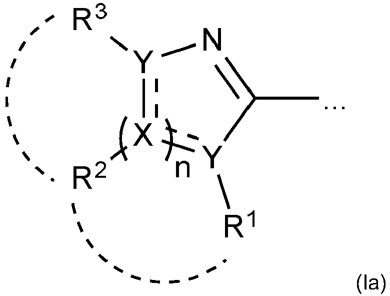 wherein
      X = C, N, O or S.
      Y = C, N or S or R¹Y = O
      n = 1 or n > 1.
      R¹, R² and R³ R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, diarylamino, diheteroarylamino, aryl heteroarylamino, arylthio, heteroarylthio, aryloxy, heteroaryloxy.
      - - - = optional chemical bond
EM2. In a particular embodiment, of EM1 or EM55, said amide-cleaving directing group (ACDG) is a hydrogen bond-based amide-cleaving directing group (HBDG): a directing group that answers to the definition of an amide-cleaving directing group (ACDG), but that also bears an exocyclic substituent in alpha-position of the endocyclic imino function. Said exocyclic substituent contains a heteroatom that is positioned in alpha, beta or gamma position of said endocyclic imino function. In another particular embodiment of EM1, the provided primary amide-containing compound can be replaced by a secondary amide containing compound: in this particular embodiment the use of a HBDG instead of an ACDG is not necessarly the preferred embodiment, whereas in the case of a provided primary amide-containing compound a HBDG is the preferred embodiment over an ACDG.
EM3. In yet a further embodiment, of EM1, EM55 or EM2 said heteronucleophile is part of the *N-*ACDG amide, thus resulting in an intramolecular cleavage reaction;
EM4. In yet a further embodiment of EM1, EM55 or EM2, said *N*-ACDG amide is an *Nⁿ*-ACDGₙ amide that is cleaved 1 to n times in the presence of a metal catalyst and a heteronucleophile, after the n introductions of the ACDG in **1** to maximum n steps. For the defined maximum number of n cleavage steps, the maximum number of n different heteronucleophiles can be used. Said *Nⁿ*-ACDGₙ amide is a compound having a structure wherein the structural motive ACDG-NH-CO- can be found n times. When identifying the sum of the number of ACDG-NH-CO structural motives, a -CO- moiety can be used one or two times to identify this motive.
EM5. In yet a further embodiment of EM4, said *Nⁿ*-ACDGₙ amide is an *Nⁿ*-HBDGₙ amide. Said *Nⁿ*-HBDGₙ amide is a compound having a structure wherein the structural motive HBDG-NH-CO- can be found n times. When identifying the sum of the number of HBDG-NH-CO structural motives, a -CO-moiety can be used one or two times to identify this motive.
EM6. In yet a further embodiment EM4 or EM5, said *Nⁿ*-ACDGₙ amide is an *N,N'*-ACDG₂ amide having the general structure HBDG-NH-CO-X-CO-NH-HBDG, in which X can consists of any functional group or combinations of functional groups.
EM7. In a particular embodiment of the method according to EM6, said n HetNu (with n = 1 or 2) are part of the *Nⁿ*-ACDGₙ amide, thus resulting in an intramolecular cleavage reaction.
EM8. In a preferred embodiment of the method for cleavage of an *Nⁿ*-ACDGₙ amide, wherein n = 2 and said *Nⁿ*-ACDGₙ is an *N,N'*-ACDG₂, said an *Nⁿ*-ACDGₙ amide has the general structure ACDG-NH-CO-NH-ACDG.
EM9. In another embodiment of the method according to EM8, the *N,N'*-ACDG₂ amide (n = 2) can be named a new smart and safe phosgene analogue, since metal-catalyst activation is needed for reaction with said HetNu, and since by smart temperature regulation the number of cleavages (1 or 2) in one reaction step can be regulated, thus allowing the use of two different HetNu for two subsequent cleavage reactions via a two step protocol.
EM10. In a preferable embodiment of the methods according to embodiments EM1 to EM9, EM55, said ACDG is a substituted or unsubstituted pyridin-2-yl moiety.
EM11. In a more advantageous embodiment of the method according to EM10, said ACDG is an HBDG that is a C3-substituted pyridin-2-yl moiety, preferably an alkyl nicotinate or a 3-alkoxy pyridin-2-yl moiety.
EM12. In yet a more advantageous embodiment of the method according to EM11, said ACDG is a *tert*-butyl nicotinate.
EM13. In a preferable embodiment of the methods according to embodiments EM1 to EM9, EM55, said ACDG is a substituted or unsubstituted imidazol-2-yl moiety.
EM14. In a preferable embodiment of the method according to EM13, said ACDG is an imidazol-2-yl moiety that is a HBDG, preferentially being an alkyl 1*H*-imidazole-1-carboxylate.
EM15. In yet a more advantageous embodiment of the method according to EM14, said ACDG is a *tert*-butyl 1*H*-imidazole-1-carboxylate.
EM16. In a particular embodiment of the method according to anyone of EM1-15 or EM55, said metal catalyst contains one of the following metals: Al, Zn, Cu, Ni, Co, Fe, Mn, Pd, Ru, Rh, Pb, Zr, Sc, Ti, Cr, Ga, Ge, Se, Zr, Nb, Ag, Cd, In, Sn, Sb, Pb, Te, Ir, Re, Os, Pt, Au, W, Eu, La, Yb, Ta. Additionally, in the preferred embodiment of the method, said metal catalyst preferably contains Zn or Co as a metal.
EM17. In a yet another particular embodiment of the methods according to anyone of EM1 to EM16, EM55, said metal catalyst has valence 2 or 3.
EM18. In a preferred embodiment of the method according to anyone of EM1 to EM17, EM55, said nucleophile is a HetNu that is selected from the list comprising alcohols (ROH), thiols (RSH) and amines (RNH₂ or RNHR').
EM19. In yet another embodiment of the method according to anyone EM4 to EM9, at least 2 and maximum 3 of said n heteronucleophiles are part of one molecule.
EM20. In a preferred embodiment of the method according to anyone of EM1 to EM12, EM55, said directing group is a *tert*-butyl nicotinate; said metal catalyst is Zn(OAc)₂ or a hydrate or solvate thereof and said HetNu is an alcohol (ROH) or an amine (RNH₂ or RNHR').
EM21. In another preferred embodiment of the method according to EM1 to EM9, EM55, said directing group is *tert*-butyl 1*H*-imidazole-1-carboxylate; said metal catalyst is Zn(OAc)₂ or a hydrate or solvate thereof and said heteronucleophile is an alcohol (ROH) or an amine (RNH₂ or RNHR').
EM22. In one embodiment of the method according to anyone of the previous embodiments EM1 to EM2, EM55, said heteronucleophile has, caused by steric or electronic properties, unfavorable nucleophilic properties, leading to the embodiment of said method at temperatures ranging from 40 to 160 °C, with 60 to 160 °C being the preferred embodiment. In this embodiment, said heternucleophiles can for example be selected from the list comprising, but to limited to, the following nucleophiles: tertiary alcohols, tertiairy amines, secondary cyclic or acyclic alcohols or amines that show branching in one or both beta-positions of the hydroxyl function, (substituted) phenols and (substituted) anilines bearing an electron-withdrawing group in ortho- or para-position, given the fact that the indicated tertiary or secondary alcohol or amine function is the heteronucleophilic moiety that attacks the carbonyl moiety of the *Nⁿ*-ACDGₙ amide (preferable an *Nⁿ*-HBDGₙ amide) and not a(nother) heteronucleophilic entity that is present in said HetNu.
EM23. In another embodiment of the method according to anyone of EM1-EM21, EM55, said heteronucleophile has no sterically or electronically challenging properties and wherein the reaction temperature is room temperature to 100 °C.
EM24. In a particular embodiment of the method according to anyone of the previous embodiments EM1 to EM23, EM55, said to be cleaved *N*-ACDG amide is immobilized on a solid phase resin before the functionalization via the attack of the HetNu.
EM25. In another embodiment of EM24, said HetNu is part of the *N*-ACDG amide, thus resulting in an intramolecular cleavage reaction.
EM26. In a particular embodiment of the method according to EM24 to EM25, the solid phase resin is a polystyrene, polyacrylamide, polyethyleneglycol, cellulose, polyethylene, polyester, latex, polyamide, polydimethylacrylamide, copolymer polyethyleneglycol-polystrene, copolymer polyethyleneglycol-polyacrylamide or one of their derivatives.
EM27. In a particular embodiment of the method according to anyone of EM1 to EM26, EM55 said method is used for the synthesis of amide-containing compounds such as depicted in formula (I), esters, thioesters, carbonates, thiocarbonates and thiocarbamates. In another particular embodiment of the method according to anyone of EM1 to EM23, EM55 said method is used for the synthesis of polycarbonates, polyurea and polycarbamates.
EM28. In yet another embodiment of the methods according to anyone of EM1 to EM27, EM55, said ACDG is recuperated as an ACDG-NH₂ that can be used as a starting material or converted to a starting material for the synthesis of the *Nₙ*-ACDGₙ amide.
EM29. In a particular embodiment of the application, the *N*-ACDG amide for use according to anyone of EM1 to EM10, EM16 to EM19, EM22 to EM28, EM55, is selected from the following list: N-(pyridin-2-yl)amide, *N*-(n-C₁₋₁₀ alkoxypyridin-2-yl)amide; with n = 3-6 and alkoxy being optionally substituted, *N*-(n-halopyridin-2-yl)amide; with n = 3-6, C₁₋₁₀ alkyl n-amidonicotinate; with n = 2 or 6 and alkyl being optionally substituted, *N*-(n-nitropyridin-2-yl)amide; with n = 3-6, *N*-(n-cyanopyridin-2-yl)amide; with n = 3-6; *N*-(n-trifluoromethylpyridin-2-yl)amide; with n = 3-6; *N*-(n-C₁₋₁₀ alkylpyridin-2-yl)amide; with n = 3-6 and alkyl being optionally substituted, *N*-(n-arylpyridin-2-yl)amide with n = 3-6 and aryl being optionally substituted; *N*-(n-heteroarylpyridin-2-yl)amide; with n = 3-6 and heteroaryl being optionally substituted, *N*-(n-(C₁₋₁₀ alkoxymethyl)pyridin-2-yl)amide; with n = 3-6 and C₁₋₁₀ alkoxymethyl being optionally substituted, *N*-(n-(di-C₁₋₁₀alkylamino)pyridin-2-yl)amide; with n = 3-6 and alkyl being optionally substituted, *N*-(n-(amino C₁₋₁₀ alkyl)pyridin-2-yl)amide; with n = 3-6 and alkyl being optionally substituted; *S*-C₁₋₁₀ alkyl 2-benzamidopyridine-3-carbothioate; with alkyl being optionally substituted, *N*-(n-(C₁₋₁₀ alkylthio C₁₋₁₀ alkyl)pyridin-2-yl)amide; with n = 3-6 and alkyl being optionally substituted, 2-amido-*N*-C₁₋₁₀ alkylnicotinamide; with alkyl being optionally substituted; *N*-(n-C₁₋₁₀ alkylcarbonylpyridin-2-yl)amide; with n = 3-6 and alkyl being optionally substituted; *N*-(1-oxo-3,4-dihydro-1*H*-pyrano[3,4-c]pyridin-8-yl)amide, *N*-(8-oxo-5,6,7,8-tetrahydroisoquinolin-1-yl)amide, N-(8-oxo-5,6,7,8-tetrahydro-2,7-naphthyridin-1-yl)amide, *N*-(1-oxo-3,4-dihydro-1*H*-thiopyrano[3,4-*c*]pyridin-8-yl)amide, *N*-(3,4-dihydro-2*H*-pyrano[2,3-*c*]pyridin-8-yl)amide, *N*-(7-oxo-6,7-dihydro-5*H-*cyclopenta[*c*]pyridin-1-yl)amide, *N*-(3-oxo-1,3-dihydrofuro[3,4-*c*]pyridin-4-yl)amide, *N*-(3-oxo-1,3-dihydrothieno[3,4-c]pyridin-4-yl)amide, *N*-(3-oxo-2,3-dihydro-1*H*-pyrrolo[3,4-c]pyridin-4-yl)amide, *N-*(2,3-dihydrofuro[2,3-c]pyridin-7-yl)amide. With 'amide' being a moiety of the general structure according to formula (I). 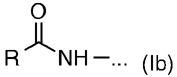 wherein R is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀alkylamino, C₁₋₁₀dialkylamino, C₁₋₁₀ alkyl aryl amino, C₁₋₁₀ alkyl heteroaryl amino, heteroaryl arylamino, diheteroarylamino, diarylamino, heteroaryl arylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, or heteroarylamino.
EM30. In a particular embodiment of the application, an *N*-ACDG amide wherein the ACDG is a pyridin-2-yl directing group attached to the *N*-atom of said amide according to formula (IIa), or a tautomer, isomer, salt, hydrate or solvate thereof; can be used according to anyone of EM1 to EM10, EM16 to EM19, EM22 to EM28, EM55. 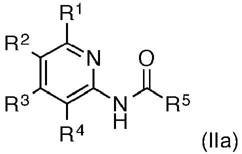 wherein
   R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, nitro, halo, hydroxyl, amino, thio, acylamino, C₁₋₁₀ alkoxy, aryloxy, heteroaryloxy, C₁₋₁₀alkylthio, C₁₋₁₀alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy.
   R⁴ is hydrogen, halo, nitro, cyano, trifluoromethyl, aryl, heteroaryl, C₁₋₁₀ alkyl, C₁₋₁₀ alkenyl, C₁₋₁₀ alkynyl, arylalkyl or heteroarylalkyl.
   or R⁴ is XR⁶ wherin X is -S-, -O-, -N-, -NH-, -C=O-, -C=N-R⁷ or -C=S-,
   R⁶ is hydrogen, aryl, heteroaryl, heteroarylalkyl, arylalkyl, C₁₋₂₅ alkyl, C₁₋₂₅ alkenyl, C₁₋₂₅ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy or arylcarbonyl.
   R⁷ is hydrogen, aryl, heteroaryl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀alkylthio, cyano, C₁₋₁₀alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino or heteroarylamino.
   R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, heteroaryl aryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino or heteroarylamino.
EM31. In another embodiment, an *N*-ACDG amide wherein the ACDG is a pyridin-2-yl directing group attached to the *N*-atom of said amide according to formula (IIb), or a tautomer, isomer, salt, hydrate or solvate thereof can be described for use according to anyone of EM1 to EM10, EM16 to EM19, EM22 to EM28, EM55. 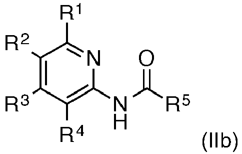 wherein
   R¹ is hydrogen.
   R² is trifluoromethyl, C₁₋₁₀ alkoxycarbonyl, C₂₋₁₀ alkyl, aryl. Aryl is an optionally substituted aryl or heteroaryl group.
   R³ is hydrogen,
   R⁴ is hydrogen, fluoro, chloro, trifluoromethyl, C₁₋₁₀ alkoxy, C₁₋₁₀ alkoxycarbonyl, C₁₋₁₀ alkylcarbonyl, *N,N'* C₁₋₅ dialkyl carboxamide or C₁₋₁₀ alkyl.
   R⁵ is a hydrogen, aryl, heteroaryl, heteroarylalkyl, arylalkyl, C₁₋₁₀ alkyl, C₁₋₁₀ alkenyl, C₁₋₁₀ alkynyl, or a pharmaceutically acceptable salt thereof, or an enantiomer, or a stereoisomeric form thereof, or a solvate thereof, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino or heteroarylamino.
   At least 1 substituent from R² and R⁴ is hydrogen, but not both substituents from R² and R⁴ are hydrogen.
EM32. In a particular embodiment of EM29, said amide is a peptide backbone (n >1) of the general structure Pg-(AAⁱ)ₙ-NH- with Pg a nitrogen protecting group and AA^{m} = a peptidogenic or non-peptidogenic amino acid, n = i = 1 to 10; AA¹, AA²,..., AAⁱ, AAⁱ⁺¹,... AA^{m} with m = 1 to 10.
EM33. In yet another embodiment, a compound of the general structure *N*-HBDG amide wherein the HBDG is an *N*-(3-C₁₋₁₀ alkoxy pyridin-2-yl) directing group attached to the *N*-atom of said amide according to formula (IIc), or a tautomer, isomer, salt, hydrate, or solvate thereof, is used according to anyone of EM1 to EM11, EM16 to EM20, EM22 to EM28, EM55. 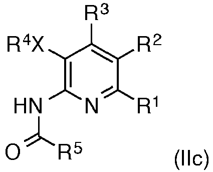 wherein
   X is O, NR⁶, or S.
   R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀alkylthio, C₁₋₁₀alkylamino, C₁₋₁₀ dialkylamino, C₁₋₁₀ alkoxycarbonyl, C₁₋₁₀ alkyl esters, C₁₋₁₀alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy.
   R⁴ and and R⁶ are each independently selected from the list comprising: hydrogen, C₁₋₁₀ alkyl, C₁₋₂₅ alkenyl, C₁₋₂₅ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀alkylthio, cyano, C₁₋₁₀alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀alkyl heteroaryl amino, diheteroarylamino, diarylamino, aryl heteroarylamino, arylthio, heteroarylthio, aryloxy, heteroaryloxy.
   R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, heteroaryl aryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
EM34. In yet another embodiment, a compound of the general structure *N*-HBDG amide wherein the HBDG is an *N*-(3-C₁₋₁₀ alkoxymethyl pyridin-2-yl) directing group attached to the *N*-atom of said amide according to formula (IId), or a tautomer, isomer, salt, hydrate, or solvate thereof, is used according to anyone of EM1 to EM11, EM16 to EM19, EM22 to EM28, EM55. 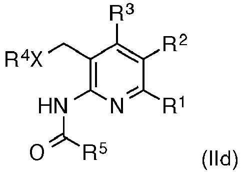 wherein
   X is O, NR⁶, or S.
   R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, nitro, halo, hydroxyl, thio, amino, acylamino, C₁₋₁₀ alkoxy, aryloxy, heteroaryloxy, C₁₋₁₀alkylthio, C₁₋₁₀alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroaylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl hetroarylamino, arylthio, heteroarylthio, aryloxy, heteroaryloxy.
   R⁴ and R⁶ are each independently selected from the list comprising: is hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀alkylthio, cyano, C₁₋₁₀alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀alkyl heteroaryl amino, diheteroarylamino, diarylamino, aryl heteroarylamino, arylthio, heteroarylthio, aryloxy, heteroaryloxy.
   R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀alkoxy, C₁₋₁₀alkylthio, C₁₋₁₀alkylamino, C₁₋₁₀dialkylamino, C₁₋₁₀alkyl aryl amino, C₁₋₁₀alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀alkyl, trifluoromethyl, C₁₋₁₀alkoxy, C₁₋₁₀ alkoxycarbonyl, trifluoromethoxy, C₁₋₁₀alkylthio, cyano, C₁₋₁₀alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
EM35. In yet another embodiment, a compound of the general structure *N*-HBDG amide wherein the HBDG is a C₁₋₁₀ alkyl nicotinate directing group attached to the *N*-atom of said amide according to formula (IIe) or a tautomer, isomer, salt, hydrate, or solvate thereof, is used according to anyone of EM1 to EM11, EM16 to EM19, EM22 to EM28, EM55. 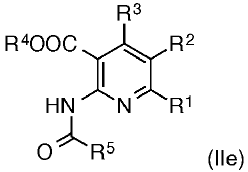 wherein
   R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, nitro, halo, hydroxyl, amino, acylamino, C₁₋₁₀ alkoxy, aryloxy, heteroaryloxy, C₁₋₁₀alkylthio, C₁₋₁₀alkylamino, C₁₋₁₀ dialkylamino, C₁₋₁₀ alkoxycarbonyl, C₁₋₁₀alkyl esters, C₁₋₁₀alkyl aryl amino, C₁₋₁₀alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl diarylamino, diheteroarylamino, aryl heteroarylamino, arylthio, heteroarylthio, aryloxy, heteroaryloxy.
   R⁴ is a hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroarylamino, diarylamino, diheteroarylamino, aryl heteroarylamino, arylthio, heteroarylthio, aryloxy or heteroaryloxy.
   R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
EM36. In yet another embodiment of EM30 to EM35, -NH-CO-R⁵- is a peptide backbone (n >1) of the general structure Pg-(AAⁱ)ₙ-NH- with Pg a nitrogen protecting group and AA^{m} = a peptidogenic or non-peptidogenic amino acid, n = i = 1 to 10; AA¹, AA²,..., AAⁱ, AAⁱ⁺¹,... AA^{m} with m = 1 to 10.
EM37. A compound of the general structure *N*-HBDG amide wherein the HBDG is a *tert*-butyl nicotinate directing group attached to the *N*-atom of said amide according to formula (IIf), or a tautomer, isomer, salt, hydrate, or solvate thereof. 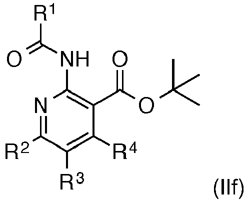 wherein
   R¹ is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarnbylaminoalkyl, aryloxy, arylcarbonyl, arylamino, diarylamino, heteroarylamino, diheteroarylamino, heteroaryl aryl amino.
   R², R³ and R⁴ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀alkylthio, C₁₋₁₀alkylamino, C₁₋₁₀ dialkylamino, C₁₋₁₀ alkoxycarbonyl, C₁₋₁₀ alkyl esters, C₁₋₁₀alkyl aryl amino, C₁₋₁₀ alkyl heteroaryl amino, diheteroarylamino, diarylamino, heteroaryl arylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM38. A compound of the general formula (IIg) for the use in the process according to anyone of EM1 to EM10, EM16 to EM19, EM22 to EM28, EM55. 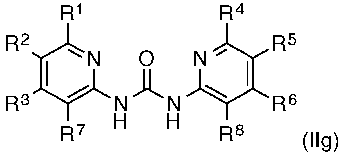 wherein
   R¹, R², R³, R⁴, R⁵, R⁶, R⁷ and R⁸ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM39. A compound wherein said compound has the structure following formula (IIh), or a tautomer, isomer, salt, hydrate, or solvate thereof. 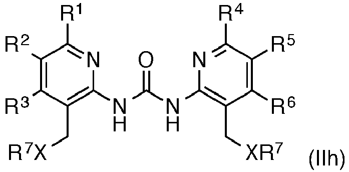 wherein
   R¹, R², R³, R⁴, R⁵, and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is O, NR⁸, or S.
   R⁷ is hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R⁸ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM40. A compound wherein said compound has the structure following formula (IIi), or a tautomer, isomer, salt, hydrate, or solvate thereof. 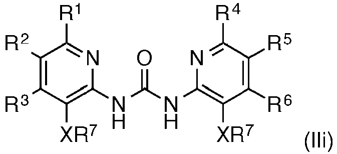 wherein
   R¹, R², R³, R⁴, R⁵, and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is O, NR⁸, or S.
   R⁷ is hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R⁸ is hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM41. A compound wherein said compound has the structure following formula (IIj), or a tautomer, isomer, salt, hydrate, or solvate thereof. 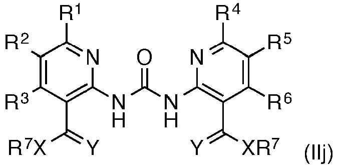 wherein
   R¹, R², R³, R⁴, R⁵, and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X = O, NR⁸, or S.
   R⁷ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R⁸ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
   Y is O, S or NR⁹.
   R⁹ = hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM42. A compound wherein said compound has the structure following formula (Ilk), or a tautomer, isomer, salt, hydrate, or solvate thereof. 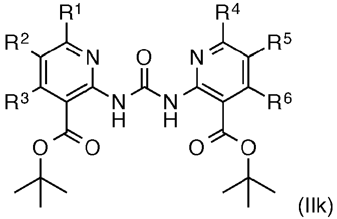 wherein
   R¹, R², R³, R⁴, R⁵, and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM43. A compound of the general formula (III) for the use in the process according to anyone of the embodiments EM1 to EM10, EM16 to EM19, EM22 to EM28, EM55. 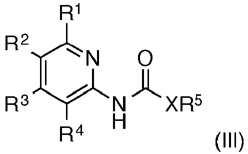 wherein
   R¹, R², R³, R⁴ are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, hydroxyl, amino, thio, acylamino, C₁₋₁₀ alkoxy, aryloxy, heteroaryloxy, C₁₋₁₀alkylthio, C₁₋₁₀alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is O, NH, NR⁶ or S.
   R⁶ is aryl, heteroaryl, heteroarylalkyl, arylalkyl, C₁₋₂₅ alkyl, C₁₋₂₅ alkenyl, C₁₋₂₅ alkynyl; wherein said aryl, heteroaryl or alkyl is optionally substituted with one to 10 substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
   R⁵ is hydrogen, C₁₋₁₀ alkyl, C₁₋₂₅ alkenyl, C₁₋₂₅ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM44. A compound wherein said compound has the structure according to formula (IIm) or a tautomer, isomer, salt, hydrate, or solvate thereof. 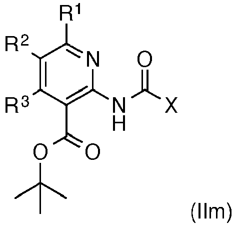 wherein
   R¹, R², and R³ are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, hydroxyl, amino, thio, acylamino, C₁₋₁₀ alkoxy, aryloxy, heteroaryloxy, C₁₋₁₀ alkylthio, C₁₋₁₀ alkylamino, C₁₋₁₀dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is OR⁴, NHR⁴, NR⁴R⁵ or SR⁴.
   R⁴ is hydrogen, C₁₋₁₀ alkyl, C₁₋₂₅ alkenyl, C₁₋₂₅ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀ alkyl heteroaryl amino, arylthio, heteroarylthio, aryloxy, or heteroaryloxy, diarylamino, diheteroarylamino, aryl heteroarylamino.
   R⁵ is aryl, heteroaryl, heteroarylalkyl, arylalkyl, C₁₋₂₅ alkyl, C₁₋₂₅ alkenyl, C₁₋₂₅ alkynyl; wherein said aryl, heteroaryl or alkyl is optionally substituted with one to 10 substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
EM45. A *N*-ACDG amide wherein the ACDG is an imidazol-2-yl directing group attached to the N-atom of said amide according to formula (IIIa) or a tautomer, isomer, salt, hydrate or solvate thereof; for use according to anyone of the embodiments EM1 to EM9, EM13, EM16 to EM20, EM22 to EM28, EM55. 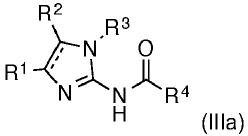 wherein
   R¹ and R² are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R³ is hydrogen, aryl, heteroaryl, heteroarylalkyl, arylalkyl, C₁₋₂₅ alkyl, C₁₋₁₀ alkenyl, C₁₋₂₅ alkynyl, arylalkyl, heteroarylalkyl
   or R³ is XR⁵ wherin X is -C=O-, -C=N-R⁶ or -C=S-.
   R⁵ is hydrogen, aryl, heteroaryl, heteroarylalkyl, arylalkyl, C₁₋₂₅ alkyl, C₁₋₂₅ alkenyl, C₁₋₂₅ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀alkyl heteroaryl amino, diarylamino, diheteroarylamino, aryl heteroarylamino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
   R⁶ is hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
   R⁴ is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM46. A compound of the general structure according to formula (IIIb), or a tautomer, isomer, salt, hydrate, or solvate thereof, for use according to anyone of embodiments EM1 to EM9, EM13 to EM14, EM16 to EM20, EM22 to EM28, EM55. 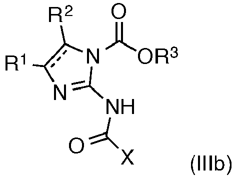 wherein
   R¹ and R² are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀alkyl heteroarylamino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀alkyl heteroaryl amino, arylthio, heteroarylthio, aryloxy, heteroaryloxy, diarylamino, diheteroarylamino, aryl heteroarylamino.
   R³ is a hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroarylamino, arylthio, heteroarylthio, aryloxy or heteroaryloxy, diarylamino, diheteroarylamino, aryl heteroarylamino.
   X is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM47. A compound of the general structure according to formula (IIIc), or a tautomer, isomer, salt, hydrate, or solvate thereof, for use according to anyone of embodiments EM1 to EM9, EM13 to EM20, EM22 to EM28, EM55. 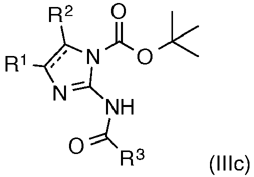 wherein
   R¹ and R² are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R³ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM48. The compound according to anyone of EM44 to EM46 wherein -NH-CO-"R"- is an amino acid (n = 1) or peptide backbone (n >1) of the general structure Pg-(AAⁱ)ₙ-NH- with Pg a nitrogen protecting group and AA^{m} = a peptidogenic or non-peptidogenic amino acid, n = i = 1 to 10; AA¹, AA²,..., AA¹, AAⁱ⁺¹,... AA^{m} with m = 1 to 10.
EM49. A compound according to the formula (IIId) or a tautomer, isomer, salt, hydrate, or solvate thereof. 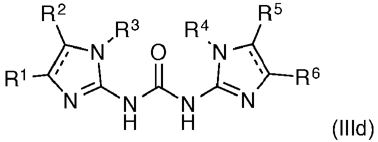 wherein
   R¹, R², R⁵ and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy.
   R³ and R⁴ are each independently selected from the list comprising hydrogen, aryl, heteroaryl, heteroarylalkyl, arylalkyl, C₁₋₂₅ alkyl, C₁₋₁₀ alkenyl, C₁₋₂₅ alkynyl, arylalkyl, heteroarylalkyl or R³ and/or R⁴ is XR⁷ wherein X is -C=O-, -C=N-R⁸, -C=S-,
   R⁷ is hydrogen, aryl, heteroaryl, heteroarylalkyl, arylalkyl, C₁₋₁₀ alkyl, C₁₋₁₀ alkenyl, C₁₋₁₀ alkynyl, C₁₋₁₀ alkoxy, C₁₋₁₀ alkylthio, C₁₋₁₀ alkylamino, C₁₋₁₀ dialkylamino, C₁₋₁₀alkyl aryl amino, C₁₋₁₀ alkyl heteroaryl amino, diarylamino, diheteroarylamino, aryl heteroarylamino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
   R⁸ is hydrogen, aryl, heteroaryl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino. R³ and R⁴ are not both hydrogen.
EM50. A compound according to the formula (IIIe), or a tautomer, isomer, salt, hydrate, or solvate thereof. 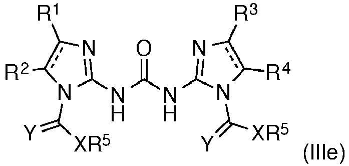 wherein
   R¹, R², R³ and R⁴ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀alkyl aryl amino, C₁₋₁₀alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is O, NR⁶, or S.
   R⁶ is hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   R⁵ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   Y = O, S or NR⁷.
   R⁷ = hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM51. A compound according to the formula (IIIe'), or a tautomer, isomer, salt, hydrate, or solvate thereof. 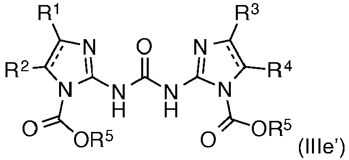 wherein
   R¹, R², R³ and R⁴ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀alkyl aryl amino, C₁₋₁₀alkyl heteroaryl amino, arylthio, heteroarylthio, aryloxy, heteroaryloxy, diarylamino, diheteroarylamino, aryl heteroarylamino.
   R⁵ is a hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroarylamino, diarylamino, diheteroarylamino, aryl heteroarylamino, arylthio, heteroarylthio, aryloxy or heteroaryloxy.
EM52. A compound according to the formula (IIIe"), or a tautomer, isomer, salt, hydrate, or solvate thereof. 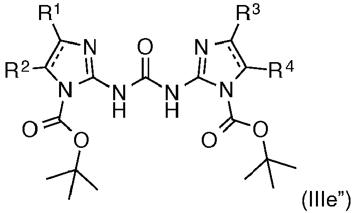 wherein
   R¹, R², R³ and R⁴ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
EM53. A compound according to the formula (IIIf), or a tautomer, isomer, salt, hydrate, or solvate thereof, for use according to anyone of embodiments EM1 to EM 9, EM13 to EM14, EM16 to EM20, EM22 to EM28. 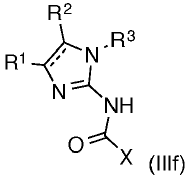 wherein
   R¹ and R² are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀alkyl aryl amino, C₁₋₁₀alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is O, NR⁴ or S.
   R⁴ = aryl, heteroaryl, heteroarylalkyl, arylalkyl, C₁₋₂₅ alkyl, C₁₋₂₅ alkenyl, C₁₋₂₅ alkynyl; wherein said aryl, heteroaryl or alkyl is optionally substituted with one to 10 substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
   R³ is hydrogen, aryl, heteroaryl, heteroarylalkyl, arylalkyl, C₁₋₂₅ alkyl, C₁₋₁₀ alkenyl, C₁₋₂₅ alkynyl, arylalkyl, heteroarylalkyl
   or R³ is XR⁵ wherin X is -C=O-, -C=N-R⁶, -C=S-,
   R⁵ is hydrogen, aryl, heteroaryl, heteroarylalkyl, arylalkyl, C₁₋₂₅ alkyl, C₁₋₂₅ alkenyl, C₁₋₂₅ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
   R⁶ is hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
   R⁴ is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
EM54. A compound according to the formula (IIIg), or a tautomer, isomer, salt, hydrate, or solvate thereof, for use according to anyone of the embodiments EM1 to EM9, EM13 to EM20, EM22 to EM28, EM55. 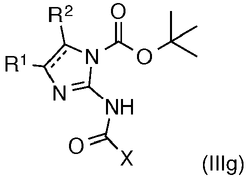 wherein
   R¹ and R² are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.
   X is OR³, NR³R⁴ or SR⁴.
   R³ is hydrogen, C₁₋₁₀ alkyl, C₁₋₂₅ alkenyl, C₁₋₂₅ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, or heteroaryloxy, diarylamino, diheteroarylamino, aryl heteroarylamino.
   R⁴ is aryl, heteroaryl, heteroarylalkyl, arylalkyl, C₁₋₂₅ alkyl, C₁₋₂₅ alkenyl, C₁₋₂₅ alkynyl; wherein said aryl, heteroaryl or alkyl is optionally substituted with one to 10 substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.
EM55. A method for the catalytic cleavage of amide-containing compounds; said method comprising the steps of:
   - Providing an ACDG-precursor;
   - Converting said ACDG-precursor compound into an ACDG-containing (amide-cleaving directing group-containing) compound, by introducing an amide-cleaving directing group (ACDG), according to the formula (Ia), resulting in the formation of an N-ACDG amide of formula ACDG-NH-CO-, and this by the reaction of said ACDG-precursor with an ACDG-introducing compound in the presence or absence of catalyst(s), ligand(s), reactant(s), reagent(s) and/or solvent(s) to an ACDG-containing (amide-cleaving directing group-containing) compound of the formula ACDG-NH-CO-) 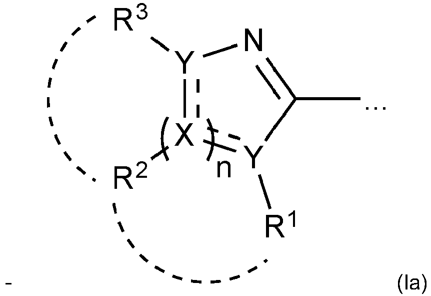 wherein
      X is selected from C, N, O or S.
      Y is selected from C, N or S; or Y = O or S and R¹ is absent
      n is selected from 1, 2, 3, 4, 5, 6, 7 or 8.
      R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein each of said aryl, heteroaryl or alkyl is optionally and independently substituted with from 1 to 5 substituents selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy;
      - - - is an optional chemical bond; if one or more optional chemical bonds are present, a polycyclic structure is meant;
   - cleaving said ACDG-containing compound (namely the *N*-ACDG amide) in the presence of a (hetero)nucleophile; and a metal catalyst according to formula Ib, or a salt, solvate or hydrate thereof

      M_{y}Lₓ(Ib)

      wherein
      M is a metal or semi-metal; preferably Zn, Mn, Ni or Co; most preferably Zn;
      L represents an organic or inorganic ligand; preferably a carboxylate ligand, most preferably an acetate ligand;
      x is selected from 0-30; and
      y is selected from 1, 2, 3 or 4; most preferably 1;
      said *N*-ACDG amide is the compound having the structure ACDG-NH-CO-, and within the scope of our definition of *N*-ACDG amide we include groups in which the structural unit ACDG-NHCO-is part of a larger group or structural entity, namely: urea, oxamide, carbamate, thiocarbamate; said (hetero)nucleophile may be comprised in said ACDG-containing compound;

### EXAMPLES

All NMR-spectra were measured at 30 °C, unless mentioned otherwise. m.p.: melting point. *t*Bu *nic*: *tert*-butyl nicotinate, py: pyridin-2-yl, TMB: 1,3,5-trimethoxybenzene.

### Example 1. Synthesis of amide-containing compounds bearing N-py directing groups

### Example 1.1. Synthesis of N-(pyridin-2-yl)benzamides 1

The following (C3- and C5-substituted) *N*-(pyridin-2-yl)benzamides **1** can be synthesised via catalytic amidation of the corresponding (C3- or C5-substituted) 2-halopyridine **2** (with halo = I, Br or Cl) (1.0-5.0 equiv) with benzamide **(3a)** (1.0-5.0 equiv) using a transition metal-source (1.0-50.0 mol%) like for example Pd(OAc)₂, Pd₂(dba)₃ or Cul, a ligand like for example XantPhos, 1,1'-bis(diphenylphosphino)ferrocene, 1,1'-bis(dicyclohexylphosphino)ferrocene or trans-N,N'-dimethylcyclohexane-1,2-diamine (1.0-50.0 mol%), a base (1.0-15.0 equiv) like for example Cs₂CO₃, KO*t*Bu or K₂CO₃ and an organic solvent like for example 1,4-dioxane or 2-MeTHF and a reaction temperature from room temperature to 140 °C, as depicted in the general Example Scheme E1 (Buchwald-Hartwig amidation reaction). 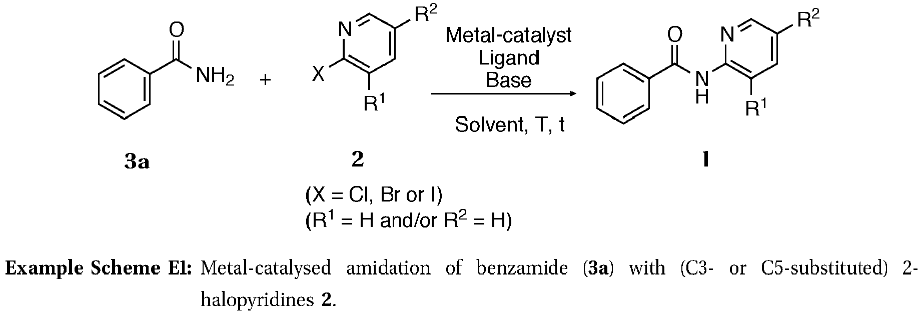 *N*-(Pyridin-2-yl)benzamide **(1a).** White crystals; m.p. 85 °C. ¹³C-NMR (100 MHz, CDCl₃): *δ*: 166.0, 151.8, 148.1, 138.6, 134.5, 132.4, 129.0, 127.4, 120.1, 114.4.
*N*-(3-Fluoropyridin-2-yl)benzamide **(1b).** White crystals; m.p. 99 °C. ¹³C-NMR (100 MHz, CDCl₃): *δ*: 165.7, 153.3, 150.7, 143.5 (d, *J* = 5.42 Hz), 140.4 (d, 1H, *J* = 12.79 Hz), 133.5, 132.3, 128.2 (d, *J* = 82.31 Hz), 124.8 (d, *J* = 17.96 Hz), 122.3 (d, *J* = 3.42 Hz).
*N*-(5-Fluoropyridin-2-yl)benzamide **(1c).** White crystals; m.p. 111 °C. ¹³C-NMR (100 MHz, CDCl₃): *δ*: 165.6 (d, *J* = 1.87 Hz), 156.3 (d, *J* = 253.49 Hz), 148.0 (d, *J* = 1.92 Hz), 135.3 (d, *J* = 25.88 Hz), 134.3, 132.2, 128.8, 127.3, 125.3 (d, *J* = 19.34 Hz), 115.2 (d, *J* = 4.04 Hz). *N*-(3-Chloropyridin-2-yl)benzamide **(1d).** White crystals; m.p. 88 °C. ¹³C-NMR (100 MHz, CDCl₃): *δ*: 164.9, 147.8, 147.0, 138.1, 134.2, 132.4, 128.9, 127.5, 122.6, 121.3.
*N*-(5-Chloropyridin-2-yl)benzamide **(1e).** White crystals; m.p. 124 °C. ¹³C-NMR (100 MHz, CDCl₃: *δ*: 165.6, 149.9, 146.6, 138.1, 134.0, 132.4, 128.9, 127.2, 126.9, 114.8.
Methyl 2-benzamidonicotinate **(1f).** White crystals; m.p. 100 °C. ¹³C-NMR (100 MHz, CDCl₃: *δ*: 167.8, 164.6, 153.8, 153.2, 140.0, 135.0, 132.4, 129.0, 127.8, 118.5, 111.6, 53.1.
Methyl 6-benzamidonicotinate **(1g).** Shiny yellow crystals; m.p. 157 °C. ¹³C-NMR (100 MHz, CDCl₃): *δ*: 165.7, 165.4, 154.5, 150.1, 139.9, 133.7, 132.7, 129.0, 127.3, 122.2, 113.0, 52.3.
Ethyl 2-benzamidonicotinate **(1h).** White crystals; m.p. 98 °C. ¹³C-NMR (100 MHz, CDCl₃: *δ*: 167.2, 164.4, 153.5, 153.1, 139.9, 134.9, 132.1, 128.8, 127.6, 118.3, 111.8, 62.1, 14.2.
Isopropyl 2-benzamidonicotinate **(1i).** White crystals; m.p 106 °C. ¹³C-NMR (100 MHz, CDCl₃: *δ*: 166.7, 164.4, 153.3, 153.1, 139.9, 134.9, 132.1, 128.8, 127.6, 118.3, 112.1, 70.0, 21.8.
*tert*-Butyl 2-benzamidonicotinate **(4a).** White crystals; m.p. 104 °C. ¹³C-NMR (100 MHz, CDCl₃: *δ*: 166.5, 164.5, 153.1, 153.0, 140.0, 135.0, 132.0, 128.8, 127.7, 118.2, 113.0, 83.5, 28.1.
*tert*-Butyl 6-benzamidonicotinate **(1j).** Light-yellow crystals; m.p. 112 °C. ¹³C-NMR (100 MHz, CDCl₃: *δ*: 166.4, 163.8, 154.7, 149.7, 139.6, 134.1, 132.3, 128.6, 127.6, 123.6, 113.2, 81.5, 28.1.
*N*-(3-Nitropyridin-2-yl)benzamide **(1k).** Brown crystals; m.p. 96 °C. ¹³C-NMR (100 MHz, CDCl₃: *δ*: 166.6, 154.0, 145.8, 134.7, 134.4, 133.6, 132.9, 129.0, 127.7, 119.5.
*N*-(5-Nitropyridin-2-yl)benzamide (**1l**). Light-yellow crystals; m.p. 166 °C. ¹³C-NMR (100 MHz, CDCl₃: *δ*: 165.8, 155.6, 144.8, 140.7, 134.1, 133.3, 133.1, 129.1, 127.4, 113.2.
*N*-(3-Cyanopyridin-2-yl)benzamide **(1m).** Light-yellow crystals; m.p. 205 °C. ¹³C-NMR (100 MHz, DMSO-d6): *δ*: 165.9, 152.6, 152.5, 142.5, 132.7, 132.5, 128.5, 128.1, 121.3, 115.7, 105.7.
*N*-(5-Cyanopyridin-2-yl)benzamide **(1n).** Brown crystals; m.p. 182 °C. ¹³C-NMR (100 MHz, CDCl₃: *δ*: 165.9, 154.2, 151.9, 141.9, 133.5, 133.2, 129.3, 127.5, 116.9, 113.9, 105.4.
*N*-(3-Trifluoromethylpyridin-2-yl)benzamide **(1o).** White crystals; m.p. 175 °C. ¹³C-NMR (100 MHz, CDCl₃): *δ*: 173.4, 152.6, 150.8, 137.1 (q, *J* = 4.29 Hz), 134.8, 132.5, 129.2, 128.5, 124.0 (d, *J* = 12.36 Hz), 123.3 (t, *J* = 35.21 Hz), 121.4.
*N*-(5-Trifluoromethylpyridin-2-yl)benzamide **(1p).** White crystals; m.p. 111 °C. ¹³C-NMR (100 MHz, CDCl₃): *δ*: 166.1, 154.2, 145.3 (q, *J* = 4.07 Hz), 135.9 (q, *J* = 3.36 Hz), 133.8, 132.7, 129.0, 127.5, 124.8, 122.5 (t, *J* = 33.5 Hz), 113.5.
*N*-(5-Phenylpyridin-2-yl)benzamide **(1q).** White crystals; m.p. 144 °C. ¹³C-NMR (100 MHz, CDCl₃: *δ*: 166.0, 150.8, 146.0, 137.3, 136.9, 134.4, 132.9, 132.2, 129.0, 128.8, 127.8, 127.4, 126.7, 114.1.
*N*-(3-Methylpyridin-2-yl)benzamide **(1r).** White crystals; m.p. 210 °C. ¹³C-NMR (100 MHz, CDCl₃: *δ*: 166.1, 149.9, 145.6, 140.1, 134.1, 132.1, 129.3, 128.6, 127.7, 121.8, 18.4.
*N*-(5-Methylpyridin-2-yl)benzamide **(1s).** White crystals; m.p. 98 °C. ¹³C-NMR (100 MHz, CDCl₃: *δ*: 165.64, 149.43, 147.78, 139.02, 134.47, 132.09, 129.30, 128.79, 127.20, 113.69, 17.84.
*N*-(3-Ethylpyridin-2-yl)benzamide **(1t).** White crystals; m.p. 106 °C. ¹³C-NMR (100 MHz, CDCl₃: *δ*: 165.8, 149.7, 147.0, 137.9, 135.5, 134.6, 132.1, 128.8, 127.3, 113.9, 25.5, 15.4.
*N*-(5-Ethylpyridin-2-yl)benzamide **(1u)..** White crystals; m.p. 70 °C. ¹³C-NMR (100 MHz, CDCl₃: *δ*: 165.9, 149.8, 147.1, 138.2, 135.8, 134.6, 132.3, 129.0, 127.4, 114.1, 25.7, 15.6.
*N*-(3-Methoxypyridin-2-yl)benzamide **(1v).** White crystals; m.p. 69 °C. ¹³C-NMR (100 MHz, CDCl₃: *δ*: 164.8, 145.5, 142.0, 139.8, 132.0, 127.5, 120.1, 117.6, 55.9.
*N*-(5-Methoxypyridin-2-yl)benzamide **(1w).** White crystals; m.p. 110 °C. ¹³C-NMR (100 MHz, CDCl₃: *δ*: 165.7, 153.0, 145.5, 134.7, 134.4, 132.1, 128.9, 127.4, 123.7, 114.9, 56.0.
*N*-(3-Isopropoxypyridin-2-yl)benzamide **(1x).** Beige crystals; m.p. 95 °C. ¹³C-NMR (100 MHz, CDCl₃: *δ*: 164.6, 143.3, 143.0, 139.8, 135.5, 132.0, 128.9, 127.4, 119.8, 119.6, 71.8, 22.2.

### Example 1.2. Synthesis of 1,3-di(pyridin-2-yl)urea (5a)

1,3-di(Pyridin-2-yl)urea **(5a)** was synthesised via the reaction of triphosgene (1.0 g, 3.37 mmol) with 2-aminopyridine **(6a)** (1.576 g, 16.74 mmol) in the presence of DMAP (2.44 g, 20.01 mmol) in CH₂Cl₂ (15.0 mL) at room temperature for 36 h. **5a** was equally synthesised via Fe(OH)₃@Fe₃O₄-catalysed (0.060 g, 0.052 mmol) reaction of 2-aminopyridine **(6a)** (0.376 g, 4.0 mmol) with urea **(3b)** (0.120 g, 2.0 mmol) in p-xylene at 140 °C for 2.5 days and obtained in 62% (0.266 g, 1.24 mmol) yield.²⁶ White crystals ; m.p. 175 °C. ¹³C-NMR (100 MHz, DMSO-d6): *δ*: 152.4, 151.9, 147.3, 138.4, 118.1, 112.4.

### Example 1.3. Synthesis of N-(pyridin-2-yl)carbamates 7

Phenethyl pyridin-2-ylcarbamate **(7a)** was obtained in 15% (0.40 g, 1.651 mmol) yield via the reaction of picolinamide **(3c)** (0.330 g, 2.70 mmol), iodobenzenediacetate (2.176 g, 6.76 mmol) and 2-phenylethanol **(8a)** (1.651 g, 13.51 mmol) in benzene (40.0 mL). White crystals, m.p. 131 °C. ¹³C-NMR (100 MHz, CDCl₃: *δ*: 153.5, 152.3, 147.6, 138.4, 137.7, 128.9, 128.6, 126.6, 118.5, 112.5, 65.6, 35.4.

### Example 2. Synthesis of amide-containing compounds bearing N-tBu nic directing groups

### Example 2.1. Synthesis of di-tert-butyl 2,2'[carbonylbis(azanediyl)]dinicotinate (5b)

di-*tert*-Butyl 2,2'-[carbonylbis(azanediyl)]dinicotinate **(5b)** was obtained in 24% (0.156 g, 0.376 mmol) yield via the reaction of triphosgene (0.190 g, 0.64 mmol) with *tert*-butyl 2-aminonicotinate **(6b)** (0.619 g, 3.19 mmol) in the presence of DMAP (0.467 g, 3.82 mmol) in CH₂Cl₂ (12.0 mL) at room temperature for 96 h. Colorless solid ; m.p. 133 °C. ¹H-NMR (400 MHz, CDCl₃: *δ*: 11.73 (br s, 2H), 8.54-8.51 (m, 2H), 8.23-8.20 (m, 2H), 7.03-6.99 (m, 2H), 1.60 (s, 18H).

### Example 2.2. Synthesis of tert-Butyl 3-[3-(tert-butoxycarbonyl)pyridin-2-yl]-2,4-dioxo-3,4-dihydro-2H-pyrido[1,2-a][1,3,5]triazine-9-carboxylate (9a)

*tert*-Butyl 3-[3-(*tert*-butoxycarbonyl)pyridin-2-yl]-2,4-dioxo-3,4-dihydro-2*H*-pyrido[1,2-*a*][1,3,5]-triazine-9-carboxylate **(9a)** was obtained in 68% (3.17 g, 7.20 mmol) yield via the reaction of triphosgene (2.10 g, 7.08 mmol) with *tert*-butyl 2-aminonicotinate **(6b)** (6.87 g, 35.4 mmol) in the presence of DMAP (5.19 g, 42.5 mmol) in CH₂Cl₂ (15.0 mL) at room temperature for 72 h. Light-pink crystals ; m.p. 143 °C. ¹H-NMR (400 MHz, DMSO-d6): *δ*: 8.84 (dd, 1H, *J* = 4.71 Hz, 1.76 Hz), 8.57 (dd, 1H, *J* = 7.07 Hz, 1.43 Hz), 8.48 (dd, 1H, *J* = 7.80 Hz, 1.91 Hz), 8.15 (dd, 1H, *J* = 6.97 Hz, 1.91 Hz), 7.75 (dd, 1H, *J* = 7.48 Hz, 4.63 Hz), 7.05 (t, 1H, *J* = 7.07 Hz), 1.56 (s, 9H), 1.33 (s, 9H).

### Example 2.3. Synthesis of tert-butyl 2-amidonicotinates 4

Based on the general conditions for synthesis of *tert*-butyl 2-amidonicotinates **4,** as depicted in Example Scheme E2, the following examples of the synthesis of *tert*-butyl 2-amidonicotinates **4** can be listed:

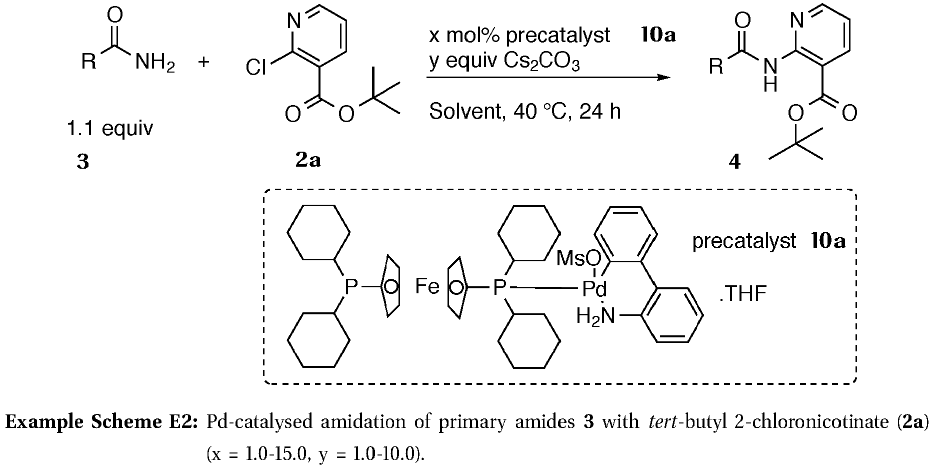

Using the following conditions: 5.0 mol% **10a,** 1.0 equiv **2a,** 1.1 equiv amide **3,** 1.2 equiv Cs₂CO₃, 2-MeTHF (anhydrous), 40 °C, 24 h.
*tert*-Butyl 2-benzamidonicotinate **(4a)** was obtained in 97% (1.45 g, 4.86 mmol) yield. White crystals; m.p 104 °C. ¹³C-NMR (100 MHz, DMSO-d6): *δ*: 165.6, 165.0, 150.6, 149.1, 138.8, 133.5, 132.1, 128.4, 127.9, 122.4, 120.4, 81.2, 27.6.
*tert*-Butyl 2-(4-fluorobenzamido)nicotinate **(4b)** was obtained in 69% (1.49 g, 4.71 mmol) yield. White crystals; m.p. 126 °C. ¹³C-NMR (100 MHz, CDCl₃: *δ*: 166.8, 166.6, 164.1, 163.5, 153.3 (d, *J* = 4.76 Hz), 140.2, 131.4, (d, *J* = 3.10 Hz), 130.3 (d, *J* = 9.00 Hz), 118.5, 116.0 (d, *J* = 21.90 Hz), 113.2, 83.8, 28.3.
*tert*-Butyl 2-(3-chlorobenzamido)nicotinate **(4c)** was obtained in 83% (1.26 g, 3.78 mmol) yield. White crystals; m.p. 108 °C. ¹³C-NMR (100 MHz, CDCl₃: *δ*: 166.5, 163.1, 153.0, 152.9, 140.1, 136.8, 135.0, 132.1, 130.1, 128.2, 125.4, 118.5, 113.1, 83.8, 28.1.
*tert*-Butyl 2-(2-chlorobenzamido)nicotinate **(4d)** was obtained in 78% (1.18 g, 3.55 mmol). White crystals; m.p. 95 °C. ¹³C-NMR (100 MHz, CDCl₃: *δ*: 165.8, 164.6, 152.6, 152.2, 140.0, 136.4, 131.3, 131.0, 130.3, 129.5, 127.1, 118.8, 113.6, 83.5, 28.1.
*tert*-Butyl 2-[4-(methoxycarbonyl)benzamido]nicotinate **(4e)** was obtained in 35% (0,33 g, 0,91 mmol) yield. White crystals; m.p. 188 °C. ¹³C-NMR (100 MHz, CDCl₃: *δ*: 166.6, 166.3, 163.6, 153.0, 152.9, 140.1, 138.8, 133.2, 130.0, 127.7, 118.6, 113.1, 83.8, 52.4, 28.1.
*tert*-Butyl 2-[4-(trifluoromethyl)benzamido]nicotinate **(4f)** was obtained in 87% (1.60 g, 4.37 mmol) yield. White crystals; m.p. 156 °C. ¹³C-NMR (100 MHz, DMSO-d6): *δ*: 166.9, 164.6, 150.6, 148.7, 139.0, 137.4, 131.9 (q, J = 31.61 Hz), 128.3, 125.5 (q, J = 3.77 Hz), 123.9 (q, J = 274.55 Hz), 122.7, 120.8, 81.2, 27.6.
*tert*-Butyl 2-(4-methylbenzamido)nicotinate **(4g)** was obtained in 92% (1.45 g, 4.86 mmol). White crystals; m.p. 127 °C. ¹³C-NMR (100 MHz, CDCl₃): *δ*: 166.5, 164.4, 153.2, 152.9, 142.6, 139.9, 132.2, 129.4, 127.7, 118.0, 113.0, 83.4, 28.1, 21.5.
*tert*-Butyl 2-(4-methoxybenzamido)nicotinate **(4h)** was obtained in 85% (1.40 g, 4.26 mmol) yield. White crystals; m.p. 148 °C. ¹³C-NMR (100 MHz, CDCl₃): *δ*: 166.6, 164.0, 162.7, 153.3, 153.0, 140.0, 129.7, 127.3, 118.0, 114.0, 112.9, 83.4, 55.5, 28.2.
*tert*-Butyl 2-(2-methoxybenzamido)nicotinate **(4i)** was obtained in 98% (1.61 g, 4.90 mmol) yield. White crystals; m.p. 103 °C. ¹³C-NMR (100 MHz, CDCl₃): *δ*: 165.5, 163.5, 157.5, 152.3, 152.1, 139.6, 133.2, 133.0, 122.8, 121.1, 118.3, 115.5, 111.2, 82.3, 55.5, 28.1.
*tert*-Butyl 2-(2-phenylacetamido)nicotinate **(4j)** was obtained in 48% (0.750 g, 2.41 mmol) yield. White crystals; m.p. 84 °C. ¹³C-NMR (100 MHz, CDCl₃: *δ*: 169.6, 165.8, 152.4, 152.4, 139.8, 134.4, 129.7, 128.7, 127.1, 118.1, 113.0, 83.2, 45.8, 28.1.
*tert*-Butyl 2-(2-phenylbutanamido)nicotinate **(4k)** was obtained in 99% (1.70 g, 4.99 mmol) yield. White crystals; m.p. 106 °C. ¹³C-NMR (100 MHz, CDCl₃): *δ*: 171.5, 166.0, 152.6, 152.5, 139.7,139.4, 128.6, 128.2, 127.1, 118.0, 112.9, 83.1, 57.0, 28.0, 26.2, 12.2.
*tert*-Butyl 2-(nicotinamido)nicotinate (**4l**) was obtained in 45% (0.61 g, 2.25 mmol) yield. White crystals; m.p. 144 °C. ¹³C-NMR (100 MHz, CDCl₃: *δ*: 166.6, 162.7, 153.1, 152.9, 152.7, 148.8, 140.1, 135.7, 130.7, 123.6, 118.7, 113.1, 83.9, 28.1.
*tert*-Butyl 2-(furan-2-carboxamido)nicotinate **(4m)** was obtained in 45% (0.720 g, 2.25 mmol) yield. White crystals; m.p. 111 °C. ¹³C-NMR (100 MHz, CDCl₃: *δ*: 166.9, 159.7, 153.3, 153.2, 146.1, 144.1, 140.2, 124.2, 118.3, 112.7, 109.0, 83.8, 28.3.
*tert*-Butyl 2-(thiophene-2-carboxamido)nicotinate **(4n)** was obtained in 66% (0.904 g, 2.97 mmol) yield. White crystals; m.p. 121 °C. ¹³C-NMR (100 MHz, CDCl₃: *δ*: 166.8, 159.1, 153.2, 153.1, 140.7, 140.2, 131.9, 129.0, 128.0, 118.3, 112.8, 83.8, 28.3.
*tert*-Butyl 2-acetamidonicotinate **(4o)** was obtained in 75% (0.80 g, 3.75 mmol) yield. White crystals; m.p 81 °C. ¹³C-NMR (100 MHz, CDCl₃: *δ*: 169.8, 165.9, 152.6, 152.4, 140.2, 117.9, 112.4, 83.4, 28.2, 26.0.
*tert*-Butyl 2-octanamidonicotinate **(4p)** was obtained in 100% (1.60 g, 4.99 mmol) yield. Yellow oil. ¹³C-NMR (100 MHz, CDCl₃: *δ*: 172.1, 166.1, 152.7, 152.6, 140.0, 117.8, 112.6, 83.3, 38.7, 31.7, 29.2, 29.1, 28.2, 25.3, 22.6, 14.1.
*tert*-Butyl 2-dodecanamidonicotinate **(4q)** was obtained in 75% (1.14 g, 3.75 mmol) yield. Yellow oil. ¹³C-NMR (100 MHz, CDCl₃: *δ*: 172.1, 166.1, 152.7, 152.6, 140.0, 117.8, 112.5, 83.3, 38.8, 31.9, 29.6, 29.5, 29.4, 29.3, 29.3, 28.2, 25.3, 22.7, 14.1.
*tert*-Butyl 2-isobutyramidonicotinate **(4r)** was obtained in 86% (1.13 g, 4.28 mmol) yield. White crystals; m.p. 62 °C. ¹³C-NMR (100 MHz, CDCl₃: *δ*: 175.2, 166.2, 152.9, 152.7, 139.9, 117.8, 112.8, 83.2, 37.4, 28.1, 19.4.
*tert*-Butyl 2-(cyclopropanecarboxamido)nicotinate **(4s)** was obtained in 61% (0.800 g, 3.05 mmol) yield. White crystals; m.p. 144 °C. ¹³C-NMR (100 MHz, CDCl₃: *δ*: 172.3, 166.1, 152.7, 140.0, 117.8, 112.3, 83.3, 77.2, 21.1, 16.4, 9.0.
*tert*-Butyl 2-(cyclohexanecarboxamido)nicotinate **(4t)** was obtained in 59% (0.890 g, 2.92 mmol) yield. White crystals; m.p. 92 °C. ¹³C-NMR (100 MHz, CDCl₃): *δ*: 174.2, 166.1, 152.8, 152.7, 140.0, 117.8, 112.8, 83.1, 47.1, 29.5, 28.0, 25.7, 27.6.
*tert*-Butyl 2-(adamantane-1-carboxamido)nicotinate **(4u)** was obtained in 95% (1.70 g, 4.76 mmol) yield. White crystals; m.p. 102 °C. ¹³C-NMR (100 MHz, DMSO-d6, 100 C): *δ*: 174.3, 167.8, 150.2, 149.8, 137.9, 118.8, 118.7, 81.2, 40.7, 38.0, 35.6, 27.3.
*tert*-Butyl 2-((8*E*,11*E*)-octadeca-8,11-dienamido)nicotinate (*tert*-butyl 2-(linoleylamido)nicotinate) **(4v)** was obtained in 83% (1.54 g, 3.37 mmol) yield. Colorless oil. ¹³C-NMR (100 MHz, CDCl₃): *δ*: 172.1, 166.1, 152.7, 152.6, 140.0, 130.2, 130.1, 128.0, 128.0, 117.8, 112.5, 83.3, 38.7, 31.5, 29.7, 29.4, 29.3, 29.3, 29.2, 28.2, 27.6, 27.2, 25.7, 25.3, 22.6, 14.1.
*tert*-Butyl 2-{[*N*-(*tert*-butoxycarbonyl)glycyl]amino}nicotinate **(4w)** (Boc-Gly-NH-*t*Bu *nic)* was obtained in 85% (1.49 g, 4.25 mmol) yield. White crystals; m.p. 129 °C. ¹³C-NMR (100 MHz, CDCl₃): *δ*: 169.7, 165.7, 155.9, 152.2, 152.1, 140.2, 118.1, 112.2, 83.4, 79.7, 46.7, 28.4, 28.1.
*tert*-Butyl 2-({*N*-[(benzyloxy)carbonyl]glycyl}amino)nicotinate **(4x)** (Cbz-Gly-NH-*t*Bu *nic)* was obtained in 56% (1.08 g, 2.81 mmol) yield. White crystals; m.p. 124 °C. ¹³C-NMR (100 MHz, CDCl₃): *δ*: 169.4, 165.7, 156.4, 152.2, 152.0, 140.3, 136.5, 128.5, 128.1, 118.2, 112.1, 83.6, 67.0, 47.1, 28.1.
*tert*-Butyl 2-[1-(*tert*-butoxycarbonyl)piperidine-2-carboxamido]nicotinate (*tert*-butyl 2-[1-(*tert-*butoxycarbonyl)pipecolinamido]nicotinate **(4y)** was obtained in 52% (1.06 g, 2.60 mmol) yield. White crystals ; m.p 118 °C. ¹³C-NMR (100 MHz, DMSO-d6, 100 C): *δ*: 169.7, 164.5, 154.3, 150.5, 149.3, 138.3, 118.8, 117.8, 81.4, 78.7, 54.7, 41.0, 27.5, 27.3, 25.6, 23.7, 19.2.
*tert*-Butyl 2-{[1-(*tert*-butoxycarbonyl)-L-prolyl]amino}nicotinate **(4z)** (Boc-L-Pro-NH-*t*Bu *nic)* was obtained in 80% (1.566 g, 4.00 mmol) yield. Brown crystals; m.p. 98 °C. ¹³C-NMR (100 MHz, DMSO-d6, 100 °C): *δ*: 170.4, 164.5, 153.1, 150.5, 149.4, 138.3, 118.7, 117.2, 81.5, 78.3, 60.2, 46.1, 29.3, 27.5, 27.3, 22.8.
(*R*)-*tert*-Butyl 2-[2-(2-oxopyrrolidin-1-yl)butanamido]nicotinate **(4aa)** (Keppra-NH-*t*Bu nic) was obtained in 71% (3.57 mmol, 1.24 g). Light-yellow oil. ¹³C-NMR (100 MHz, CDCl₃): *δ*: 176.3, 168.4, 166.2, 152.8, 152.4, 139.9, 118.4, 113.0, 83.5, 57.7, 43.6, 30.8, 28.2, 21.1, 18.0, 10.8.
*tert*-Butyl 2-{[*N*²,*N*⁶-bis(*tert*-butoxycarbonyl)-L-lysyl]amino}nicotinate **(4ab)** (Boc-L-Lys(Boc)-NH-*t*Bu *nic)* was obtained in 56% (2.0655 g, 3.95 mmol) yield. White crystals; m.p. 162 °C. ¹³C-NMR (100 MHz, CDCl₃): *δ*: 165.9, 156.1, 155.7, 152.6, 152.3, 140.1, 118.3, 112.8, 83.5, 79.8, 79.0, 55.5, 40.2, 32.8, 29.6, 28.5, 28.4, 28.1, 22.6.
- Using the following conditions: 5.0 mol% **10a,** 1.0 equiv **2a,** 1.1 equiv amide **3,** 2.0 equiv Cs₂CO₃, 2-MeTHF (anhydrous), 40 C, 24 h.
*tert*-Butyl 2-(4-chlorobenzamido)nicotinate **(4ac)** was obtained in 90% (1.36 g, 4.09 mmol) yield. White crystals; m.p. 132 °C. ¹³C-NMR (100 MHz, CDCl₃: *δ*: 166.6, 163.4, 153.0, 153.0, 140.0, 138.4, 133.4, 129.1, 129.0, 118.4, 113.1, 83.7, 28.1.
*tert*-Butyl 2-{[*N*-(*tert*-butoxycarbonyl)-L-valyl]amino}nicotinate **(4ad)** (Boc-L-Val-NH-*t*Bu *nic* was obtained in 98% (1.991 g, 4.89 mmol) yield. White crystals, m.p. 132 °C. ¹³C-NMR (100 MHz, CDCl₃): *δ*: 170.5, 166.0, 155.9, 152.7, 152.3, 140.0, 118.3, 112.8, 83.4, 79.7, 60.7, 31.1, 28.4, 28.1, 19.6, 17.2.
*tert*-Butyl 2-{3-[(*tert*-butoxycarbonyl)amino]propanamido}nicotinate **(4ae)** (Boc-*β*-Ala-NH-*t*Bu nic) was obtained in 100% (1.825 g, 5.0 mmol) yield. White crystals; m.p. 99 °C. ¹³C-NMR (100 MHz, CDCl₃): *δ*: 171.4, 165.9, 156.0, 152.4, 152.4, 140.2, 118.1, 112.5, 83.5, 79.0, 38.3, 36.1, 28.4, 28.2.
*tert*-Butyl 2-{[*N*-(*tert*-butoxycarbonyl)-L-isoleucyl]amino}nicotinate **(4af)** (Boc-L-Ile-NH-*t*Bu *nic)* was obtained in 99% (0.716 g, 1.76 mmol) yield. dr = (2S, 3S):(2R, 3S) = 87:12. Colorless oil. ¹³C-NMR (100 MHz, DMSO-d6, 100 C): characterisation of the major diastereomer (2S, 3S)-**4af:** *δ*: 169.7, 164.4, 154.7, 150.2, 148.8, 138.2, 119.0, 81.3, 77.9, 59.3, 36.3, 27.6, 27.3, 23.8, 14.9, 10.3. ¹³C-NMR (100 MHz, DMSO-d6, 100 C): characterisation of the minor diastereomer (2R, 3S)**-4af**: *δ*: 170.0, 164.4, 154.7, 150.3, 148.9, 138.3, 118.7, 81.3, 77.9, 58.2, 36.1, 27.6, 27.3, 23.8, 13.7, 10.7.
*tert*-Butyl 2-{[N-(*tert*-butoxycarbonyl)-*O*-*tert*-butyl-L-seryl]amino}nicotinate **(4ag)** (Boc-Ser(OtBu)-NH-*t*Bu *nic*) was obtained in 87% (1.893 g, 4.33 mmol) yield. Brown oil. ¹³C-NMR (100 MHz, CDCl₃): *δ*: 173.0, 168.9, 165.4, 155.2, 152.2, 151.7, 139.7, 118.1, 113.0, 82.7, 79.4, 73.3, 61.6, 28.0, 27.7, 27.0.
- Using the following conditions: 5.0 mol% **10a,** 1.0 equiv **2a,** 1.1 equiv amide **3,** 5.0 equiv Cs₂CO₃, 2-MeTHF (anhydrous), 40 C, 24 h.
*tert*-Butyl 2-(4-nitrobenzamido)nicotinate **(4ah)** was obtained in 32% (0.572 g, 1.593 mmol) yield. Brown crystals; m.p 136 °C. ¹³C-NMR (100 MHz, CDCl₃): *δ*: 166.7, 162.4, 153.1, 152.7, 150.0, 140.5, 140.1, 128.8, 124.0, 118.9, 113.2, 84.1, 28.2.
- Using the following conditions: 5.0 mol% **10a,** 1.0 equiv **2a,** 1.1 equiv amide **3,** 7.5 equiv Cs₂CO₃, 2-MeTHF (anhydrous), 40 C, 24 h.
*tert*-Butyl 2-pivalamidonicotinate **(4ai)** was obtained in 83% (1.16 g, 4.16 mmol) yield. White crystals; m.p 77 °C. ¹³C-NMR (100 MHz, CDCl₃): *δ*: 176.3, 166.4, 153.2, 152.8, 139.8, 117.9, 113.1, 83.2, 40.6, 28.1, 27.5.
1-(*tert*-Butoxycarbonyl)-L-prolyl-L-leucyl-*N*-[3-*tert*-butoxycarbonyl)pyridin-2-yl)]glycinamide **(4aj)** (Boc-L-Pro-Leu-Gly-NH-*t*Bu *nic)* was obtained in 100% (1.790 g, 3.20 mmol) yield. de > 99%. White crystals; m.p. 182 °C (decomposition). ¹³C-NMR (100 MHz, DMSO-d6, 100 C): *δ*: 171.8, 171.5, 167.7, 164.4, 153.3, 150.5, 149.0, 138.8, 118.9, 117.5, 81.6, 78.2, 59.1, 50.8, 46.0, 43.0, 40.5, 27.6, 27.3, 23.7, 22.8, 22.3, 21.4.
- Using the following conditions: 10.0 mol% **10a,** 1.0 equiv **2a,** 1.1 equiv amide **3,** 7.5 equiv Cs₂CO₃, 2-MeTHF (anhydrous), 40 C, 24 h.
*tert*-Butyl 2-{[*N*-*tert*-butoxycarbonyl)-L-methionyl]amino}nicotinate **(4ak)** (Boc-L-Met-NH-*t*Bu *nic)* was obtained in 65% (1.377 g, 3.24 mmol) yield. Yellow crystals; m.p. 117 °C. ¹³C-NMR (100 MHz, DMSO-d6): *δ*: 170.7, 165.0, 155.4, 150.8, 148.9, 138.8, 119.7, 119.4, 81.5, 78.2, 54.2, 31.2, 29.7, 28.1, 27.6, 14.5.
- Using the following conditions: 5.0 mol% **10a,** 1.0 equiv **2a,** 1.1 equiv amide **3,** 7.5 equiv Cs₂CO₃, 2-MeTHF:DMF 1:1 (anhydrous), 40 C, 24 h.
*tert*-Butyl 2-(3-nitrobenzamido)nicotinate **(4al)** was obtained in 100% (1.715 g, 5.0 mmol) yield. Brown crystals; m.p 132 °C. ¹³C-NMR (100 MHz, CDCl₃): *δ*: 166.9, 162.3, 153.3, 152.9, 148.8, 140.3, 137.0, 133.6, 130.2, 126.7, 123.0, 119.0, 113.4, 84.3, 28.3.
*tert*-Butyl 2-(2,2,2-triphenylacetamido)nicotinate **(4am)** was obtained in 60% (1.05 g, 2.260 mmol) yield. Brown oil. ¹³C-NMR (100 MHz, CDCl₃): δ: 171.3, 163.4, 152.3, 152.2, 140.0, 139.3, 130.8, 127.9, 126.8, 118.3, 114.4, 82.7, 69.2, 28.0.
*tert*-Butyl 2-{[*N*-(*tert*-butoxycarbonyl)-D-phenylalanyl]amino}nicotinate **(4an)** (Boc-D-Phe-NH-*t*Bu *nic*) was obtained in 67% (1.479 g, 3.35 mmol) yield. White crystals ; m.p. 159 °C. ¹³C-NMR (100 MHz, DMSO-d6): *δ*: 170.9, 165.0, 152.2, 150.8, 148.9, 139.9, 138.1, 129.2, 127.9, 126.1, 119.8, 119.6, 81.5, 78.0, 56.3, 36.8, 28.1, 27.7.

### Example 3. Catalyst screening for catalytic py-directed amide alcoholysis

Benzamide **(3a)** equipped with a *py*-DG **(1a)** can be cleaved with *i*PrOH **(11a)** in the presence of a metal catalyst (e.g. Fe, Cu, Zn, Co, Mn, Ni, Al; 10.0 mol%) towards isopropyl benzoate **(12a)** and 2-aminopyridine **(6a)** at a maximum temperature of 140 °C (Example table E1).

**Example Table E1: Transition metal-catalysed directed cleavage of N-(pyridin-2-yl)benzamide (1a): Catalyst evaluation at 140 °C.**

| | | | | |
|---|---|---|---|---|
| 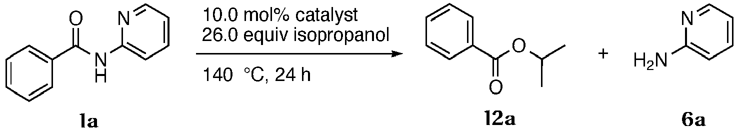 | | | | |

| Entry | Catalyst (10.0 mol%) | **1a¹** | **12a¹** | **6a¹** |
|---|---|---|---|---|
| 1 | Fe(II)(ClO₄)₂ | 69% | 26% | 30% |
| 2 | Cu(OAc) (anhydrous) | trace | 93% | quant. |
| 3 | Zn(OAc)₂.17H₂O | 0% | 91% | 82% |
| 4 | Co(OAc)₂.4H₂O | 1% | 96% | 93% |
| 5 | MnBr₂.2H₂O | 0% | 97% | 99% |
| 6 | Ni(acac)₂ (4% H₂O) | 0% | quant. | quant. |
| 7 | AlCl₃ | 47% | 50% | 55% |

| | | | | |
|---|---|---|---|---|
| ¹GC-yield determined with 1,3,5-trimethoxybenzene as an internal standard. | | | | |

**Example Table E2: Directing group evaluation in transition metal-catalyzed directed cleavage of C3- and C5-substituted N-(pyridin-2-yl)benzamides 1 using Zn(OAc)₂.17H₂O as a catalyst.**

| | | | | | | |
|---|---|---|---|---|---|---|
| 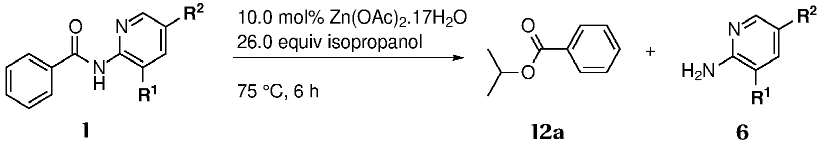 | | | | | | |

| Entry | R¹ | R² | **1¹** | **12a¹** | **6¹** | Mass balance |
|---|---|---|---|---|---|---|
| 1 | H | H | **1a** (96%) | **12a** (trace) | **6a** (trace) | quant. |
| 2² | F | H | **1b** (33%) | **12a** (34%) | **6c** (n.m.) | 67% |
| 3 | H | F | **1c** (65%) | **12a** (15%) | **6d** (n.m.) | 80% |
| 4 | Cl | H | **1d** (3%) | **12a** (94%) | **6e** (84%) | 97% |
| 5 | H | Cl | le (70%) | **12a** (24%) | **6f** (n.m.) | 94% |
| 6 | COOMe | H | **1f** (0%) | **12a** (96%) | **6g** (92%) | 96% |
| 7 | H | COOMe | **1g** (49%) | **12a** (15%) | **6h** (n.m.) | 64% |
| 8 | COOEt | H | **1h** (4%) | **12a** (95%) | **6i** (quant.) | 99% |
| 9 | COO*i*Pr | H | **1i** (0%) | **12a** (91%) | **6j** (n.m.) | 91% |
| 10 | COO*t*Bu | H | **4a** (0%) | **12a** (96%) | **6b** (n.m.) | 96% |
| 11 | H | COOtBu | **1j** (35%)³ | **12a** (32%) | **6k** (n.m.) | 81% |
| 12 | NO₂ | H | **1k** (0%) | **12a** (94%) | **6l** (95%) | 94% |
| 13 | H | NO₂ | **1l** (85%) | **12a** (6%) | **6m** (trace) | 91% |
| 14 | CN | H | **1m** (41%) | **12a** (17%) | **6n** (n.m.) | 58% |
| 15 | H | CN | **In** (63%) | **12a** (9%) | **6o** (trace) | 72% |
| 16 | CF₃ | H | **1o** (93%) | **12a** (6%) | **6p** (n.m.) | 99% |
| 17 | H | CF₃ | **1p** (95%) | **12a** (3%) | **6q** (n.m.) | 98% |
| 18 | Me | H | **1r** (12%) | **12a** (18%) | **6r** (n.m.) | 30% |
| 19 | H | Me | **1s** (74%) | **12a** (6%) | **6s** (trace) | 80% |
| 20 | Et | H | **1t** (68%) | **12a** (19%) | **6t** (n.m.) | 87% |
| 21 | H | Et | **1u** (80%) | **12a** (15%) | **6u** (n.m.) | 95% |
| 22 | H | Ph | **1q** (95%) | **12a** (3%) | **6v** (trace) | 98% |
| 23 | OMe | H | **1v** (0%) | **12a** (92%) | **6w** (91%) | 92% |
| 24 | H | OMe | **1w** (quant.) | **12a** (4%) | **6x** (trace) | quant. |
| 25 | O*i*Pr | H | **1x** (trace) | **12a** (85%) | **6y** (n.m.) | 85% |

| | | | | | | |
|---|---|---|---|---|---|---|
| ¹GC-yield determined with 1,3,5-trimethoxybenzene as an internal standard, n.m. = not measured. ²Observation of by-products in GC-chromatogram. ³Also 14% Boc-deprotected starting material **1a** was observed. | | | | | | |

### Example 4. Directing group evaluation for catalytic py-directed amide alcoholysis

Different C3- and C5-substituted directing group were found to be suitable for the Zn(OAc)₂.17H₂O-catalysed directed cleavage of **1** with *i*PrOH **(10a).** Both electron-withdrawing (F, Cl, COOR, NO₂, CN, CF₃) and electron-donating (Alkyl, Ph, OR) groups in C3- and C5-position of the *py*-DG were evaluated (Example Table E2).

### Example 5. Scope of tBu nic-directed catalytic cleavage of amide-containing compounds with alcohols 11

### Example 5.1. Scope of tBu nic-directed catalytic amide cleavage with alcohols 11

*N*-(Pyridin-2-yl)amides **1,** such as *tert*-butyl 2-amidonicotinates **4,** can undergo catalytic directed cleavage at room temperature to 140 °C with a primary, secondary or *tert*iary aliphatic or aromatic alcohol nucleophile **11** (1.0 equiv to suprastoichiometric amounts) in the presence of a metal catalyst and in the presence or absence of a reaction solvent towards the corresponding esters **13.** Based on the general scheme depicted Example Scheme E3, the following examples can be listed (with R" = R'): 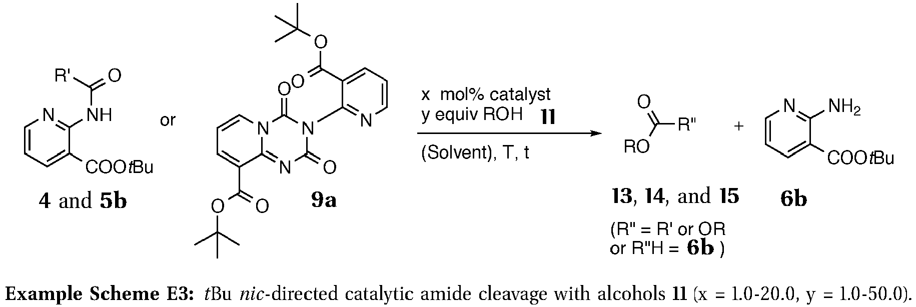
- Using 4.0 mol% Zn(OAc)₂.17H₂O, 1.0 equiv **4**, 3.0 equiv **11,** *t*BuOAc, 40 °C
   2-Benzyl 1-(*tert*-butyl) (S)-pyrrolidine-1,2-dicarboxylate (Boc-L-Pro-OBn) **(13a)** was obtained in 85% (0.258 g, 0.846 mmol) yield. Reaction time: 72 h. Colorless oil. ¹³C-NMR (100 MHz, DMSO-d6, 100 C): *δ*: 171.1, 152.6, 135.5, 127.7, 127.4, 127.1, 78.4, 65.3, 58.3, 45.7, 29.3, 27.5, 22.8.
   Benzhydryl benzoate **(13b)** was obtained in 56% (0.162 g, 0.562 mmol) yield. Reaction time: 48 h. White crystals; m.p. 88 °C. ¹³C-NMR (100 MHz, CDCl₃): *δ*: 165.6, 140.3, 133.1, 130.3, 129.8, 128.6, 128.4, 128.0, 127.2, 77.5.
   3-Phenylpropyl benzoate **(13c)** was obtained in 70% (0.169 g, 0.703 mmol) yield. Reaction time: 24 h. Colorless oil. ¹³C-NMR (100 MHz, CDCl₃): *δ*: 166.5, 141.2, 132.8, 130.4, 129.6, 128.5, 128.4, 128.3, 126.0, 64.3, 32.3, 30.3.
   *tert*-Butyl 3-(benzoyloxy)piperidine-1-carboxylate **(13d)** was obtained in 65% (0.198 g, 0.650 mmol) yield. Reaction time: 48 h. Brown crystals; m.p. 73 °C. ¹³C-NMR (100 MHz, DMSO-d6, 100 C): *δ*: 164.5, 153.6, 132.4, 129.8, 128.5, 127.9, 78.1, 68.1, 46.3, 43.0, 27.9, 27.4, 20.6.
- Using 10.0 mol% Zn(OAc)₂.17H₂O, 1.0 equiv **4,** 3.0 equiv **11,** 2-MeTHF, 50 °C, 72 h Cholesteryl pivalate **(13e)** was obtained in 60% (0.280 g, 0.595 mmol) yield. White crystals; m.p. 160 °C. ¹³C-NMR (100 MHz, CDCl₃): *δ*: 178.0, 139.8, 122.5, 73.5, 56.7, 56.1, 50,0, 42.3, 39.7, 39.5, 38.6, 38.0, 37.0, 36.6, 36.2, 35.8, 31.9, 31.9, 28.2, 28.0, 27.7, 27.2, 24.3, 23.8, 22.8, 22.6, 21.0, 19.4, 18.7, 11.9.
- Using 8.0 mol% Zn(OAc)₂.17H₂O, 1.0 equiv **4,** 3.0 equiv **11,** *t*BuOAc, 50 °C, 48 h (1*R*,2*S*,5*R*)-2-Isopropyl-5-methylcyclohexyl 3-nitrobenzoate (L-Menthyl 3-nitrobenzoate) **(13f)** was obtained in 76% (0.233 g, 0.763 mmol) yield. Reaction time: 48 h. Colorless oil. ¹³C-NMR (100 MHz, CDCl₃): *δ*: 164.0, 148.3, 135.3, 132.6, 129.6, 127.2, 124.5, 76.1, 47.1, 40.9, 34.2, 31.5, 26.5, 23.5, 22.0, 20.8, 16.5.

The side-product of the exemplary catalytic directed amide cleavage reactions, *tert*-butyl 2-aminonicotinate **(6b)** can be isolated:
*tert*-Butyl-2-aminonicotinate **(6b).** White crystals; m.p. 119 °C. ¹³C-NMR (100 MHz, CDCl₃): *δ*: 166.4, 159.5, 153.0, 140.3, 112.6, 108.1, 81.5, 28.3.

This product **6b** can be easily transformed in one step into *tert*-butyl 2-chloronicotinate **(2a)** in a yield of 44%. The process involves diazotisation using aq. HCI with addition of sodium chloride (1.5 equiv NaNO₂, 2.0 equiv NaCl, HCl(*aq*), 0 °C to room temperature, 12 h). In this way the waste product **6b** can be transformed into the reagent required for DG-introduction (*tert*-butyl 2-chloronicotinate **(2a)**).

### Example 5.2. Zn-catalysed cleavage of 5b and 9a with alcohols 11

For R' = NH-tBu *nic* in Example Scheme E3 and R" = OR, the following examples can be listed:
- Using 10.0 mol% anhydrous Zn(OAc)₂, 1.0 equiv **5b,** 5.0 equiv anhydrous benzyl alcohol **(11b),** anhydrous *t*BuOAc, 100 °C, 48 h under argon atmosphere.

Dibenzyl carbonate **(14a)** was obtained in 92% (0.111 g, 0.46 mmol) yield. Colorless oil. ¹³C-NMR (100 MHz, CDCl₃): *δ*: 155.1, 135.2, 128.6, 128.5, 128.3, 69.7. Also *tert*-butyl 2-aminonicotinate **(6b)** was obtained in 97% (0.189 g, 0.97 mmol) yield. ¹³C-NMR (100 MHz, CDCl₃): *δ*: 166.4, 159.5, 153.0, 140.3, 112.6, 108.1, 81.5, 28.3.
- Using 10.0 mol% anhydrous Zn(OAc)₂, 1.0 equiv **5b,** 5.0 equiv anhydrous benzyl alcohol **(11b),** anhydrous 2-MeTHF, 100 °C, 24 h under argon atmosphere, the following NMR-yields with TMB as internal standard can be obtained: 88% of **6b** (formation of 2.0 equiv **6b** = 97% NMR-yield) and 88% of **14a.**

Following Example Scheme E3, the following examples can be listed:
- Using 9.50 mol% anhydrous Zn(OAc)₂, 1.0 equiv **9a,** 4.70 equiv anhydrous benzyl alcohol **(11b),** anhydrous 2-MeTHF, 100 °C, 24 h under argon atmosphere, the following NMR-yields with TMB as internal standard can be obtained: 88% of **6b** (formation of 2.0 equiv **6b** = 100% NMR-yield) and 82% of **14a.**
- Using 9.50 mol% Zn(OAc)₂.17H₂O, 1.0 equiv **9a,** 28.0 equiv MeOH **(11c),** 100 °C, 24 h, the following NMR-yield with TMB as internal standard can be obtained: 78% of **6b** (formation of 2.0 equiv **6b** = 100% NMR-yield).
- Using 9.50 mol% Zn(OAc)₂.17H₂O, 1.0 equiv **9a,** 24.5 equiv alcohol **11,** 40°C, 24 h under argon atmosphere, the following NMR-yields with TMB as internal standard can be obtained:
   ∘ 56% of **5b,** 35% of *tert*-butyl 2-[(isopropoxycarbonyl)amino]nicotinate **(15a),** 34% of **6b** if alcohol = isopropanol (**11a**).
   ∘ 97% of *tert*-butyl 2-[(propoxycarbonyl)amino]nicotinate **(15b)** and 10% of dipropyl carbonate **(14b)** if alcohol = PrOH **(11d).**
- Using 9.50 mol% Zn(OAc)₂.17H₂O, 1.0 equiv **9a,** 13.0 equiv propanol **(11d),** *t*BuOAc, 40 °C, 24 h, the following NMR-yield with TMB as internal standard can be obtained: quantitative yield of *tert*-butyl 2-[(propoxycarbonyl)amino]nicotinate **(15b).**

### Example 6. Scope of tBu nic-directed catalytic cleavage of amide-containing compounds with amines 16 (transamidation, transureation)

### Example 6.1. Zn-catalysed cleavage of tert-butyl 2-amidonicotinates 4 with amines 16

*N*-(Pyridin-2-yl)amides **1,** such as *tert*-butyl 2-amidonicotinates **4,** can undergo catalytic directed cleavage at room temperature to 140 °C with a primary or secondary aromatic or aliphatic amine nucleophile **16** (1.0 equiv to suprastoichiometric amounts) in the presence of a metal catalyst and in the presence or absence of a reaction solvent towards the corresponding transamidation products **17.** Based on the general scheme depicted in Example Scheme E4, the following examples can be listed for R'''' = R': 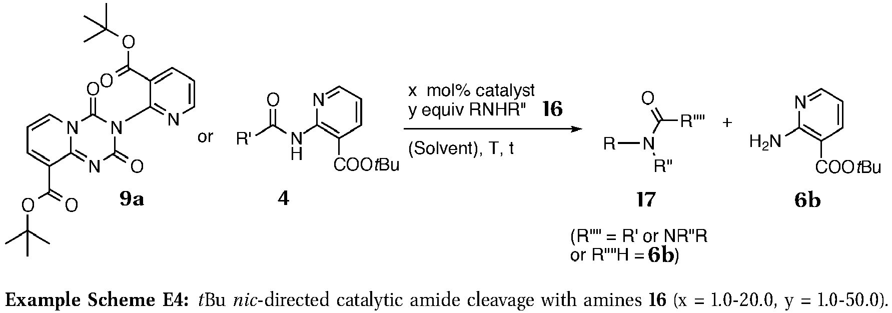
- Using 4.0 mol% Zn(OAc)₂.17H₂O, 1.0 equiv **4,** 3.0 equiv **16,** *t*BuOAc, 40 °C, 48 h.
   *N*-Hexylbenzamide **(17a)** was obtained in 68% (0.139 g, 0.675 mmol) yield. Colorless oil. ¹³C-NMR (100 MHz, CDCl₃): *δ*: 167.6, 134.9, 131.2, 128.4, 127.0, 40.2, 31.5, 29.7, 26.7, 22.6, 14.0.
   *N*-Allylbenzamide **(17b)** was obtained in 84% (0.135 g, 0.837 mmol) yield. Colorless oil. ¹³C-NMR (100 MHz, CDCl₃): *δ*: 167.5, 134.5, 134.3, 131.4, 128.5, 127.0, 116.5, 42.4.
   *N*-Isobutylbenzamide **(17c)** was obtained in 87% (0.153 g, 0.866 mmol) yield. Colorless oil. ¹³C-NMR (100 MHz, CDCl₃): *δ*: 167.7, 135.0, 131.3, 128.5, 126.9, 47.4, 28.7, 20.2.
   4-Fluoro-*N*-propylbenzamide **(17d)** was obtained in 98% (0.178 g, 0.983 mmol) yield. White crystals; m.p. 67 °C. ¹³C-NMR (100 MHz, CDCl₃): *δ*: 166.8, 164.6 (d, *J* = 250.95 Hz), 131.1 (d, *J* = 3.27 Hz), 129.3 (d, *J* = 8.74 Hz), 115.4 (d, *J* = 21.11 Hz), 41.9, 22.9, 11.4.
   *N*-(4-Fluorobenzyl)-3-nitrobenzamide **(17e)** was obtained in 100% (0.274 g, 1.0 mmol) yield. White crystals; m.p 129 °C. ¹³C-NMR (100 MHz, DMSO-d6): *δ*: 164.0, 147.8, 135.6, 135.3 (d, *J* = 3.23 Hz), 133.7, 130.1, 129.4, (d, *J* = 8.09 Hz), 125.9, 121.9, 115.0 (d, *J* = 21.36 Hz), 42.2.
   *N*-Benzylcyclohexanecarboxamide **(17f)** was obtained in 89% (0.193 g, 0.889 mmol) yield. Colorless oil. ¹³C-NMR (100 MHz, CDCl₃): *δ*: 176.9, 138.4, 128.6, 127.7, 127.3, 45.2, 43.5, 29.6, 28.0, 25.7.
- Using 8.0 mol% Zn(OAc)₂.17H₂O, 1.0 equiv **4,** 3.0 equiv **16,** *t*BuOAc, 60 °C, 48 h. (4-Methoxypiperidin-1-yl)(p-tolyl)methanone **(17g)** was obtained in 77% (0.178 g, 0.765 mmol) yield. Colorless oil. ¹³C-NMR (100 MHz, CDCl₃): *δ*: 175.0, 170.8, 139.8, 132.8, 129.1, 127.0, 75.4, 55.7, 31.4, 21.0.

The side-product of the exemplary catalytic directed amide cleavage reactions, *tert*-butyl 2-aminonicotinate **(6b)** can be isolated:
*tert*-Butyl-2-aminonicotinate **(6b).** White crystals ; m.p. 119 °C. ¹³C-NMR (100 MHz, CDCl₃): *δ*: 166.4, 159.5, 153.0, 140.3, 112.6, 108.1, 81.5, 28.3.

This product **6b** can be easily transformed in one step into *tert*-butyl 2-chloronicotinate **(2a)** in a yield of 44%. The process involves diazotisation using aq. HCI with addition of sodium chloride (1.5 equiv NaNO₂, 2.0 equiv NaCl, HCI(aq), 0 °C to room temperature, 12 h). In this way the waste product **6b** can be transformed into the reagent required for DG-introduction (*tert*-butyl 2-chloronicotinate (**2a**)).

### Example 6.2. Zn-catalysed cleavage of 9a with amines 16

Following Example Scheme E4, the following example can be listed:
- Using 15.0 mol% Zn(OAc)₂.17H₂O, 1.0 equiv **9a,** 6.0 equiv **16,** *t*BuOAc, 100 °C, 48 h.
1,3-bis(Benzo[d]oxazol-2-yl)urea **(18a)** was obtained in 59% (0.087 g, 0.296 mmol) yield. White crystals ; m.p. 130 °C. ¹³C-NMR (100 MHz, DMSO-d6): *δ*: 162.7 (C*_{quat}*), 147.8 (C*_{quat}*), 143.4 (C*_{quat}*), 123.4 (CH), 119.9 (CH), 115.3 (CH), 108.3 (CH).

### Example 7. Synthesis of amide-containing compounds bearing N-benzimidazol-2-yl directing groups

1,3-bis(1-Propyl-1*H*-benzo[*d*]imidazol-2-yl)urea **(19a)** and 10-propyl-3-(1-propyl-1*H*-benzo[*d*]-imidazol-2-yl)benzo[4,5]imidazo[1,2-*a*][1,3,5]triazine-2,4(3*H*,10*H*)-dione **(9b)** were obtained in 8% (16.0 mg, 0.045 mmol) and 12% (0.027 g, 0.067 mmol) yield respectively via a reaction of 1-propyl-1*H-*benzo[*d*]imidazol-2-amine **(20a)** (0.200 g, 1.141 mmol), 4-nitrophenylchloroformate (0.127 g, 0.628 mmol) and DIPEA (0.258 mL, 1.484 mmol) in CH₂Cl₂ (4.0 mL) at room temperature. Reaction time 16 h. 1,3-bis(1-Propyl-1*H*-benzo[*d*]imidazol-2-yl)urea **(19a)** White solid ; m.p. 178 °C. ¹H-NMR (400 MHz, CDCl₃): *δ*: 7.30-7.29 (m, 2H), 7.13-7.09 (m, 6H), 4.02 (t, 4H, *J* = 7.20 Hz), 1.78 (d, 4H, *J* = 7.60 Hz), 0.88 (t, 6H, *J* = 7.60 Hz). 10-propyl-3-(1-propyl-1*H*-benzo[*d*]imidazol-2-yl)benzo[4,5]imidazo[1,2-*a*][1,3,5]triazine-2,4(3H,10H)-dione **(9b).** Colorless solid ; m.p. 222 °C. ¹H-NMR (400 MHz, DMSO-d6): *δ*: 8.01 (d, 1H, *J* = 7.60 Hz), 7.75 (d, 1H, *J* = 8.00 Hz), 7.71-7.67 (m, 2H), 7.54-7.49 (t, 2H, *J* = 8.00 Hz), 7.41-7.28 (m, 3H), 4.21-4.04 (m, 4H), 1.91-1.81 (m, 2H), 1.79-1.69 (m, 2H), 1.00 (t, 3H, *J* = 7.20 Hz), 0.87 (t, 3H, *J* = 7.20 Hz).

*tert*-Butyl [1-(methoxymethyl)-1*H*-benzo[*d*]imidazol-2-yl]carbamate **(21a)** was obtained in 64% (0.500 g, 1.80 mmol) yield via reaction of 1-(methoxymethyl)-1*H*-benzo[*d*]imidazol-2-amine **(20b)** (0.500 g, 2.82 mmol), Boc₂O (739 mg, 3.39 mmol) and Et₃N (0.590 mL, 4.23 mmol) in CH₂Cl₂ (10.0 mL) at room temperature. Reaction time 16 h. White solid ; m.p. 98 °C. ¹H-NMR (400 MHz, DMSO-d6): *δ*: 7.55 (d, 1H, *J* = 8.00 Hz), 7.41 (s, 1H), 7.15-7.09 (m, 2H), 7.02 (t, 1H, *J* = 7.60 Hz), 5.24 (s, 2H), 3.28 (s, 3H), 1.64 (s, 9H).

*tert*-Butyl 2-[(*tert*-butoxycarbonyl)amino]-1*H*-benzo[*d*]imidazole-1-carboxylate (**21b**) was synthesised via a literature protocol²⁷ and obtained in 81% (5.040 g, 15.13 mmol) yield (92% purity). White crystals; m.p. 122 °C. ¹H-NMR (400 MHz, CDCl₃): *δ*: 9.81 (br s, 1H), 7.68 (ddd, 1H, *J* = 2.11 Hz, 0.49 Hz, 0.65 Hz), 7.66 (ddd, 1H, *J* = 2.25 Hz, 1.14 Hz, 0.68 Hz), 7.27 (td, 1H, *J* = 7.61 Hz, 1.20 Hz), 7.19 (td, 1H, *J* = 7.83 Hz, 1.12 Hz), 1.74 (s, 9H), 1.57 (s, 9H).

1,3-bis(1*H*-Benzo[*d*]imidazol-2-yl)urea **(19b)** was synthesised based on a literature protocol²⁸ and 380 was obtained in 30% (0.306 g, 0.48 mmol) yield. White crystals ; m.p. 357 °C. ¹³C-NMR (100 MHz, TFA-d): δ: 152.6, 144.5, 129.8, 129.4, 115.4.

*tert*-Butyl [1-(methoxymethyl)-1*H*-benzo[*d*]imidazol-2-yl]carbamate **(21c)** was obtained in 64% (0.177 g) via a reaction of 1-(methoxymethyl)-1*H*-benzo[*d*]imidazol-2-amine **(20a)** (0.500 g, 2.82 mmol), Boc₂O (739.0 mg, 3.39 mmol) and Et₃N (0.590 mL, 4.23 mmol) in CH₂Cl₂ (10.0 mL) at ambient temperature. Reaction time 16 h. White solid ; m.p. 98 °C. ¹H-NMR (400 MHz, DMSO- d6): *δ*: 7.55 (d, 1H, *J* = 8.00 Hz), 7.41 (s, 1H), 7.15-7.09 (m, 2H), 7.02 (t, 1H, *J* = 7.6 Hz), 5.24 (s, 2H), 3.28 (s, 3H), 1.64 (s, 9H).

1,3-bis[1-(Methoxymethyl)-1*H*-benzo[*d*]imidazol-2-yl]urea **(19c)** was obtained in 30% (65.0 mg, 0.17 mmol) yield via reaction of 1-(methoxymethyl)-1*H*-benzo[*d*]imidazol-2-amine **(20a)** (200 mg, 1.13 mmol) with CO₂ at 50 bar pressure in a stainless steel autoclave, in the presence of phenylsilane (244.0 mg, 2.26 mmol), Ni(cod)₂ (16.0 mg, 0.056 mmol) and 1,2-bis(dicyclohexylphosphino)ethane (26.0 mg, 0.062 mmol) in THF (2.0 mL) at 100 °C. Reaction time 16 h. Colorless solid ; m.p. 130 °C. ¹H-NMR (400 MHz, CDCl₃): *δ*: 8.82 (s, 1H), 7.41 (s, 2H), 7.29 (d, 2H, *J* = 2.10 Hz), 5.57 (s, 2H), 3.39 (s, 3H), 1.27 (s, 2H).

10-(Methoxymethyl)-3-(1-(methoxymethyl)-1*H*-benzo[*d*]imidazol-2-yl)benzo[4,5]imidazo[1,2-*a*]-[1,3,5]triazine-2,4(3*H*,10*H*)-dione **(9c)** was obtained in 26% (1.52 g, 3.70 mmol) yield via reaction of 1-(methoxymethyl)-1*H*-benzo[*d*]imidazol-2-amine **(20b)** (5.05 g, 28.5 mmol), 4- nitrophenylchloroformate (3.16 g, 15.67 mmol) and DIPEA (6.45 ml, 37.0 mmol) in CH₂Cl₂ (30.0 mL) at room temperature for 16 h. Colorless solid; m.p. 236 °C. ¹H-NMR (400 MHz, CDCl₃): *δ*: 8.17 (d, 1H, *J* = 8.00 Hz), 7.85 (d, 1H, *J* = 7.60 Hz), 7.57-7.48 (m, 3H), 7.42-7.25 (m, 3H), 5.66 (d, 1H, *J* = 10.80 Hz), 5.62 (d, 1H, *J* = 10.80 Hz), 5.49 (d, 1H, *J* = 11.60 Hz), 5.45 (d, 1H, *J* = 11.20 Hz), 3.49 (s, 3H), 3.31 (s, 3H).

### Example 8. Synthesis of amide-containing compounds bearing N-imidazol-2-yl directing groups

1,3-Bis(1-propyl-1*H*-imidazol-2-yl)urea **(19d)** was obtained in 10% (0.410 g, 1.60 mmol) yield via a reaction of 1-propyl-1*H*-imidazol-2-amine **(20a)** (4.00 g, 32.0 mmol), 4-nitrophenylchloroformate (3.54 g, 17.58 mmol) and DIPEA (7.24 mL, 41.5 mmol) in anhydrous CH₂Cl₂ (80.0 mL) at 0 °C to room temperature under argon atmosphere. Reaction time 16 h. Yellow crystals ; m.p. 92 °C. ¹H-NMR (400 MHz, CDCl₃): *δ*: 8.00-10.50 (br, 2H), 6.74 (s, 2H), 6.64 (s, 2H), 3.79 (s, 4H), 1.74-1.72 (m, 4H), 0.88 (s, 6H).

1,3-di(1*H*-imidazol-2-yl)urea **(19e)** was obtained in 6% (0.037 g, 0.448 mmol) yield via the reaction of 2-aminoimidazole hemisulfate **(20c)** (1.806 g, 6.83 mmol) with CDI (0.554 g, 3.42 mmol) in the presence of Et₃N (2.127 mL, 15.18 mmol) in THF (200.0 mL) under reflux conditions for 48 h. White crystals ; m.p. 151 °C. ¹H-NMR (400 MHz, DMSO-d6): *δ*: 7.70 (s, 2H), 7.03 (s, 4H).

### Example 9. Scope of (benz)imidazolyl-directed catalytic cleavage of amide-containing compounds with alcohols 11

Amide-containing compounds with (benz)imidazol-2-yl groups can undergo catalytic directed cleavage with an alcohol nucleophile **11** in the presence of a metal catalyst. The following examples as depicted in Example Scheme E5 can be listed. 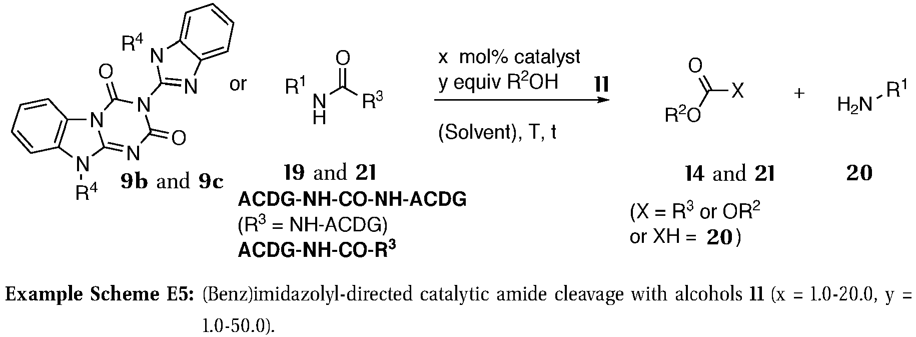
- Using 10.0 mol% Zn(OAc)₂.17H₂O, 1.0 equiv of ACDG-NH-CO-R³ **21d,** 30.0 equiv propanol **(11d),** 100 °C, 24 h; R¹ = 1*H*-benzo[*d*]imidazol-2-yl, R³ = OMe, results in the following NMR-yields (determined with TMB as an internal standard): 0% **21d,** 61% 2-aminobenzimidazole **(20d),** 40% methyl propyl carbonate **(14c),** 22% dipropyl carbonate **(14d).**
- Using 10.0 mol% Zn(OAc)₂.17H₂O, 1.0 equiv of ACDG-NH-CO-R³ **21c,** 5.0 equiv **11b,** *t*BuOAc, 40 °C, 24 h; R¹ = 1-(methoxymethyl)-1*H*-benzo[*d*]imidazol-2-yl, R³ = O*t*Bu

Benzyl *tert*-butyl carbonate **(14e)** was obtained in 60% (63.0 mg, 0.30 mmol) yield. Colorless oil. ¹³C-NMR (100 MHz, CDCl₃): *δ*: 153.6, 128.7, 128.4, 128.4, 82.4, 68.6, 27.9.
- Using 9.33 mol% Zn(OAc)₂.17H₂O, 1.0 equiv of **9c,** 4.67 equiv **11b,** *t*BuOAc, 100 °C, 24 h

Benzyl [1-(methoxymethyl)-1*H*-benzo[*d*]imidazol-2-yl]carbamate **(21e)** was obtained in 99% (0.145 g, 0.47 mmol) yield. Colorless oil. ¹³C-NMR (100 MHz, CDCl₃): *δ*: 164.0, 155.4, 137.2, 455 129.3, 128.4, 128.3, 128.1, 123.4, 123.2, 110.6, 110.2, 72.9, 67.3, 56.6. Also 1-(methoxymethyl)-1*H-*benzo[*d*]imidazol-2-amine **(20b)** was obtained in 54% (0.044 g, 0.25 mmol) yield. White crystals ; m.p. 180 °C. ¹³C-NMR (100 MHz, DMSO-d6): *δ*: 155.5, 143.4, 134.7, 121.5, 118.8, 115.3, 108.5, 72.8, 55.7.
- Using 9.33 mol% Zn(OAc)₂.17H₂O, 1.0 equiv **9c,** 4.67 equiv **11b,** *t*BuOAc, 100 °C, 24 h

Benzyl [1-(methoxymethyl)-1*H*-benzo[*d*]imidazol-2-yl]carbamate **(21e)** was obtained in 99% (0.145 g, 0.47 mmol) yield. Colorless oil. ¹³C-NMR (100 MHz, CDCl₃): *δ*: 164.0, 155.4, 137.2, 129.3, 128.4, 128.3, 128.1, 123.4, 123.2, 110.6, 110.2, 72.9, 67.3, 56.6. Also 1-(methoxymethyl)-1*H-*benzo[*d*]imidazol-2-amine **(20a)** was obtained in 50% (0.044 g, 0.25 mmol) yield. White crystals ; m.p. 180 °C. ¹³C-NMR (100 MHz, DMSO-d6): *δ*: 155.5, 143.4, 134.7, 121.5, 118.8, 115.3, 108.5, 72.8, 55.7.
- Using 9.30 mol% Zn(OAc)₂.17H₂O, 1.0 equiv **9b,** 4.67 equiv **11b,** *t*BuOAc, 100 °C, 24 h

Benzyl (1-propyl-1*H*-benzo[*d*]imidazol-2-yl)carbamate **(21f)** was obtained in 98% (0.140 g, 0.46 mmol) yield. Colorless oil. ¹³C-NMR (100 MHz, CDCl₃): *δ*: 163.8, 154.3, 137.3, 129.8, 128.2, 128.1, 127.9, 127.5, 122.5, 122.4, 110.5, 108.9, 66.9, 43.2, 21.4, 11.0. Also 1-propyl-1*H-*benzo[*d*]imidazol-2-amine **(20e)** was obtained in 69% (60.0 mg, 0.34 mmol) yield. White crystals ; m.p. 133 °C. ¹³C-NMR (100 MHz, DMSO-d6): *δ*: 155.4, 143.3, 134.8, 120.6, 118.3, 115.1, 107.9, 43.3, 22.2, 11.4.
- Using 10.0 mol% anhydrous Zn(OAc)₂, 1.0 equiv of ACDG-NH-CO-NH-ACDG **19a,** 5.0 equiv anhydrous **11b,** anhydrous *t*BuOAc, 100 °C, 24 h; R¹ = 1-propyl-1*H*-benzo[*d*]imidazol-2-yl, R³ = NHR¹

**Benzyl (1-propyl-1*H*-benzo[*d*]imidazol-2-yl)carbamate (21f)** was obtained in 60% (0.093 g, 0.30 mmol) yield. Dibenzyl carbonate **(14a)** was obtained in 21% (0.025 g, 0.10 mmol) yield. Also 1-propyl-1*H*-benzo[*d*]imidazol-2-amine **(20e)** was obtained in 66% (0.058 g, 0.33 mmol) yield.
- Using 10.0 mol% Zn(OAc)₂.17H₂O, 1.0 equiv of ACDG-NH-CO-NH-ACDG **19d,** 5.0 equiv **11,** *t*BuOAc, 100 °C, 24 h; R¹ = 1-propyl-1*H*-imidazol-2-yl, R³ = NHR¹

Benzyl (1-propyl-1*H*-imidazol-2-yl)carbamate **(21g)** was obtained in 93% (0.060 g, 0.23 mmol) yield. Colorless oil. ¹³C-NMR (100 MHz, CDCl₃): *δ*: 162.9, 150.3, 137.6, 128.2, 127.9, 127.5, 114.1, 111.5, 66.7, 46.1, 22.6, 10.9. Also 1-propyl-1*H*-imidazol-2-amine **(20a)** was obtained in 64% (0.020 g, 0.16 mmol) yield. Yellow oil. ¹³C-NMR (100 MHz, CDCl₃): *δ*: 147.7, 124.0, 115.1,46.5,23.3, 11.2.
- Using 10.0 mol% Zn(OAc)₂.17H₂O, 1.0 equiv of ACDG-NH-CO-NH-R³ **21b,** 3.0 equiv cresol **(11e),** *t*BuOAc, 100 °C, 24 h; R¹ = *tert*-butyl 1*H*-benzo[*d*]imidazol-2-yl-1-carboxylate, R³ = O*t*Bu, 65% NMR-yield of *p*-cresyl *tert*-butyl carbonate **(14f)** with TMB as internal standard was obtained.
- Using 10.0 mol% Zn(OAc)₂.17H₂O, 1.0 equiv of ACDG-NH-CO-NH-R³ **21b,** 3.0 equiv benzylalcohol **(11b),** *t*BuOAc, 100 °C, 24 h; R¹ = *tert*-butyl 1*H*-benzo[*d*]imidazol-2-yl-1-carboxylate, R³ = O*t*Bu, 88% NMR-yield of benzyl *tert*-butyl carbonate **(14g)** with TMB as internal standard was obtained.
- Using 10.0 mol% Zn(OAc)₂.17H₂O, 1.0 equiv of ACDG-NH-CO-NH-R³ **21b,** 30.0 equiv propanol **11,** 40 °C, 24 h; R¹ = *tert*-butyl 1*H*-benzo[*d*]imidazol-2-yl-1-carboxylate, R³ = O*t*Bu, 79% NMR- yield of *tert*-butyl propyl carbonate **(14h)** with TMB as internal standard was obtained.

### Example 10. Scope of (benz)imidazolyl-directed catalytic cleavage of amide- containing compounds with amines 16 (transamidation, transureation)

Amide-containing compounds with (benz)imidazol-2-yl groups can undergo catalytic directed cleavage with an amine nucleophile **16** in the presence of a metal catalyst. The following examples as depicted in Example Scheme E6 can be listed. 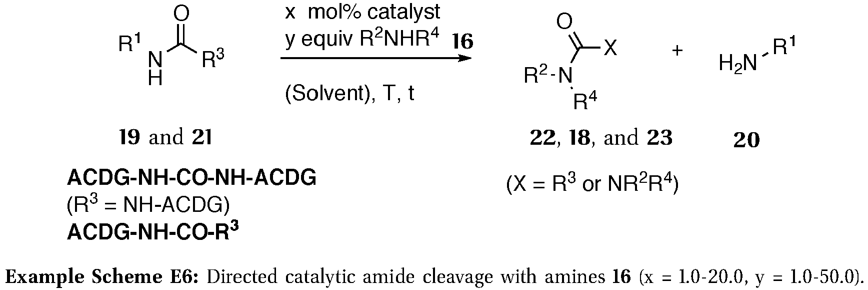
- Using 10.0 mol% Zn(OAc)₂.17H₂O, 1.0 equiv of ACDG-NH-CO-R³ **21d,** 30.0 equiv propylamine **(16a),** 100 °C, 24 h; R¹ = 1*H*-benzo[*d*]imidazol-2-yl, R³ = OMe, results in the following NMR-yields (determined with TMB as an internal standard): 0% **21d,** 19% 2-aminobenzimidazole **(20d),** 6% methyl propyl carbamate **(22a),** 13% dipropylurea **(18b).**
- Using 10.0 mol% Zn(OAc)₂.17H₂O, 1.0 equiv of ACDG-NH-CO-NH-R³ **21b,** 30.0 equiv propylamine **(16a),** 40 °C, 24 h; R¹ = *tert*-butyl 1*H*-benzo[*d*]imidazol-2-yl-1-carboxylate, R³ = O*t*Bu, 76% NMR-yield of *tert*-butyl propylcarbamate **(22b)** with TMB as internal standard was obtained.

### Example 11. Synthesis of amide-containing compounds bearing N-(pyrimidin-2-yl) directing groups

1,3-Di(pyrimidin-2-yl)urea **(24a)** was obtained in 11% (0.250 g, 1.16 mmol) yield via a reaction of 2-aminopyrimidine **(25a)** (2.0 g, 21.03 mmol), triphosgene (1.08 g, 3.63 mmol) and Et₃N (3.54 mL, 25.4 mmol) in CH₂Cl₂ (90.0 mL) at room temperature. Reaction time 16 h. Colorless solid ; m.p. 240 °C. ¹H-NMR (400 MHz, DMSO-d6): *δ*: 11.19 (s, 2H), 8.68 (t, 4H, *J* = 4.80 Hz), 7.18 (t, 4H, *J* = 4.80 Hz).

### Example 12. Catalytic cleavage of amide-containing compounds bearing N - (pyrimidin-2-yl) and N-(pyridin-2-yl) directing groups

For R³ = NH-pyrimidin-2-yl and R¹ = *py* in Example Scheme E5, the following example can be listed:
- Using 10.0 mol% Zn(OAc)₂.17H₂O, 1.0 equiv ACDG-NH-CO-NH-ACDG **24a,** 5.0 equiv benzylalcohol **11b,** *t*BuOAc, 100 °C, 24 h

Benzyl pyrimidin-2-yl carbamate **(26a)** was obtained in 42% (47.6 mg, 0.21 mmol) yield. Colorless oil. ¹³C-NMR (100 MHz, DMSO-d6): *δ*: 158.8, 158.2, 152.3, 137.0, 128.9, 128.4, 128.3, 116.8, 66.2.

For R³ = NH-py and R¹ = *py* in Example Scheme E5, the following example can be listed:
- Using 10.0 mol% Zn(OAc)₂.17H₂O, 1.0 equiv ACDG-NH-CO-NH-ACDG **5a,** 52.0 equiv isopropanol **(11a),** 140 °C, 24 h, a GC-yield of 59% of 2-aminopyridine **(6a)** was determined with TMB as an internal standard (calculated based on the formation of 2.0 equiv 2-aminopyridine **(6a)** = 100 % GC-yield).

For R³ = NH-*py* and R¹ = *py* in Example Scheme E6, the following example can be listed:
- Using 10.0 mol% Zn(OAc)₂.17H₂O, 1.0 equiv ACDG-NH-CO-NH-ACDG **5a,** 26.0 equiv isopropylamine **(16b),** 140 °C, 24 h, a NMR-yield of 55% of 2-aminopyridine **(6a),** 42% of dipropylurea **(18b)** and 53% of propyl pyridin-2-ylurea **(15c)** was determined with TMB as an internal standard (calculated based on the formation of 2.0 equiv 2-aminopyridine **(6a)** = 100% NMR-yield).

For R¹ = NH-*py* and R³ = O(CH₂)₂Ph in Example Scheme E5, the following example can be listed:
- Using 4.0 mol% Zn(OAc)₂.17H₂O, 1.0 equiv ACDG-NH-CO-R' **7a,** 26.0 equiv isopropanol **(11a),** 140 °C, 24 h leads to the formation of 2-aminopyridine **(6a)** in 72% GC-yield (determined with TMB as an internal standard).

### REFERENCES

1. Charette, A. B. & Chua, P. A New Mild Method for the Cleavage of the Amide Bond: Conversion of Secondary and Tertiary Amides to Esters. Synlett 1998, 163 (1998).
2. Glatzhofer, D. T., Roy, R. R. & Cossey, K. N. Conversion of N-aromatic amides to O-aromatic esters. Org. Lett. 4, 2349 (2002).
3. Shendage, D. M., Fröhlich, R. & Haufe, G. Highly efficient stereoconservative amidation and deamidation of α-amino acids. Org. Lett. 6, 3675 (2004).
4. Fisher, L. E. et al. Mild hydrolysis or alcoholysis of amides. Ti(IV) catalyzed conversion of primary carboxamides to carboxylic acids or esters. Can. J. Chem. 72, 142 (1994).
5. Xue, C. & Luo, F.-T. Transformation of Amides into Esters by the Use of Chlorotrimethylsilane. J. Chinese Chem. Soc. 51, 359 (2004).
6. Brown, R., Bennet, A. J. & Slebocka-Tilk, H. Recent Perspectives Concerning the Mechanism of H3O and OH promoted amide hydrolysis. Acc. Chem. Res. 25, 338 (1992).
7. Kita, Y., Nishii, Y., Higuchi, T. & Mashima, K. Zinc-catalyzed amide cleavage and esterification of β-hydroxyethylamides. Angew. Chem. Int. Ed. 51, 5723 (2012).
8. Melnyk, O. & Agouridas, V. From protein total synthesis to peptide transamidation and metathesis: playing with the reversibility of N,S-acyl or N,Se-acyl migration reactions. Curr. Opin. Chem. Biol. 22C, 137 (2014).
9. Aubé, J. A new twist on amide solvolysis. Angew. Chem. Int. Ed. 51, 3063 (2012).
10. Kita, Y., Nishii, Y., Onoue, A. & Mashima, K. Combined catalytic system of scandium triflate and boronic ester for amide bond cleavage. Adv. Synth. Catal. 355, 3391 (2013).
11. Atkinson, B. N. & Williams, J. M. J. Scandium triflate catalyzed ester synthesis using primary amides. Tetrahedron Lett. 55, 6935 (2014).
12. Siddiki, S. M. H., Touchy, A. S., Tamura, M. & Shimizu, K. Versatile and sustainable alcoholysis of amides by a reusable CeO2 catalyst. RSCAdv. 4, 35803 (2014).
13. Becerra-Figueroa, L., Ojeda-Porras, A. & Gamba-Sánchez, D. Transamidation of carboxamides catalyzed by Fe(III) and water. J. Org. Chem. 79, 4544 (2014).
14. Tamura, M., Tonomura, T., Shimizu, K. & Satsuma, A. Transamidation of amides with amines under solvent-free conditions using a CeO2 catalyst. Green Chem. 14, 717 (2012).
15. Lebleu, T., Kotsuki, H., Maddaluno, J. & Legros, J. Formylation of amines through catalyst- and solvent-free transamidation reaction. Tetrahedron Lett. 55, 362 (2014).
16. Vanjari, R., Allam, B. K. & Singh, K. N. A novel and simple transamidation of carboxamides in 1,4-dioxane without a catalyst. Tetrahedron Lett. 54, 2553 (2013).
17. Allen, C. L., Atkinson, B. N. & Williams, J. M. J. Transamidation of primary amides with amines using hydroxylamine hydrochloride as an inorganic catalyst. Angew. Chem. Int. Ed. 51, 1383 (2012).
18. Binh Nguyen, T., Al-Mourabit, A., Ermolenko, L. & Tran Huu Dau, M.-E. Hydrogen Bond Organocatalysis of Benzotriazole in Transamidation of Carboxamides with Amines. Heterocycles 88, 403 (2014).
19. Rao, S. N., Mohan, D. C. & Adimurthy, S. L-Proline: An Efficient Catalyst for Transamidation of Carboxamides with Amines Org. Lett. 15, 1496 (2013).
20. Dineen, T., Zajac, M. a & Myers, A. G. Efficient transamidation of primary carboxamides by in situ activation with N,N-dialkylformamide dimethyl acetals. J. Am. Chem. Soc. 128, 16406 (2006).
21. Shimizu, Y., Morimoto, H., Zhang, M. & Ohshima, T. Microwave-assisted deacylation of unactivated amides using ammonium-salt-accelerated transamidation. Angew. Chem. Int. Ed. Engl. 51, 8564 (2012).
22. Santos, B. V., Silva, V. M. T. M., Loureiro, J. M. & Rodrigues, A. E. Review for the Direct Synthesis of Dimethyl Carbonate. ChemBioEng Rev. 1, 214 (2014).
23. C. Nitschke, G. S. Ullmann's Fine Chem. Encycl. 1273 (2014).
24. Rousseau, G. & Breit, B. Removable directing groups in organic synthesis and catalysis. Angew. Chem. Int. Ed. 50, 2450 (2011).
25. Rouquet, G. & Chatani, N. Catalytic functionalization of C(sp2)-H and C(sp3)-H bonds by using bidentate directing groups. Angew. Chem. Int. Ed. 52, 11726 (2013).
26. Arefi, M. & Heydari, A. Transamidation of primary carboxamides, phthalimide, urea and thiourea with amines using Fe(OH)3@Fe3O4 magnetic nanoparticles as an efficient recyclable catalyst. RSC Adv. 6, 24684 (2016).
27. Fabbrizzi, P., Menchi, G., Raspanti, S., Guarna, A. & Trabocchi, A. Role of Side-Chain Bioisosteres in Determining the Binding Affinity of Click Chemistry Derived RGD Peptidomimetics to α v β 3 Integrin. European J. Org. Chem. 2014, 7595 (2014).
28. Pellizzaro, M. L., Barrett, S., Fisher, J. & Wilson, A. J. Design, synthesis and binding studies of a novel quadruple ADDA hydrogen-bond array. Org. Biomol. Chem. 10, 4899 (2012).
29. Cobo J. et al, "Reactivity of 6-aminopyrimidin-4-(3H)-ones towards dimethyl acetylenedicarboxylate (DMAD). Tandem Diels-Alder/retro Diels-Alder (DA/RDA) reaction in the synthesis of 2-aminopyridines", TETRAHEDRON, ELSEVIER SCIENCE PUBLISHERS, AMSTERDAM, NL, vol. 50, no. 34, 1 January 1994, pages 10345 - 10358.
30. Liana Hie et al, "Conversion of amides to esters by the nickel-catalysed activation of amides C-N bonds.", NATURE, Nature Publishing Group, (20150806), vol. 524, 2015, pages 79-83.
31. Sarah M. Crawford et al, "BippyPhos: a single ligand with unprecedented scope in the Buchwald-Hartwig amination of (hetero)aryl chlorides", CHEMISTRY - A EUROPEAN JOURNAL., USVCH PUBLISHERS., 2013, vol. 19, ISSN 0947-6539, pages 16760 - 16771.

## Claims

1. A method for the catalytic cleavage of primary amide-containing compounds; said method comprising the steps of:
- providing a primary amide-containing (H₂N-CO-) compound;
- converting said compound into an ACDG-containing (amide-cleaving directing group-containing) compound, by introducing an amide-cleaving directing group (ACDG), according to the formula (Ia), on the nitrogen atom of said amide, resulting in the formation of an *N*-ACDG amide of formula ACDG-NH-CO-; 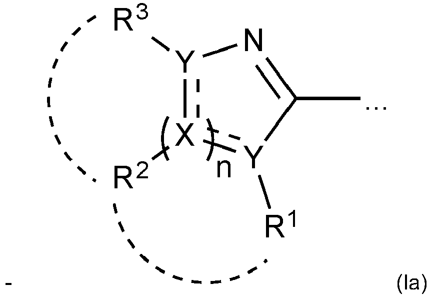 wherein
X is selected from C, N, O or S.
Y is selected from C, N or S; or Y = O or S and R¹ is absent
n is selected from 1, 2, 3, 4, 5, 6, 7 or 8.
R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein each of said aryl, heteroaryl or alkyl is optionally and independently substituted with from 1 to 5 substituents selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy;
- - - is an optional chemical bond; if one or more optional chemical bonds are present, a polycyclic structure is meant;
- cleaving said ACDG-containing compound (namely the *N*-ACDG amide) in the presence of a (hetero)nucleophile; and a metal catalyst according to formula Ib, or a salt, solvate or hydrate thereof
M_{y}Lₓ(Ib)
wherein
M is a metal selected from Zn, Mn, Ni or Co; preferably Zn;
L represents a carboxylate ligand, preferably an acetate ligand;
x is selected from 0-30; and
y is selected from 1, 2, 3 or 4; most preferably 1;
said *N*-ACDG amide is the compound having the structure ACDG-NH-CO-, and within the scope of our definition of *N*-ACDG amide we include groups in which the structural unit ACDG-NHCO-is part of a larger group or structural entity, namely: urea, oxamide, carbamate, thiocarbamate; said (hetero)nucleophile may be comprised in said ACDG-containing compound.

2. A method for the catalytic cleavage of amide-containing compounds; said method comprising the steps of:
- Providing an ACDG-precursor selected from the list comprising an amide-containing compound, LG-CO-LG (LG being a leaving group), CO₂ and CO;
- Converting said ACDG-precursor compound into an ACDG-containing (amide-cleaving directing group-containing) compound, by introducing an amide-cleaving directing group (ACDG), according to the formula (Ia), resulting in the formation of an N-ACDG amide of formula ACDG-NH-CO-, and this by the reaction of said ACDG-precursor with an ACDG-introducing compound in the presence or absence of catalyst(s), ligand(s), reactant(s), reagent(s) and/or solvent(s) to an ACDG-containing (amide-cleaving directing group-containing) compound of the formula ACDG-NH-CO-) 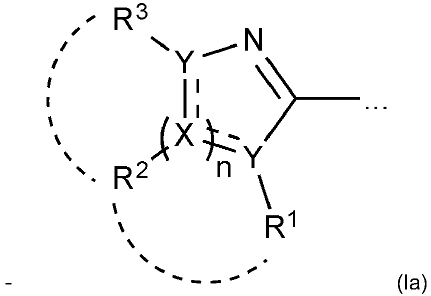 wherein
X is selected from C, N, O or S.
Y is selected from C, N or S; or Y = O or S and R¹ is absent
n is selected from 1, 2, 3, 4, 5, 6, 7 or 8.
R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀alkoxy, aryloxy, heteroaryloxy, C₁₋₁₀alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein each of said aryl, heteroaryl or alkyl is optionally and independently substituted with from 1 to 5 substituents selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy;
- - - is an optional chemical bond; if one or more optional chemical bonds are present, a polycyclic structure is meant;
- cleaving said ACDG-containing compound (namely the *N*-ACDG amide) in the presence of a (hetero)nucleophile; and a metal catalyst according to formula Ib, or a salt, solvate or hydrate thereof
M_{y}Lₓ (Ib)
wherein
M is selected from Zn, Mn, Ni or Co; preferably Zn;
L represents a carboxylate ligand, preferably an acetate ligand;
x is selected from 0-30; and
y is selected from 1, 2, 3 or 4; most preferably 1;
said *N*-ACDG amide is the compound having the structure ACDG-NH-CO-, and within the scope of our definition of *N*-ACDG amide we include groups in which the structural unit ACDG-NHCO-is part of a larger group or structural entity, namely: urea, oxamide, carbamate, thiocarbamate; said (hetero)nucleophile may be comprised in said ACDG-containing compound.

3. The method according to anyone of claims 1 or 2, wherein said amide-cleaving directing group (ACDG) is a hydrogen bond-based amide-cleaving directing group (HBDG).

4. The method according to anyone of claims 1 to 3, wherein said *N*-ACDG amide is an *Nⁿ*-ACDGₙ amide that is cleaved 1 to n times in the presence of a metal catalyst and a heteronucleophile, after the n introductions of the ACDG in 1 to maximum n steps; for the defined maximum number of n cleavage steps, the maximum number of n different heteronucleophiles can be used, with the minimum number of heteronucleophiles being 1; Said *Nⁿ*-ACDGₙ amide is a compound having a structure wherein the structural motive ACDG-NH-CO- can be found n times; When identifying the sum of the number of ACDG-NH-CO structural motives, a -CO- moiety can be used one or two times to identify this motive; said *Nⁿ*-ACDGₙ amide is preferably an *Nⁿ*-HBDGₙ amide; said *Nⁿ*-HBDGₙ amide is a compound having a structure wherein the structural motive HBDG-NH-CO- can be found n times; When identifying the sum of the number of HBDG-NH-CO structural motives, a -CO- moiety can be used one or two times to identify this motive; Additionally when said *Nⁿ*-ACDGₙ amide is an *N*,*N'*-ACDG₂ amide, the *N*,*N'-*ACDG₂ amide preferably has the general structure HBDG-NH-CO-X-CO-NH-HBDG, in which X can consists of any functional group or combinations of functional groups; additionally, in all of the above cases said n heteronucleophiles (if n = 1 or 2) can be part of the *Nⁿ*-ACDGₙ amide, thus resulting in an intramolecular cleavage reaction; additionally if n = 2 said *Nⁿ*-ACDGₙ is an *N,N'*-ACDG₂ amide that preferably has the general structure ACDG-NH-CO-NH-ACDG and that can undergo smart intermolecular amide cleavage, since metal-catalyst activation is needed for reaction with a heteronucleophile preferably not included in the *N,N'*-ACDG₂ moiety, and since by smart temperature regulation the number of cleavages (1 or 2) in one reaction step can be regulated, thus allowing the use of two different heteronucleophiles, preferably not included in the *N,N'*-ACDG₂ moiety, for two subsequent cleavage reactions via a two-step protocol; in all of the above cases, said ACDG is preferably an HBDG that is preferably C3-substituted pyridin-2-yl moiety, preferably an alkyl nicotinate (like a tert-butyl nicotinate) or a 3-alkoxy pyridin-2-yl moiety; or an an imidazol-2-yl moiety that is a HBDG, preferentially being an alkyl 1*H*-imidazole-1-carboxylate (like a *tert*-butyl 1*H*-imidazole-1-carboxylate).

5. The method according to anyone of claims 1 to 4 wherein the directing *N*-ACDG amide is selected from the list comprising:
- *N*-(pyridin-2-yl)amide, *N*-(n-C1-10 alkoxypyridin-2-yl) amide, with n = 3-6 and alkoxy being optionally substituted;
- *N*-(n-halopyridin-2-yl)amide; with n = 3-6;
- C1-10 alkyl n-amidonicotinate, with n = 2 or 6 and alkyl being optionally substituted;
- *N*-(n-nitropyridin-2-yl)amide, with n = 3-6;
- *N*-(n-cyanopyridin-2-yl)amide, with n = 3-6;
- *N*-(n-trifluoromethylpyridin-2-yl)amide, with n = 3-6;
- *N*-(n-C1-10 alkylpyridin-2-yl)amide, with n = 3-6 and alkyl being optionally substituted;
- *N*-(n-arylpyridin-2-yl)amide, with n = 3-6 and aryl being optionally substituted;
- *N*-(n-heteroarylpyridin-2-yl)amide, with n = 3-6 and heteroaryl being optionally substituted;
- *N*-(n-(C1-10 alkoxymethyl)pyridin-2-yl)amide, with n = 3-6 and C1-10 alkoxymethyl being optionally substituted;
- *N*-(n-(di-C1-10 alkylamino)pyridin-2-yl)amide, with n = 3-6 and alkyl being optionally substituted;
- *N*-(n-(amino C1-10 alkyl)pyridin-2-yl)amide, with n = 3-6 and alkyl being optionally substituted;
- S-C1-10 alkyl 2-benzamidopyridine-3-carbothioate, with alkyl being optionally substituted;
- *N*-(n-(C1-10 alkylthio C1-10 alkyl)pyridin-2-yl)amide, with n = 3-6 and alkyl being optionally substituted;
- 2-amido-*N*-C1-10 alkylnicotinamide, with alkyl being optionally substituted;,
- *N*-(n-C1-10 alkylcarbonylpyridin-2-yl)amide, with n = 3-6 and alkyl being optionally substituted;
- *N*-(1-oxo-3,4-dihydro-1*H*-pyrano[3,4-*c*]pyridin-8-yl)amide;
- *N*-(8-oxo-5,6,7,8-tetrahydroisoquinolin-1-yl)amide;
- *N*-(8-oxo-5,6,7,8-tetrahydro-2,7-naphthyridin-1-yl)amide;
- *N*-(1-oxo-3,4-dihydro-1*H*-thiopyrano[3,4-*c*]pyridin-8-yl)amide;
- *N*-(3,4-dihydro-2*H*-pyrano[2,3-*c*]pyridin-8-yl)amide;
- *N*-(7-oxo-6,7-dihydro-5*H*-cyclopenta[*c*]pyridin-1-yl)amide;
- *N*-(3-oxo-1,3-dihydrofuro[3,4-*c*]pyridin-4-yl)amide;
- *N*-(3-oxo-1,3-dihydrothieno[3,4-*c*]pyridin-4-yl)amide;
- *N*-(3-oxo-2,3-dihydro-1*H*-pyrrolo[3,4-*c*]pyridin-4-yl)amide;
- *N*-(2,3-dihydrofuro[2,3-*c*]pyridin-7-yl)amide;
wherein in all instances, 'amide' is a moiety of the general structure according to formula (Ib); 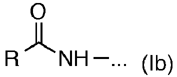 wherein R is selected from the list comprising hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀alkyl aryl amino, C₁₋₁₀alkyl heteroaryl amino, heteroaryl arylamino, diheteroarylamino, diarylamino, heteroaryl arylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, or heteroarylamino.

6. Use of an *N*-ACDG amide in a method according to anyone of claims 1-4; wherein the ACDG is a pyridin-2-yl directing group attached to the *N*-atom of an amide according to formula (IIa), or a tautomer, isomer, salt, hydrate or solvate thereof; 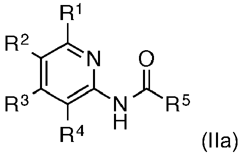 wherein
R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, nitro, halo, hydroxyl, amino, thio, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido,
alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy;
R⁴ is hydrogen, halo, nitro, cyano, trifluoromethyl, aryl, heteroaryl, C₁₋₁₀ alkyl, C₁₋₁₀ alkenyl, C₁₋₁₀ alkynyl, arylalkyl or heteroarylalkyl;
or R⁴ is XR⁶ wherin X is -S-, -O-, -N-, -NH-, -C=O-, -C=N-R⁷ or -C=S-,
R⁶ is hydrogen, aryl, heteroaryl, heteroarylalkyl, arylalkyl, C₁₋₂₅ alkyl, C₁₋₂₅ alkenyl, C₁₋₂₅ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy or arylcarbonyl;
R⁷ is hydrogen, aryl, heteroaryl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino or heteroarylamino;
R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, heteroaryl aryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino or heteroarylamino.

7. Use of a compound of the general structure *N*-HBDG amide in a method according to anyone of claims 1 to 4; wherein the HBDG is an *N*-(3-C₁₋₁₀ alkoxy pyridin-2-yl) or *N*-(3-C₁₋₁₀ alkoxymethyl pyridin-2-yl) directing group attached to the *N*-atom of said amide according to respectively formula (IIc) or (IId), or a tautomer, isomer, salt, hydrate, or solvate thereof; 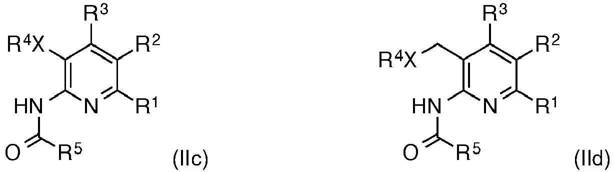 wherein
X is O, NR⁶, or S;
R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy;
R⁴ and and R⁶ are each independently selected from the list comprising: hydrogen, C₁₋₁₀ alkyl, C₁₋₂₅ alkenyl, C₁₋₂₅ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diheteroarylamino, diarylamino, aryl heteroarylamino, arylthio, heteroarylthio, aryloxy, heteroaryloxy;
R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, heteroaryl aryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.

8. Use of a compound of the general structure *N*-HBDG amide in a method according to anyone of claims 1 to 4 wherein the HBDG is a C₁₋₁₀ alkyl nicotinate directing group attached to the *N*-atom of said amide according to formula (IIe) with R⁴ preferably being an alkyl substituent and preferably being a *t*Bu-group like in formula (IIf); or a tautomer, isomer, salt, hydrate, or solvate thereof; 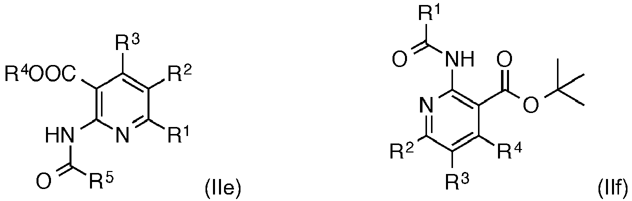 wherein
R¹, R² and R³ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl diarylamino, diheteroarylamino, aryl heteroarylamino, arylthio, heteroarylthio, aryloxy, heteroaryloxy;
R⁴ is a hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroarylamino, diarylamino, diheteroarylamino, aryl heteroarylamino, arylthio, heteroarylthio, aryloxy or heteroaryloxy;
R⁵ is hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl.

9. A compound of the general structure *N*-HBDG amide wherein the HBDG is a *tert*-butyl nicotinate directing group attached to the *N*-atom of said amide according to formula (IIf), or a tautomer, isomer, salt, hydrate, or solvate thereof; 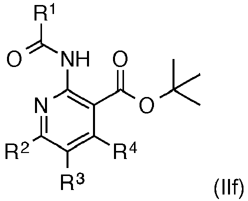 wherein
R¹ is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, diarylamino, heteroarylamino, diheteroarylamino, heteroaryl aryl amino;
R², R³ and R⁴ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, heteroaryl arylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino;
wherein R² is not selected from 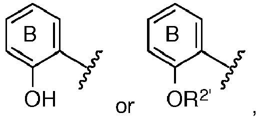 and wherein B represents a benzene ring optionally having a further substituent; and wherein
R^{2'} represents a hydrogen atom, a lower alkyl group having from 1 to 6 carbons, which may be substituted, an aryl group having from 6 to 12 carbons, which may be substituted, a heteroaryl group having from 4 to 11 carbons, which may be substituted, an arylalkyl group having from 7 to 14 carbons, which may be substituted, a heteroarylalkyl group having from 5 to 13 carbons, which may be substituted, or an acyl group having from 2 to 11 carbons; and
wherein said compound is not N-(3-tert-Butoxycarbonylpyridin-2-yl)-5-(5,6-dimethoxy-3-methyl-1,4-benzoguinon-2-yl)methyl-2-acetoxybenzamide

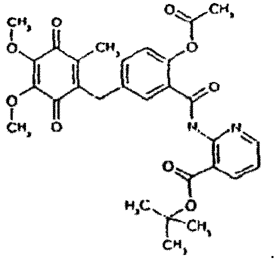

10. Use of a compound of the general formula (IIg) in a method according to anyone of claims 1 to 4; 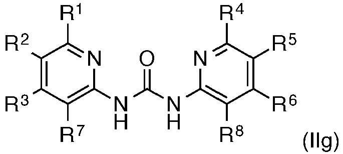 wherein
R¹, R², R³, R⁴, R⁵, R⁶, R⁷ and R⁸ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diheteroarylamino, diarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino.

11. A compound of formula (IIj) (with R⁷X preferably being O-C₁₋₁₀ alkyl or said compound having the structure according to formula (Ilk)), or a tautomer, isomer, salt, hydrate, or solvate thereof; 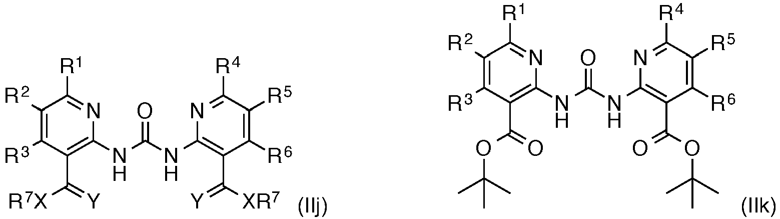 wherein
R¹, R², R³, R⁴, R⁵, and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino;
X = O, NR⁸, or S;
R⁷ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino;
R⁸ = hydrogen, aryl, heteroaryl, heteroarylalkyl, arylalkyl, C₁₋₁₀ alkyl, C₁₋₁₀ alkenyl, C₁₋₁₀ alkynyl; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino;
Y is O, S or NR⁹;
R⁹ = hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.

12. Use of an *N*-ACDG amide in a method according to anyone of claims 1 to 4; wherein the ACDG is an imidazol-2-yl directing group attached to the *N*-atom of an amide according to formula (IIIa) or a tautomer, isomer, salt, hydrate or solvate thereof; 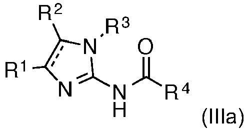 wherein
R¹ and R² are each independently selected from the list comprising: hydrogen, aryl, heteroaryl, heteroarylalkyl, arylalkyl, C₁₋₂₅ alkyl, C₁₋₂₅ alkenyl, C₁₋₂₅ alkynyl, trifluoromethyl, cyano, nitro, halo, thio, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, arylthio, heteroarylthio, aryloxy, heteroaryloxy, diarylamino, diheteroarylamino, aryl heteroarylamino;
R³ is selected from the list comprising hydrogen, aryl, heteroaryl, heteroarylalkyl, arylalkyl, C₁₋₂₅ alkyl, C₁₋₁₀ alkenyl, C₁₋₂₅ alkynyl, arylalkyl, heteroarylalkylor; R³ is preferentially XR⁵ wherin X is -C=O-, -C=N-R⁶ or -C=S-;
R⁵ is hydrogen, aryl, heteroaryl, heteroarylalkyl, arylalkyl, C₁₋₂₅ alkyl, C₁₋₂₅ alkenyl, C₁₋₂₅ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl;
R⁶ is hydrogen, aryl, heteroaryl, arylakyl, heteroarylalkyl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino.
R⁴ is a hydrogen, aryl, heteroaryl, heteroarylalkyl, arylalkyl, C₁₋₂₅ alkyl, C₁₋₂₅ alkenyl, C₁₋₂₅ alkynyl, arylamino, heteroarylamino, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy, hydroxyl, amino, thio or a pharmaceutically acceptable salt thereof, or an enantiomer, or a stereoisomeric form thereof, or a solvate thereof, wherein said aryl, heteroaryl, alkoxy or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino;
Additionally, R³ is most preferably a COOR⁸-group with R⁸ selected from the list comprising hydrogen, C₁₋₁₀ alkyl, C₁₋₁₀ alkenyl, C₁₋₁₀ alkynyl, aryl, heteroaryl, arylalkyl, heteroarylalkyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroarylamino, arylthio, heteroarylthio, aryloxy or heteroaryloxy, diarylamino, diheteroarylamino, aryl heteroarylamino; and R⁸ preferably being a C₁₋₁₀ alkyl group, preferably begin a *t*Bu-group.

13. A compound according to the formula (IIId) or a tautomer, isomer, salt, hydrate, or solvate thereof; 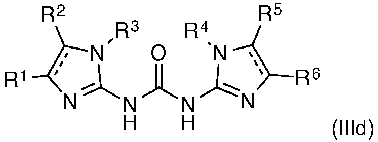 wherein
R¹, R², R⁵ and R⁶ are each independently selected from the list comprising: hydrogen, aryl, heteroaryl, heteroarylalkyl, arylalkyl, C₁₋₁₀ alkyl, C₁₋₁₀ alkenyl, C₁₋₁₀ alkynyl, trifluoromethyl, cyano, thio, nitro, halo, hydroxyl, amino, acylamino, C₁₋₁₀ alkoxy, aryloxy, heteroaryloxy, C₁₋₁₀ alkylthio, C₁₋₁₀ alkylamino, C₁₋₁₀ dialkylamino, C₁₋₁₀ alkoxycarbonyl, C₁₋₁₀ alkyl esters, C₁₋₁₀ alkyl aryl amino, C₁₋₁₀ alkyl heteroaryl amino, diarylamino, diheteroarylamino, aryl heteroarylamino, arylthio, heteroarylthio; wherein said aryl, heteroaryl or alkyl is optionally substituted with one to ten substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino, C₁₋₁₀ alkyl aryl amino, C₁₋₁₀ alkyl heteroaryl amino, diarylamino, diheteroarylamino, aryl heteroarylamino, arylthio, heteroarylthio, aryloxy, heteroaryloxy;
R³ and R⁴ are each independently selected from the list comprising hydrogen, aryl, heteroaryl, heteroarylalkyl, arylalkyl, C₁₋₂₅ alkyl, C₁₋₁₀ alkenyl, C₁₋₂₅ alkynyl, arylalkyl, heteroarylalkyl or R³ and/or R⁴ is XR⁷ wherein X is -C=O-, -C=N-R⁸, -C=S-;
R⁷ is hydrogen, aryl, heteroaryl, heteroarylalkyl, arylalkyl, C₁₋₁₀ alkyl, C₁₋₁₀ alkenyl, C₁₋₁₀ alkynyl, C₁₋₁₀ alkoxy, C₁₋₁₀ alkylthio, C₁₋₁₀ alkylamino, C₁₋₁₀ dialkylamino, C₁₋₁₀ alkyl aryl amino, C₁₋₁₀ alkyl heteroaryl amino, diarylamino, diheteroarylamino, aryl heteroarylamino, arylamino, heteroarylamino, arylthio, heteroarylthio, aryloxy, heteroaryloxy, thio, amino or hydroxyl; wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, arylamino, heteroarylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl;
R⁸ is hydrogen, aryl, heteroaryl, C₁₋₁₀ alkyl, sulfinyl, sulfonyl, wherein said aryl, heteroaryl or alkyl is optionally substituted with one to five substituents independently selected from the group consisting of halogen, aryl, heteroaryl, C₁₋₁₀ alkyl, trifluoromethyl, C₁₋₁₀ alkoxy, C₁₋₁₀ alkoxycarbonyl, trifluoromethoxy, C₁₋₁₀ alkylthio, cyano, C₁₋₁₀ alkylamino, nitro, hydroxyl, carbonyl, amino, oxime, imino, azido, hydrazine, acyl, C₁₋₁₀ alkylamino, C₁₋₁₀ dialkylamino, carboxylic acid, acylamino, C₁₋₁₀ alkyl esters, carbamate, thioamido, urea, sulfonamido, alkylsulfoxide, haloalkyl, alkenyl, alkynyl, arylalkyl, heteroarylalkyl, alkylcarbonylaminoalkyl, aryloxy, arylcarbonyl, arylamino, heteroarylamino;
R³ and R⁴ are not both hydrogen.

14. The use of the method according to anyone of claims 1 to 5 for the synthesis of a compound selected from the list comprising: amide-containing compounds, esters, thioesters, carbonates, thiocarbonates, thiocarbamates, polycarbonates, polyurea and polycarbamates.

## Patentansprüche

1. Verfahren zur katalytischen Spaltung von primären amidhaltigen Verbindungen; wobei das Verfahren die folgenden Schritte umfasst:
- Bereitstellen einer primären amidhaltigen (H₂N-CO-) Verbindung;
- Umwandeln der Verbindung in eine ACDG-haltige (Amid-abspaltende, richtungsweisende Gruppe enthaltende) Verbindung durch Einführen einer Amid-abspaltenden, richtungsweisenden Gruppe (ACDG), gemäß der Formel (la), an dem Stickstoffatom des Amids, was zur Bildung eines *N*-ACDG-Amids der Formel ACDG-NH-CO- führt; 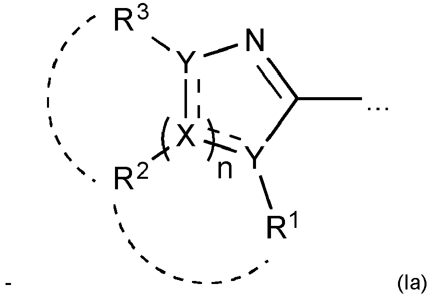 wobei
X ausgewählt ist aus C, N, O oder S.
Y ausgewählt ist aus C, N oder S; oder Y = O oder S und R¹ abwesend ist n ausgewählt ist aus 1, 2, 3, 4, 5, 6, 7 oder 8.
R¹, R² und R³ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₂₅-Alkinyl, Trifluormethyl, Cyano, Nitro, Halo, Thio, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino, C₁₋₁₀-Alkoxycarbonyl, C₁₋₁₀-Alkylester, C₁₋₁₀-Alkyl-Arylamino, C₁₋₁₀-Alkyl-Heteroarylamino, Diheteroarylamino, Diarylamino, Aryl-Heteroarylamino, Arylthio, Heteroarylthio; wobei jedes der Aryl-, Heteroaryl- oder Alkylreste wahlweise und unabhängig mit 1 bis 5 Substituenten substituiert ist, ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀ -Alkyl-Heteroarylamino, Diarylamino, Diheteroarylamino, Aryl-Heteroarylamino, Arylthio, Heteroarylthio, Aryloxy, Heteroaryloxy;
- - - eine wahlweise chemische Bindung ist; wenn eine oder mehrere wahlweise chemische Bindungen vorliegen, ist eine polycyclische Struktur gemeint;
- Spaltung der ACDG-haltigen Verbindung (nämlich des *N*-ACDG-Amids) in Gegenwart eines (Hetero)nucleophils; und eines Metallkatalysators gemäß Formel Ib, oder eines Salzes, Solvats oder Hydrats davon
M_{y}Lₓ (Ib)
wobei
M ein Metall ist, ausgewählt aus Zn, Mn, Ni oder Co; vorzugsweise Zn;
L einen Carboxylat-Liganden, vorzugsweise einen Acetat-Liganden, darstellt;
x ausgewählt ist aus 0-30; und
y ausgewählt ist aus 1, 2, 3 oder 4; am meisten bevorzugt 1;
*N*-ACDG-Amid die Verbindung ist, die die Struktur ACDG-NH-CO- aufweist, und im Umfang unserer Definition von *N*-ACDG-Amid Gruppen beinhaltet, in denen die Struktureinheit ACDG-NHCO- Teil einer größeren Gruppe oder strukturellen Einheit ist, nämlich: Harnstoff, Oxamid, Carbamat, Thiocarbamat;
(Hetero)nucleophil in der ACDG-haltigen Verbindung enthalten sein kann.

2. Verfahren zur katalytischen Spaltung von amidhaltigen Verbindungen; wobei das Verfahren die folgenden Schritte umfasst:
- Bereitstellen eines ACDG-Vorläufers, ausgewählt aus der Liste, umfassend eine amidhaltige Verbindung, LG-CO-LG (wobei LG eine Abgangsgruppe ist), CO₂ und CO;
- Umwandeln der ACDG-Vorläuferverbindung in eine ACDG-haltige (eine Amid-abspaltende richtungsweisende Gruppe enthaltende) Verbindung durch Einführen einer Amid-abspaltenden richtungsweisenden Gruppe (ACDG) gemäß der Formel (la), was zur Bildung eines N-ACDG-Amids der Formel ACDG-NH-CO- führt, und dies durch die Reaktion des ACDG-Vorläufers mit einer ACDG-einführenden Verbindung in Gegenwart oder Abwesenheit von Katalysator(en), Ligand(en), Reaktant(en), Reagenz(en) und/oder Lösungsmittel(n) zu einer ACDG-enthaltenden (Amid-spaltende richtungsweisende Gruppe enthaltende) Verbindung der Formel ACDG-NH-CO-) 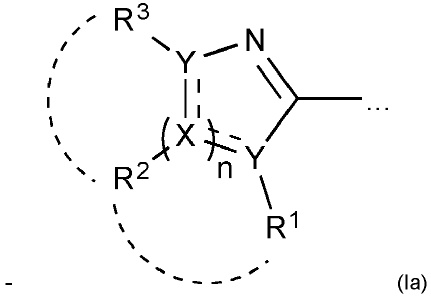 wobei
X ausgewählt ist aus C, N, O oder S.
Y ausgewählt ist aus C, N oder S; oder Y = O oder S und R¹ abwesend ist n ausgewählt ist aus 1, 2, 3, 4, 5, 6, 7 oder 8.
R¹, R² und R³ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₂₅-Alkinyl, Trifluormethyl, Cyano, Nitro, Halo, Thio, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino, C₁₋₁₀-Alkoxycarbonyl, C₁₋₁₀-Alkylester, C₁₋₁₀-Alkyl-Arylamino, C₁₋₁₀-Alkyl-Heteroarylamino, Diheteroarylamino, Diarylamino, Aryl-Heteroarylamino, Arylthio, Heteroarylthio; wobei jedes der Aryl-, Heteroaryl- oder Alkylreste wahlweise und unabhängig mit 1 bis 5 Substituenten substituiert ist, ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀ -Alkyl-Heteroarylamino, Diarylamino, Diheteroarylamino, Aryl-Heteroarylamino, Arylthio, Heteroarylthio, Aryloxy, Heteroaryloxy;
- - - eine wahlweise chemische Bindung ist; wenn eine oder mehrere wahlweise chemische Bindungen vorliegen, ist eine polycyclische Struktur gemeint;
- Spaltung der ACDG-haltigen Verbindung (nämlich des *N*-ACDG-Amids) in Gegenwart eines (Hetero)nucleophils; und eines Metallkatalysators gemäß Formel Ib, oder eines Salzes, Solvats oder Hydrats davon
M_{y}Lₓ (Ib)
wobei
M ausgewählt aus Zn, Mn, Ni oder Co; vorzugsweise Zn;
L einen Carboxylat-Liganden, vorzugsweise einen Acetat-Liganden, darstellt;
x ausgewählt ist aus 0-30; und
y ausgewählt ist aus 1, 2, 3 oder 4; am meisten bevorzugt 1;
*N*-ACDG-Amid die Verbindung ist, die die Struktur ACDG-NH-CO- aufweist, und im Umfang unserer Definition von *N*-ACDG-Amid Gruppen beinhaltet, in denen die Struktureinheit ACDG-NHCO- Teil einer größeren Gruppe oder strukturellen Einheit ist, nämlich: Harnstoff, Oxamid, Carbamat, Thiocarbamat;
(Hetero)nucleophil in der ACDG-haltigen Verbindung enthalten sein kann.

3. Verfahren nach einem der Patentansprüche 1 oder 2, wobei die Amid-spaltende richtungsweisende Gruppe (ACDG) eine auf Wasserstoffbrückenbindung basierende Amid-spaltende richtungsweisende Gruppe (HBDG) ist.

4. Verfahren nach einem der Patentansprüche 1 bis 3, wobei *N*-ACDG-Amid ein *Nⁿ*-ACDGₙ-Amid ist, das in Gegenwart eines Metallkatalysators und eines Heteronucleophils nach den n Einführungen des ACDG in 1 bis maximal n Schritten 1 bis n-mal gespalten wird; für die definierte maximale Anzahl von n Spaltungsschritten kann die maximale Anzahl von n verschiedenen Heteronucleophilen verwendet werden, wobei die minimale Anzahl von Heteronucleophilen 1 ist; wobei das *Nⁿ*-ACDGₙ -Amid eine Verbindung mit einer Struktur ist, wobei das Strukturmotiv ACDG-NH-CO- n-mal zu finden ist; Wenn die Summe der Anzahl von ACDG-NH-CO-Strukturmotiven identifiziert wird, kann eine -CO-Einheit ein- oder zweimal verwendet werden, um dieses Motiv zu identifizieren; das *Nⁿ*-ACDGₙ-Amid ist vorzugsweise ein *Nⁿ*-HBDGₙ-Amid; das *Nⁿ*-HBDGₙ-Amid ist eine Verbindung mit einer Struktur, wobei das Strukturmotiv HBDG-NH-CO- n-mal gefunden werden kann; Wenn die Summe der Anzahl von HBDG-NH-CO-Strukturmotiven identifiziert wird, kann eine -CO- Einheit ein- oder zweimal verwendet werden, um dieses Motiv zu identifizieren; zusätzlich, wenn das *Nⁿ*-ACDGₙ-Amid ein *N*,*N*'-ACDG₂-Amid ist, weist das *N*,*N'*-ACDG₂-Amid vorzugsweise die allgemeine Struktur HBDG-NH-CO-X-CO-NH-HBDG auf, in der X aus einer beliebigen funktionellen Gruppe oder Kombinationen von funktionellen Gruppen bestehen kann; zusätzlich können in allen vorstehenden Fällen die n Heteronucleophile (wenn n = 1 oder 2) Teil des *Nⁿ*-ACDGₙ-Amids sein, was somit zu einer intramolekularen Spaltungsreaktion führt; zusätzlich, wenn n = 2, ist das *Nⁿ*-ACDGₙ ein *N,N'*-ACDG₂-Amid, das vorzugsweise die allgemeine Struktur ACDG-NH-CO-NH-ACDG aufweist und das einer intelligenten intermolekularen Amidspaltung unterzogen werden kann, da eine Metallkatalysatoraktivierung für die Reaktion mit einem Heteronucleophil benötigt wird, das vorzugsweise nicht im *N*,*N'*-ACDG₂-Anteil enthalten ist, und da durch intelligente Temperaturregelung die Anzahl der Spaltungen (1 oder 2) in einem Schritt der Reaktion reguliert werden kann, wodurch die Verwendung von zwei verschiedenen Heteronucleophilen, die vorzugsweise nicht in dem *N,N*'-ACDG₂ -Anteil enthalten sind, für zwei nachfolgende Spaltungsreaktionen über ein zweistufiges Protokoll ermöglicht wird; in allen vorhergehenden Fällen ist das ACDG vorzugsweise ein HBDG, das vorzugsweise ein C3-substituierter Pyridin-2-yl-Anteil ist, vorzugsweise ein Alkylnicotinat (wie ein tert-Butylnicotinat) oder ein 3-Alkoxy-Pyridin-2-yl-Anteil; oder ein Imidazol-2-yl-Anteil, der ein HBDG ist, vorzugsweise ein Alkyl-1*H*-imidazol-1-carboxylat (wie ein *tert*-Butyl 1*H*-imidazol-1-carboxylat).

5. Verfahren nach einem der Patentansprüche 1 bis 4, wobei das richtungsweisende *N*-ACDG-Amid ausgewählt ist aus der Liste, umfassend:
- *N*-(pyridin-2-yl)amid, *N*-(n-C1-10-Alkoxypyridin-2-yl)amid, wobei n = 3-6 und Alkoxy wahlweise substituiert ist;
- *N*-(n-Halopyridin-2-yl)amid; mit n = 3-6;
- C1-10-Alkyl-n-amidonicotinat, wobei n = 2 oder 6 und Alkyl wahlweise substituiert ist;
- *N*-(n-Nitropyridin-2-yl)amid,mit n = 3-6;
- *N*-(n-Cyanopyridin-2-yl)amid, mit n = 3-6;
- *N*-(n-Trifluormethylpyridin-2-yl)amid, mit n = 3-6;
- *N*-(n-C1-10-Alkylpyridin-2-yl)amid, wobei n = 3-6 und Alkyl wahlweise substituiert ist;
- *N*-(n-Arylpyridin-2-yl)amid, wobei n = 3-6 und Aryl wahlweise substituiert ist;
- *N*-(n-Heteroarylpyridin-2-yl)amid,wobei n = 3-6 und Heteroaryl wahlweise substituiert ist;
- *N*-(n-(C1-10-Alkoxymethyl)pyridin-2-yl)amid, wobei n = 3-6 und C1-10-Alkoxymethyl wahlweise substituiert ist;
- *N*-(n-(Di-C1-10-alkylamino)pyridin-2-yl)amid, wobei n = 3-6 und Alkyl wahlweise substituiert ist;
- *N*-(n-(Amino-C1-10-alkyl)pyridin-2-yl)amid, wobei n = 3-6 und Alkyl wahlweise substituiert ist;
- S-C1-10 Alkyl 2-benzamidopyridin-3-carbothioat, wobei Alkyl wahlweise substituiert ist;
- *N*-(n-(C1-10-Alkylthio-C1-10-alkyl)pyridin-2-yl)amid, wobei n = 3-6 und Alkyl wahlweise substituiert ist;
- 2-amido-*N*-C1-10-alkylnicotinamid, wobei Alkyl wahlweise substituiert ist;,
- *N*-(n-C1-10-Alkylcarbonylpyridin-2-yl)amid, wobei n = 3-6 und Alkyl wahlweise substituiert ist;
- *N*-(1-oxo-3,4-dihydro-1*H*-pyrano[3,4-*c*]pyridin-8-yl)amid;
- *N*-(8-oxo-5,6,7,8-tetrahydroisoquinolin-1-yl)amid;
- *N*-(8-oxo-5,6,7,8-tetrahydro-2,7-naphthyridin-1-yl)amid;
- *N*-(1-oxo-3,4-dihydro-1*H*-thiopyrano[3,4-c]pyridin-8-yl)amid;
- *N*-(3,4-dihydro-2*H*-pyrano[2,3-*c*]pyridin-8-yl)amid;
- *N*-(7-oxo-6,7-dihydro-5*H*-cyclopenta[*c*]pyridin-1-yl)amid;
- *N*-(3-oxo-1,3-dihydrofuro[3,4-*c*]pyridin-4-yl)amid;
- *N*-(3-oxo-1,3-dihydrothieno[3,4-*c*]pyridin-4-yl)amid;
- *N*-(3-oxo-2,3-dihydro-1*H*-pyrrolo[3,4-*c*]pyridin-4-yl)amid;
- *N*-(2,3-Dihydrofuro[2,3-*c*]pyridin-7-yl)amid;
wobei in allen Instanzen "Amid" ein Anteil der allgemeinen Struktur gemäß der Formel (Ib) ist; 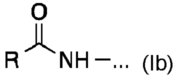 wobei R ausgewählt ist aus der Liste umfassend Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Heteroaryl-Arylamino, Diheteroarylamino, Diarylamino, Heteroaryl-Arylamino, Arylthio, Heteroarylthio, Aryloxy, Heteroaryloxy, Hydroxyl, Amino, Thio oder ein pharmazeutisch verträgliches Salz davon oder ein Enantiomer oder eine stereoisomere Form davon oder ein Solvat davon, wobei das Aryl, Heteroaryl, Alkoxy oder Alkyl wahlweise mit einem bis zehn Substituenten substituiert ist, unabhängig ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino oder Heteroarylamino.

6. Verwendung eines *N*-ACDG-Amids in einem Verfahren nach einem der Patentansprüche 1-4, wobei das ACDG eine an das *N*-Atom eines Amids der Formel (IIa) gebundene richtungsweisende Pyridin-2-yl-Gruppe ist, oder ein Tautomer, Isomer, Salz, Hydrat oder Solvat davon; 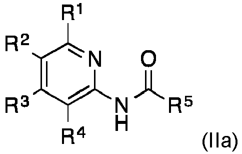 wobei
R¹, R² und R³ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Nitro, Halogen, Hydroxyl, Amino, Thio, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino, C₁₋₁₀-Alkoxycarbonyl, C₁₋₁₀-Alkylester, C₁₋₁₀-Alkyl-Arylamino, C₁₋₁₀-Alkyl-Heteroarylamino, Arylthio, Heteroarylthio; wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀-Alkyl-Heteroarylamino, Arylthio, Heteroarylthio, Aryloxy, Heteroaryloxy;
R⁴ Wasserstoff, Halogen, Nitro, Cyano, Trifluormethyl, Aryl, Heteroaryl, C₁₋₁₀-Alkyl, C₁₋₁₀-Alkenyl, C₁₋₁₀-Alkinyl, Arylalkyl oder Heteroarylalkyl ist;
oder R⁴ ist XR⁶, wobei X -S-, -O-, -N-, -NH-, -C=O-, -C=N-R⁷ oder -C=S- ist,
wobei R⁶ Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Arylamino, Heteroarylamino, Arylthio, Heteroarylthio, Aryloxy, Heteroaryloxy, Thio, Amino oder Hydroxyl ist; wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Arylamino, Heteroarylamino Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy oder Arylcarbonyl;
wobei R⁷ Wasserstoff, Aryl, Heteroaryl, C₁₋₁₀-Alkyl, Sulfinyl, Sulfonyl ist, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino oder Heteroarylamino;
wobei R⁵ Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Diarylamino, Diheteroarylamino, Heteroaryl-Arylamino, Arylthio, Heteroarylthio, Aryloxy, Heteroaryloxy, Hydroxyl, Amino, Thio oder ein pharmazeutisch akzeptables Salz davon oder ein Enantiomer oder eine stereoisomere Form davon oder ein Solvat davon ist, wobei das Aryl, Heteroaryl, Alkoxy oder Alkyl wahlweise mit einem bis zehn Substituenten substituiert ist, unabhängig ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino oder Heteroarylamino.

7. Verwendung einer Verbindung der allgemeinen Struktur *N*-HBDG-Amid in einem Verfahren nach einem der Patentansprüche 1 bis 4, wobei das HBDG eine an das *N*-Atom des Amids gebundene richtungsweisende *N*-(3-C₁₋₁₀-Alkoxypyridin-2-yl)- oder *N*-(3-C₁₋₁₀-Alkoxymethylpyridin-2-yl)-Gruppe gemäß der Formel (IIc) bzw. (IId) ist, oder ein Tautomer, Isomer, Salz, Hydrat oder Solvat davon; 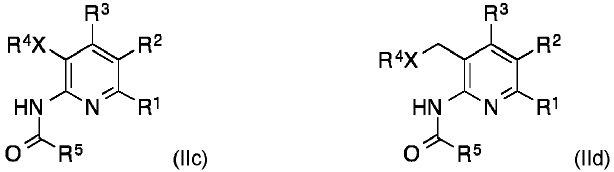 wobei
X O, NR⁶ oder S ist;
R¹, R² und R³ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₂₅-Alkinyl, Trifluormethyl, Cyano, Nitro, Halo, Thio, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester C₁₋₁₀-Alkyl-Arylamino, C₁₋₁₀-Alkyl-Heteroarylamino, Diheteroarylamino, Diarylamino, Aryl-Heteroarylamino, Arylthio, Heteroarylthio; wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl Aryloxy, Arylcarbonyl, Arylamino, Heteroarylamino, C₁₋₁₀-Alkyl-Arylamino, C₁₋₁₀ -Alkyl-Heteroarylamino, Diarylamino, Diheteroarylamino, Aryl-Heteroarylamino, Arylthio, Heteroarylthio, Aryloxy, Heteroaryloxy;
R⁴ und R⁶ jeweils unabhängig voneinander ausgewählt sind aus der Liste, umfassend: Wasserstoff, C₁₋₁₀-Alkyl, C₁₋₂₅-Alkenyl, C₁₋₂₅-Alkinyl, Aryl, Heteroaryl, Arylalkyl, Heteroarylalkyl, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀-Alkyl-Heteroarylamino, Diheteroarylamino, Diarylamino, Aryl-Heteroarylamino, Arylthio, Heteroarylthio, Aryloxy, Heteroaryloxy;
R⁵ Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Diarylamino, Diheteroarylamino, Heteroaryl-Arylamino, Arylthio, Heteroarylthio, Aryloxy, Heteroaryloxy, Hydroxyl, Amino, Thio oder ein pharmazeutisch akzeptables Salz davon oder ein Enantiomer oder eine stereoisomere Form davon oder ein Solvat davon ist, wobei das Aryl, Heteroaryl, Alkoxy oder Alkyl wahlweise mit einem bis zehn Substituenten substituiert ist, unabhängig ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl.

8. Verwendung einer Verbindung der allgemeinen Struktur *N*-HBDG-Amid in einem Verfahren nach einem der Patentansprüche 1 bis 4, wobei das HBDG eine an das *N*-Atom des Amids gebundene richtungsweisende C₁₋₁₀-Alkylnicotinat-Gruppe gemäß Formel (IIe) ist, wobei R⁴ vorzugsweise ein Alkylsubstituent ist und vorzugsweise eine *t*Bu-Gruppe wie in Formel (IIf) ist; oder ein Tautomer, Isomer, Salz, Hydrat oder Solvat davon; 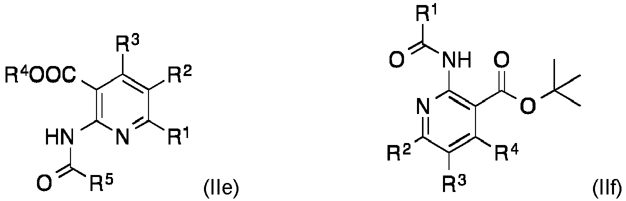 wobei
R¹, R² und R³ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Nitro, Halo, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio; wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀ -Alkyl-Diarylamino, Diheteroarylamino, Aryl-Heteroarylamino, Arylthio, Heteroarylthio, Aryloxy, Heteroaryloxy;
R⁴ ein Wasserstoff, C₁₋₁₀-Alkyl, C₁₋₁₀-Alkenyl, C₁₋₁₀-Alkinyl, Aryl, Heteroaryl, Arylalkyl, Heteroarylalkyl ist, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀ -Alkyl, Trifluormethyl, C₁₋₁₀ -Alkoxy, C₁₋₁₀ -Alkoxycarbonyl, Trifluormethoxy, C₁₋₁₀ - Alkylthio, Cyano, C₁₋₁₀ -Alkylamino, Nitro, Hydroxyl, Carbonyl, Amino, Oxim, Imino, Azido, Hydrazin, Acyl, C₁₋₁₀ -Alkylamino, C₁₋₁₀ -Dialkylamino, Carbonsäure, Acylamino, C₁₋₁₀ -Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio, Aryloxy oder Heteroaryloxy;
R⁵ Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio, Aryloxy, Heteroaryloxy, Hydroxyl, Amino, Thio oder ein pharmazeutisch akzeptables Salz davon oder ein Enantiomer oder eine stereoisomere Form davon oder ein Solvat davon ist, wobei das Aryl, Heteroaryl, Alkoxy oder Alkyl wahlweise mit einem bis zehn Substituenten substituiert ist, unabhängig ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl.

9. Verbindung der allgemeinen Struktur *N*-HBDG-Amid, wobei das HBDG eine an das *N*-Atom des Amids gebundene, richtungsweisende *tert*-Butylnicotinat-Gruppe gemäß der Formel (IIf) ist, oder ein Tautomer, Isomer, Salz, Hydrat oder Solvat davon; 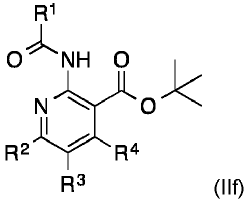 wobei
R¹ ein Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Arylthio, Heteroarylthio, Aryloxy, Heteroaryloxy Hydroxyl, Amino, Thio oder ein pharmazeutisch verträgliches Salz davon oder ein Enantiomer oder eine stereoisomere Form davon oder ein Solvat davon ist, wobei das Aryl, Heteroaryl, Alkoxy oder Alkyl wahlweise mit einem bis zehn Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Diarylamino, Heteroarylamino, Diheteroarylamino, Heteroaryl-Arylamino;
R², R³ und R⁴ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Thio, Nitro, Halo, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester C₁₋₁₀-Alkyl-Arylamino, C₁₋₁₀-Alkyl-Heteroarylamino, Diheteroarylamino, Diarylamino, Heteroaryl-Arylamino, Arylthio, Heteroarylthio; wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino;
wobei R² nicht ausgewählt ist aus 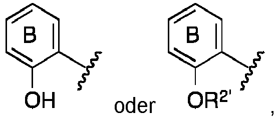 und wobei B einen Benzolring darstellt, der wahlweise einen weiteren Substituenten aufweist; und wobei R^{2'} ein Wasserstoffatom, eine Niederalkylgruppe mit 1 bis 6 Kohlenstoffen, die substituiert sein kann, eine Arylgruppe mit 6 bis 12 Kohlenstoffen, die substituiert sein kann, eine Heteroarylgruppe mit 4 bis 11 Kohlenstoffen, die substituiert sein kann, eine Arylalkylgruppe mit 7 bis 14 Kohlenstoffen, die substituiert sein kann, eine Heteroarylalkylgruppe mit 5 bis 13 Kohlenstoffen, die substituiert sein kann, oder eine Acylgruppe mit 2 bis 11 Kohlenstoffen aufweist; und
wobei die Verbindung nicht N-(3-tert-Butoxycarbonylpyridin-2-yl)-5-(5,6-Dimethoxy-3-methyl-1,4-benzoguinon-2-yl)methyl-2-acetoxybenzamid ist

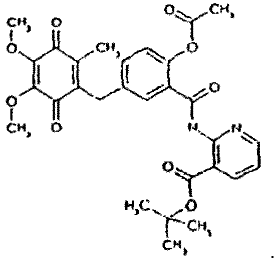

10. Verwendung einer Verbindung der allgemeinen Formel (IIg) in einem Verfahren nach einem der Patentansprüche 1 bis 4; 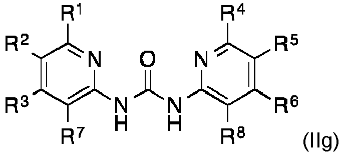 wobei
R¹, R², R³, R⁴, R⁵, R⁶, R⁷ und R⁸ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Thio, Nitro, Halo, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester C₁₋₁₀-Alkyl-Arylamino, C₁₋₁₀-Alkyl-Heteroarylamino, Diheteroarylamino, Diarylamino, Aryl-Heteroarylamino, Arylthio, Heteroarylthio; wobei das Aryl, Heteroaryl oder Alkyl wahlweise mit einem bis zehn Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino.

11. Verbindung der Formel (IIj) (wobei R⁷X vorzugsweise O-C₁₋₁₀-Alkyl ist oder die Verbindung die Struktur gemäß Formel (IIk) aufweist), oder ein Tautomer, Isomer, Salz, Hydrat oder Solvat davon; 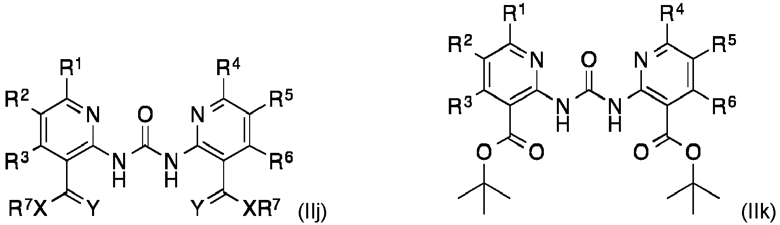 wobei
R¹, R², R³, R⁴, R⁵ und R⁶ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Thio, Nitro, Halo, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester, C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio; wobei das Aryl, Heteroaryl oder Alkyl wahlweise mit einem bis zehn Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino;
X = O, NR⁸, oder S;
R⁷ = Wasserstoff, Aryl, Heteroaryl, Heteroarylalkyl, Arylalkyl, C₁₋₁₀-Alkyl, C₁₋₁₀-Alkenyl, C₁₋₁₀-Alkinyl ist; wobei das Aryl, Heteroaryl oder Alkyl wahlweise mit einem bis zehn Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino;
R⁸ = Wasserstoff, Aryl, Heteroaryl, Heteroarylalkyl, Arylalkyl, C₁₋₁₀-Alkyl, C₁₋₁₀-Alkenyl, C₁₋₁₀-Alkinyl ist; wobei das Aryl, Heteroaryl oder Alkyl wahlweise mit einem bis zehn Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino;
Y O, S oder NR⁹ ist;
R⁹ = Wasserstoff, Aryl, Heteroaryl, Arylalkyl, Heteroarylalkyl, C₁₋₁₀-Alkyl, Sulfinyl, Sulfonyl ist, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino.

12. Verwendung eines *N*-ACDG-Amids in einem Verfahren nach einem der Patentansprüche 1 bis 4, wobei das ACDG eine an das *N*-Atom eines Amids der Formel (IIIa) gebundene richtungsweisende Imidazol-2-yl-Gruppe ist, oder ein Tautomer, Isomer, Salz, Hydrat oder Solvat davon; 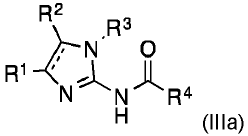 wobei
R¹ und R² jeweils unabhängig voneinander ausgewählt sind aus der Liste, umfassend: Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Trifluormethyl, Cyano, Nitro, Halo, Thio, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino, C₁₋₁₀-Alkoxycarbonyl, C₁₋₁₀-Alkylester, C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio; wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkylheteroarylamino, Arylthio, Heteroarylthio, Aryloxy, Heteroaryloxy, Diarylamino, Diheteroarylamino, Arylheteroarylamino;
R³ ausgewählt ist aus der Liste umfassend Wasserstoff, Aryl, Heteroaryl, Heteroarylalkyl, Arylalkyl, C₁₋₂₅-Alkyl, C₁₋₁₀-Alkenyl, C₁₋₂₅-Alkinyl, Arylalkyl, Heteroarylalkyl oder; R³ vorzugsweise XR⁵ ist, wobei X - C=O-, -C=N-R⁶ oder -C=S- ist;
wobei R⁵ Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, C₁₋₁₀-Alkoxy, C₁₋₁₀-Alkylthio, C₁₋₁₀-Alkylamino, C₁₋₁₀-Dialkylamino, C₁₋₁₀-Alkylarylamino, C₁₋₁₀-Alkylheteroarylamino, Diarylamino, Diheteroarylamino, Arylheteroarylamino, Arylamino, Heteroarylamino, Arylthio, Heteroarylthio, Aryloxy, Heteroaryloxy, Thio, Amino oder Hydroxyl ist; wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Arylamino, Heteroarylamino Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl;
R⁶ Wasserstoff, Aryl, Heteroaryl, Arylalkyl, Heteroarylalkyl, C₁₋₁₀-Alkyl, Sulfinyl, Sulfonyl ist, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino.
R⁴ ein Wasserstoff, Aryl, Heteroaryl, Heteroarylalkyl, Arylalkyl, C₁₋₂₅-Alkyl, C₁₋₂₅-Alkenyl, C₁₋₂₅-Alkinyl, Arylamino, Heteroarylamino, C₁₋₁₀-Alkoxy, C₁₋₁₀-Alkylthio, C₁₋₁₀-Alkylamino, C₁₋₁₀-Dialkylamino, C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio, Aryloxy, Heteroaryloxy, Hydroxyl, Amino, Thio oder ein pharmazeutisch akzeptables Salz davon; oder ein Enantiomer oder eine stereoisomere Form davon oder ein Solvat davon ist, wobei das Aryl, Heteroaryl, Alkoxy oder Alkyl wahlweise mit einem bis zehn Substituenten substituiert ist, unabhängig ausgewählt aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino;
Zusätzlich ist R³ am meisten bevorzugt eine COOR⁸-Gruppe, wobei R⁸ ausgewählt ist aus der Liste umfassend Wasserstoff, C₁₋₁₀-Alkyl, C₁₋₁₀-Alkenyl, C₁₋₁₀-Alkinyl, Aryl, Heteroaryl, Arylalkyl, Heteroarylalkyl, wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkyl-Arylamino, C₁₋₁₀-Alkyl-Heteroarylamino, Arylthio, Heteroarylthio, Aryloxy oder Heteroaryloxy, Diarylamino, Diheteroarylamino, Aryl-Heteroarylamino; und R⁸ vorzugsweise eine C₁₋₁₀-Alkylgruppe ist, die vorzugsweise mit einer *t*Bu-Gruppe beginnt.

13. Verbindung gemäß der Formel (IIId) oder ein Tautomer, Isomer, Salz, Hydrat oder Solvat davon; 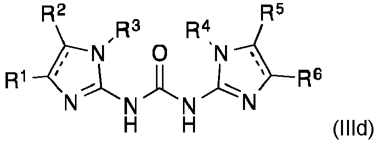 wobei
R¹, R², R⁵ und R⁶ jeweils unabhängig voneinander ausgewählt sind aus der Liste, umfassend: Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, Trifluormethyl, Cyano, Thio, Nitro, Halo, Hydroxyl, Amino, Acylamino, C₁₋₁₀-Alkoxy, Aryloxy, Heteroaryloxy, C₁₋₁₀-Alkylthio, C₁₋₁₀-Alkylamino, C₁₋₁₀-Dialkylamino C₁₋₁₀-Alkoxycarbonyl, C₁₋₁₀-Alkylester C₁₋₁₀-Alkyl-Arylamino, C₁₋₁₀-Alkyl-Heteroarylamino, Diarylamino, Diheteroarylamino, Aryl-Heteroarylamino, Arylthio, Heteroarylthio; wobei das Aryl, Heteroaryl oder Alkyl wahlweise mit einem bis zehn Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino, C₁₋₁₀-Alkylarylamino, C₁₋₁₀-Alkyl-Heteroaryl-Amino, Diarylamino, Diheteroarylamino, Aryl-Heteroarylamino, Arylthio, Heteroarylthio, Aryloxy, Heteroaryloxy;
R³ und R⁴ jeweils unabhängig voneinander ausgewählt sind aus der Liste umfassend Wasserstoff, Aryl, Heteroaryl, Heteroarylalkyl, Arylalkyl, C₁₋₂₅-Alkyl, C₁₋₁₀-Alkenyl, C₁₋₂₅-Alkinyl, Arylalkyl, Heteroarylalkyl oder R³ und/oder R⁴ XR⁷ ist, wobei X -C=O-, -C=N-R⁸, -C=S- ist;
R⁷ Wasserstoff, Aryl, Heteroaryl, Heteroarylalkyl, Arylalkyl, C₁₋₁₀-Alkyl, C₁₋₁₀-Alkenyl, C₁₋₁₀-Alkinyl, C₁₋₁₀-Alkoxy, C₁₋₁₀-Alkylthio, C₁₋₁₀-Alkylamino, C₁₋₁₀-Dialkylamino, C₁₋₁₀-Alkylarylamino, C₁₋₁₀-Alkylheteroarylamino, Diarylamino, Diheteroarylamino, Arylheteroarylamino, Arylamino, Heteroarylamino, Arylthio, Heteroarylthio, Aryloxy, Heteroaryloxy, Thio, Amino oder Hydroxyl ist; wobei das Aryl, Heteroaryl oder Alkyl wahlweise mit einem bis fünf Substituenten substituiert ist, die unabhängig ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Arylamino, Heteroarylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl;
R⁸ Wasserstoff, Aryl, Heteroaryl, C₁₋₁₀-Alkyl, Sulfinyl, Sulfonyl ist, wobei das Aryl, Heteroaryl oder Alkyl wahlweise mit einem bis fünf Substituenten substituiert ist, die unabhängig voneinander ausgewählt sind aus der Gruppe bestehend aus Halogen, Aryl, Heteroaryl, C₁₋₁₀-Alkyl, Trifluormethyl, C₁₋₁₀-Alkoxy, C₁₋₁₀-Alkoxycarbonyl, Trifluormethoxy, C₁₋₁₀-Alkylthio, Cyano, C₁₋₁₀-Alkylamino, Nitro, Hydroxyl, Carbonyl, Amino, Oxim, Imino, Azido, Hydrazin, Acyl, C₁₋₁₀-Alkylamino, C₁₋₁₀-Dialkylamino, Carbonsäure, Acylamino, C₁₋₁₀-Alkylester, Carbamat, Thioamido, Harnstoff, Sulfonamido, Alkylsulfoxid, Halogenalkyl, Alkenyl, Alkinyl, Arylalkyl, Heteroarylalkyl, Alkylcarbonylaminoalkyl, Aryloxy, Arylcarbonyl, Arylamino, Heteroarylamino;
R³ und R⁴ nicht beide Wasserstoff sind.

14. Verwendung des Verfahrens nach einem der Patentansprüche 1 bis 5 für die Synthese einer Verbindung, ausgewählt aus der Liste, umfassend: amidhaltige Verbindungen, Ester, Thioester, Carbonate, Thiocarbonate, Thiocarbamate, Polycarbonate, Polyharnstoff und Polycarbamate.

## Revendications

1. Procédé pour le clivage catalytique de composés primaires contenant un amide ; ledit procédé comprenant les étapes de :
- fourniture d'un composé primaire contenant de l'amide (H₂N-CO-) ;
- conversion dudit composé en un composé contenant un ACDG (contenant un groupe de direction à clivage d'amide), en introduisant un groupe de direction à clivage d'amide (ACDG), selon la formule (la), sur l'atome d'azote dudit amide, entraînant la formation d'un amide N-ACDG de formule ACDG-NH-CO- ; 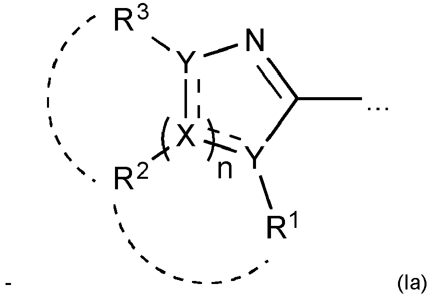 dans laquelle
X est sélectionné parmi C, N, O ou S.
Y est sélectionné parmi C, N ou S ; ou Y = O ou S et R¹ est absent n est sélectionné parmi 1, 2, 3, 4, 5, 6, 7 ou 8.
R¹, R² et R³ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₂₅, un trifluorométhyle, un cyano, un nitro, un halo, un thio, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, alkyle en C₁₋₁₀ hétéroaryle amino, un dihétéroarylamino, un diarylamino, un aryle hétéroarylamino, un arylthio, un hétéroarylthio ; dans lequel chacun desdits aryle, hétéroaryle ou alkyle est optionnellement et indépendamment substitué par 1 à 5 substituants sélectionnés à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio, un aryloxy, un hétéroaryloxy ;
- - - est une liaison chimique optionnelle ; si une ou plusieurs liaisons chimiques optionnelles sont présentes, on entend une structure polycyclique ;
- clivage dudit composé contenant un ACDG (à savoir l'amide *N*-ACDG) en présence d'un (hétéro)nucléophile ; et d'un catalyseur métallique selon la formule Ib, ou d'un sel, d'un solvate ou d'un hydrate de celui-ci
M_{y}Lₓ(Ib)
dans laquelle
M est un métal sélectionné parmi Zn, Mn, Ni ou Co ; de préférence Zn ;
L représente un ligand de carboxylate, de préférence un ligand d'acétate ;
x est sélectionné entre 0 et 30 ; et
y est sélectionné parmi 1, 2, 3 ou 4 ; de préférence 1 ;
ledit amide *N*-ACDG est le composé ayant la structure ACDG-NH-CO-, et dans le cadre de notre définition d'amide *N*-ACDG, nous incluons des groupes dans lesquels l'unité structurelle ACDG-NHCO- fait partie d'un groupe ou d'une entité structurelle plus large, à savoir : urée, oxamide, carbamate, thiocarbamate ;
ledit (hétéro)nucléophile peut être inclus dans ledit composé contenant un ACDG.

2. Procédé pour le clivage catalytique de composés contenant un amide ; ledit procédé comprenant les étapes de :
- fourniture d'un précurseur d'ACDG sélectionné dans la liste comprenant un composé contenant un amide, LG-CO-LG (LG étant un groupe sortant), CO₂ et CO ;
- conversion dudit composé précurseur d'ACDG en un composé contenant un ACDG (contenant un groupe de direction à clivage d'amide), en introduisant un groupe de direction à clivage d'amide (ACDG), selon la formule (la), entraînant la formation d'un amide N-ACDG de formule ACDG-NH-CO-, et ce par la réaction dudit précurseur d'ACDG avec un composé introductif d'ACDG en présence ou absence de catalyseur(s), ligand(s), réactant(s), réactif(s) et/ou solvant(s) à un composé contenant un ACDG (contenant un groupe de direction à clivage d'amide) de la formule ACDG-NH-CO- 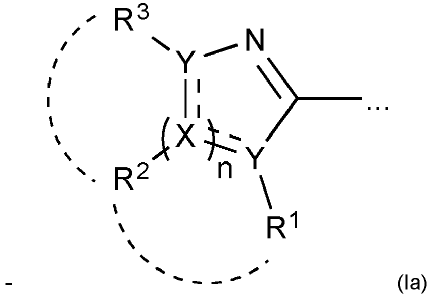 dans laquelle
X est sélectionné parmi C, N, O ou S.
Y est sélectionné parmi C, N ou S ; ou Y = O ou S et R¹ est absent
n est sélectionné parmi 1, 2, 3, 4, 5, 6, 7 ou 8.
R¹, R² et R³ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₂₅, un trifluorométhyle, un cyano, un nitro, un halo, un thio, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, alkyle en C₁₋₁₀ hétéroaryle amino, un dihétéroarylamino, un diarylamino, un aryle hétéroarylamino, un arylthio, un hétéroarylthio ; dans lequel chacun desdits aryle, hétéroaryle ou alkyle est optionnellement et indépendamment substitué par 1 à 5 substituants sélectionnés à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio, un aryloxy, un hétéroaryloxy ;
- - - est une liaison chimique optionnelle ; si une ou plusieurs liaisons chimiques optionnelles sont présentes, on entend une structure polycyclique ;
- clivage dudit composé contenant un ACDG (à savoir l'amide *N*-ACDG) en présence d'un (hétéro)nucléophile ; et d'un catalyseur métallique selon la formule Ib, ou d'un sel, d'un solvate ou d'un hydrate de celui-ci
M_{y}Lₓ(Ib)
dans laquelle
M est sélectionné parmi Zn, Mn, Ni ou Co ; de préférence Zn ;
L représente un ligand de carboxylate, de préférence un ligand d'acétate ;
x est sélectionné entre 0 et 30 ; et
y est sélectionné parmi 1, 2, 3 ou 4 ; de préférence 1 ;
ledit amide *N*-ACDG est le composé ayant la structure ACDG-NH-CO-, et dans le cadre de notre définition d'amide *N*-ACDG, nous incluons des groupes dans lesquels l'unité structurelle ACDG-NHCO- fait partie d'un groupe ou d'une entité structurelle plus large, à savoir : urée, oxamide, carbamate, thiocarbamate ;
ledit (hétéro)nucléophile peut être inclus dans ledit composé contenant un ACDG.

3. Procédé selon l'une quelconque des revendications 1 ou 2, dans lequel ledit groupe de direction à clivage d'amide (ACDG) est un groupe de direction à clivage d'amide à base de liaison hydrogène (HBDG).

4. Procédé selon l'une quelconque des revendications 1 à 3, dans lequel ledit amide *N*-ACDG est un amide *Nⁿ*-ACDGₙ qui est clivé 1 à n fois en présence d'un catalyseur métallique et d'un hétéronucléophile, après les n introductions de l'ACDG dans 1 à maximum n étapes ; pour le nombre maximal défini de n étapes de clivage, le nombre maximal de n différents hétéronucléophiles peut être utilisé, avec le nombre minimal d'hétéronucléophiles étant 1 ; Ledit amide *Nⁿ*-ACDGₙ est un composé ayant une structure dans laquelle le motif structurel ACDG-NH-CO- peut être trouvé n fois ; Lors de l'identification de la somme du nombre de motifs structurels ACDG-NH-CO, une fraction -CO- peut être utilisée une ou deux fois pour identifier ce motif ; ledit amide *Nⁿ*-ACDGₙ est de préférence un amide *Nⁿ*-HBDGₙ ; ledit amide *Nⁿ*-HBDGₙ est un composé ayant une structure dans laquelle le motif structurel HBDG-NH-CO- peut être trouvé n fois ; Lors de l'identification de la somme du nombre de motifs structurels HBDG-NH-CO, une fraction -CO- peut être utilisée une ou deux fois pour identifier ce motif ; En outre, lorsque ledit amide *Nⁿ*-ACDGₙ est un amide *N,N'*ACDG₂, l'amide *N,N'*-ACDG₂ a de préférence la structure générale HBDG-NH-CO-X-CO-NH-HBDG, dans laquelle X peut consister en tout groupe fonctionnel ou combinaisons de groupes fonctionnels ; en outre, dans tous les cas précédents, lesdits n hétéronucléophiles (si n = 1 ou 2) peuvent faire partie de l'amide *Nⁿ*-ACDGₙ, entraînant ainsi une réaction de clivage intramoléculaire ; en outre si n = 2, ledit *Nⁿ*-ACDGₙ est un amide *N,N'*-ACDG₂ qui a de préférence la structure générale ACDG-NH-CO-NH-ACDG et qui peut subir un clivage d'amide intermoléculaire intelligent, puisque l'activation de catalyseur métallique est nécessaire pour la réaction avec un hétéronucléophile de préférence non inclus dans la fraction *N,N'-*ACDG₂, et puisque, par une régulation de température intelligente, le nombre de clivages (1 ou 2) dans une étape de réaction peut être régulé, permettant ainsi l'utilisation de deux hétéronucléophiles différents, de préférence non inclus dans la fraction *N,N'-*ACDG₂, pour deux réactions de clivage ultérieures par un protocole à deux étapes ; dans tous les cas précédents, ledit ACDG est de préférence un HBDG qui est de préférence une fraction pyridin-2-yle substituée en C3, de préférence un nicotinate d'alkyle (comme un nicotinate tert-butyle) ou une fraction pyridin-2-yle 3-alcoxy ; ou une fraction imidazol-2-yle qui est un HBDG, de préférence étant un alkyle 1*H*-imidazole-1-carboxylate (comme un *tert*-butyle 1*H*-imidazole-1-carboxylate).

5. Procédé selon l'une quelconque des revendications 1 à 4 dans lequel l'amide *N*-ACDG de direction est sélectionné dans la liste comprenant :
- un *N*-(pyridin-2-yl)amide, un *N*-(n-alcoxypyridine en C1-10-2-yl) amide, avec n = de 3 à 6 et l'alcoxy étant optionnellement substitué ;
- un *N*-(n-halopyridin-2-yl)amide ; avec n = de 3 à 6 ;
- un N-amidonicotinate d'alkyle en C1-10, n = 2 ou 6 et l'alkyle étant optionnellement substitué ;
- un *N*-(n-nitropyridin-2-yl)amide, avec n = de 3 à 6 ;
- un *N*-(n-cyanopyridine-2-yl)amide, avec n = de 3 à 6 ;
- un *N*-(n-trifluorométhylpyridin-2-yl)amide, avec n = de 3 à 6 ;
- un *N*-(n-alkylpyridine en C1-10-2-yl)amide, avec n = de 3 à 6 et l'alkyle étant optionnellement substitué ;
- un *N*-(n-arylpyridin-2-yl)amide, avec n = de 3 à 6 et l'aryle étant optionnellement substitué ;
- un *N*-(n-hétéroarylpyridin-2-yl)amide, avec n = de 3 à 6 et l'hétéroaryle étant optionnellement substitué ;
- un *N*-(n-(alcoxyméthyle en C1-10)pyridin-2-yl)amide, avec n = de 3 à 6 et l'alcoxyméthyle en C₁₋₁₀ étant optionnellement substitué ;
- un *N*-(n-(di-alkylamino en C1-10)pyridin-2-yl)amide, avec n = de 3 à 6 et l'alkyle étant optionnellement substitué ;
- un *N*-(n-(amino alkyle en C1-10)pyridin-2-yl)amide, avec n = de 3 à 6 et l'alkyle étant optionnellement substitué ;
- un S-alkyle en C₁₋₁₀ 2-benzamidopyridine-3-carbothioate, avec l'alkyle étant optionnellement substitué ;
- un *N*-(n-(alkylthio en C₁₋₁₀ alkyle en C1-10)pyridin-2-yl)amide, avec n = de 3 à 6 et l'alkyle étant optionnellement substitué ;
- un 2-amido-*N*-alkylnicotinamide en C1-10, avec l'alkyle étant optionnellement substitué ;
- un *N*-(n-alkylcarbonylpyridine en C1-10-2-yl)amide, avec n = de 3 à 6 et l'alkyle étant optionnellement substitué ;
- un *N*-(1-oxo-3,4-dihydro-1*H*-pyrano[3,4-c]pyridin-8-yl)amide ;
- un *N*-(8-oxo-5,6,7,8-tétrahydroisoquinoléine-1-yl)amide ;
- un *N*-(8-oxo-5,6,7,8-tétrahydro-2,7-naphthyridin-1-yl)amide ;
- un *N*-(1-oxo-3,4-dihydro-1*H*-thiopyrano[3,4-*c*]pyridin-8-yl)amide ;
- un *N*-(3,4-dihydro-2*H*-pyrano[2,3-*c*]pyridin-8-yl)amide ;
- un *N*-(7-oxo-6,7-dihydro-5*H*-cyclopenta[*c*]pyridin-1-yl)amide ;
- un *N*-(3-oxo-1,3-dihydrofuro[3,4-*c*]pyridin-4-yl)amide ;
- un *N*-(3-oxo-1,3-dihydrothiéno[3,4-*c*]pyridin-4-yl)amide ;
- un *N*-(3-oxo-2,3-dihydro-1*H*-pyrrolo[3,4-*c*]pyridin-4-yl)amide ;
- un *N*-(2,3-dihydrofuro[2,3-*c*]pyridin-7-yl)amide ;
dans lequel dans tous les cas, « amide » est une fraction de la structure générale selon la formule (Ib) ; 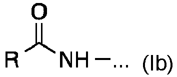 où R est sélectionné dans la liste comprenant un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle aryle amino en C₁₋₁₀, un alkyle en C₁₋₁₀ hétéroaryle amino, un hétéroaryle arylamino, un dihétéroarylamino, un diarylamino, un hétéroaryle arylamino, un arylthio, un hétéroarylthio, un aryloxy, un hétéroaryloxy, un hydroxyle, un amino, un thio ou un sel pharmaceutiquement acceptable de ceux-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, ou un hétéroarylamino.

6. Utilisation d'un amide *N*-ACDG dans un procédé selon l'une quelconque des revendications 1-4 ; dans laquelle l'ACDG est un groupe de direction pyridin-2-yle attaché à l'atome *N* d'un amide selon la formule (IIa), ou un tautomère, isomère, sel, hydrate ou solvate de celui-ci ; 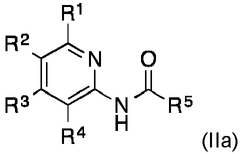 dans laquelle
R¹, R² et R³ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un nitro, un halo, un hydroxyle, un amino, un thio, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle aryle amino en C₁₋₁₀, alkyle hétéroaryle amino en C₁₋₁₀, un arylthio, un hétéroarylthio ; dans lequel ledit aryle, hétéroaryle ou alkyle est optionnellement substitué par un à cinq substituants indépendamment sélectionnés à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle aryle amino en C₁₋₁₀, un alkyle hétéroaryle amino en C₁₋₁₀, un arylthio, un hétéroarylthio, un aryloxy, un hétéroaryloxy ;
R⁴ est un hydrogène, un halo, un nitro, un cyano, un trifluorométhyle, un aryle, un hétéroaryle, un alkyle en C₁₋₁₀, un alcényle en C₁₋₁₀, un alcynyle en C₁₋₁₀, un arylalkyle ou un hétéroarylalkyle ;
ou R⁴ est XR⁶ où X est -S-, -O-, -N-, -NH-, -C=O-, -C=N-R⁷ ou -C=S-,
R⁶ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un arylamino, un hétéroarylamino, un arylthio, un hétéroarylthio, un aryloxy, un hétéroaryloxy, un thio, un amino ou un hydroxyle ; dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un arylamino, un hétéroarylamino, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy ou un arylcarbonyle ;
R⁷ est un hydrogène, un aryle, un hétéroaryle, un alkyle en C₁₋₁₀, un sulfinyle, un sulfonyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino ou un hétéroarylamino ;
R⁵ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle en C₁₋₁₀aryle amino, un alkyle en C₁₋₁₀ hétéroaryle amino, un diarylamino, un dihétéroarylamino, un hétéroaryle aryle amino, un arylthio, un hétéroarylthio, un aryloxy, un hétéroaryloxy, un hydroxyle, un amino, un thio ou un sel pharmaceutiquement acceptable de ceux-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par n halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino ou un hétéroarylamino.

7. Utilisation d'un composé de l'amide de la structure générale *N*-HBDG dans un procédé selon l'une quelconque des revendications 1 à 4 ; dans laquelle le HBDG est un groupe de direction *N*-(3-alcoxy en C₁₋₁₀ pyridin-2-yle) ou *N*-(3-alcoxyméthyle en C₁₋₁₀ pyridin-2-yle) attaché à l'atome *N*dudit amide selon la formule respective (IIc) ou (IId), ou un tautomère, isomère, sel, hydrate ou solvate de celui-ci ; 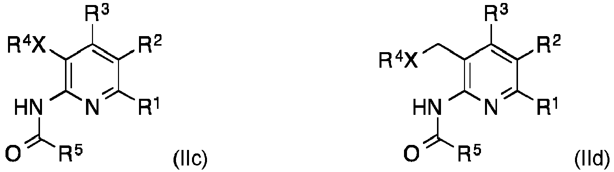 dans laquelle
X est O, NR⁶, ou S ;
R¹, R² et R³ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₂₅, un trifluorométhyle, un cyano, un nitro, un halo, un thio, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle aryle amino en C₁₋₁₀, alkyle hétéroaryle amino en C₁₋₁₀, un dihétéroarylamino, un diarylamino, un aryle hétéroarylamino, un arylthio, un hétéroarylthio ; dans lequel ledit aryle, hétéroaryle ou alkyle est optionnellement substitué par un à cinq substituants sélectionnés indépendamment à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle aryle amino en C₁₋₁₀, un alkyle hétéroaryle amino en C₁₋₁₀, un diarylamino, un dihétéroarylamino, un aryle hétéroarylamino, un arylthio, un hétéroarylthio, un aryloxy, un hétéroaryloxy ;
R⁴ et R⁶ sont chacun indépendamment sélectionnés dans la liste comprenant : un hydrogène, un alkyle en C₁₋₁₀, un alcényle en C₁₋₂₅, un alcynyle en C₁₋₂₅, un aryle, un hétéroaryle, un arylalkyle, un hétéroarylalkyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un dihétéroarylamino, un diarylamino, un aryle hétéroarylamino, un arylthio, un hétéroarylthio, un aryloxy, un hétéroaryloxy ;
R⁵ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un diarylamino, un dihétéroarylamino, un hétéroaryle aryle amino, un arylthio, un hétéroarylthio, un aryloxy, un hétéroaryloxy, un hydroxyle, un amino, un thio ou un sel pharmaceutiquement acceptable de ceux-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle.

8. Utilisation d'un composé de l'amide de la structure générale *N*-HBDG dans un procédé selon l'une quelconque des revendications 1 à 4 dans laquelle le HBDG est un groupe de direction de nicotinate d'alkyle en C₁₋₁₀ attaché à l'atome *N* dudit amide selon la formule (IIe) avec R⁴ étant de préférence un substituant d'alkyle et étant de préférence un groupe *t*Bu comme dans la formule (IIf) ; ou un tautomère, isomère, sel, hydrate ou solvate de celui-ci ; 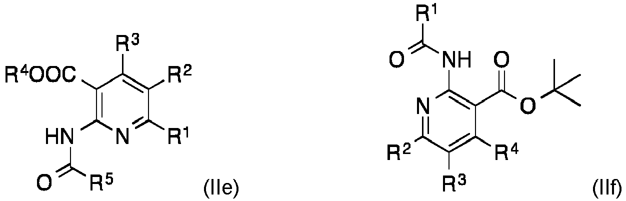 dans laquelle
R¹, R² et R³ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un nitro, un halo, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, alkyle en C₁₋₁₀ hétéroaryle amino, un diarylamino, un dihétéroarylamino, un aryle hétéroarylamino, un arylthio, un hétéroarylthio ; dans lequel ledit aryle, hétéroaryle ou alkyle est optionnellement substitué par un à cinq substituants sélectionnés indépendamment à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀, un diarylamino, un dihétéroarylamino, un aryle hétéroarylamino, un arylthio, un hétéroarylthio, un aryloxy, un hétéroaryloxy ;
R⁴ est un hydrogène, un alkyle en C₁₋₁₀, un alcényle en C₁₋₁₀, un alcynyle en C₁₋₂₅, un aryle, un hétéroaryle, un arylalkyle, un hétéroarylalkyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroarylamino, un diarylamino, un dihétéroarylamino, un aryle hétéroarylamino, un arylthio, un hétéroarylthio, un aryloxy ou un hétéroaryloxy ;
R⁵ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio, un aryloxy, un hétéroaryloxy, un hydroxyle, un amino, un thio ou un sel pharmaceutiquement acceptable de ceux-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle.

9. Composé de l'amine de la structure générale *N*-HBDG dans lequel le HBDG est un groupe de direction de nicotinate *tert*-butyle attaché à l'atome N dudit amide selon la formule (IIf), ou un tautomère, isomère, sel, hydrate ou solvate de celui-ci ; 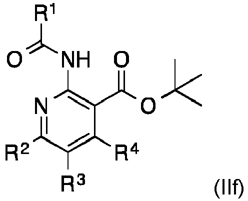 dans laquelle
R¹ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un arylthio, un hétéroarylthio, un aryloxy, un hétéroaryloxy, un hydroxyle, un amino, un thio ou un sel pharmaceutiquement acceptable de ceux-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un diarylamino, un hétéroarylamino, un dihétéroarylamino, un hétéroaryle aryle amino ; R², R³ et R⁴ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un thio, un nitro, un halo, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle aryle amino en C₁₋₁₀, alkyle hétéroaryle amino en C₁₋₁₀, un dihétéroarylamino, un diarylamino, un hétéroaryle hétéroamino, un arylthio, un hétéroarylthio ; dans lequel ledit aryle, hétéroaryle ou alkyle est optionnellement substitué par un à cinq substituants sélectionnés indépendamment à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle, un hétéroarylamino ;
où R² n'est pas sélectionné parmi 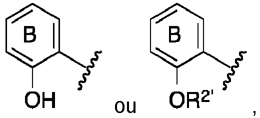 et où B représente un cycle benzène ayant optionnellement un autre substituant ; et dans lequel R²' représente un atome d'hydrogène, un groupe alkyle inférieur ayant de 1 à 6 atomes de carbone qui peut être substitué, un groupe aryle ayant de 6 à 12 atomes de carbone qui peut être substitué, un groupe hétéroaryle ayant de 4 à 11 atomes de carbone qui peut être substitué, un groupe arylalkyle ayant de 7 à 14 atomes de carbone qui peut être substitué, un groupe hétéroarylalkyle ayant de 5 à 13 atomes de carbone qui peut être substitué, ou un groupe acyle ayant de 2 à 11 atomes de carbone ; et
dans lequel ledit composé n'est pas N-(3-tert-Butoxycarbonylpyridin-2-yl)-5-(5,6-diméthoxy-3-méthyl-1,4-benzoguinon-2-yl)méthyl-2-acétoxybenzamide

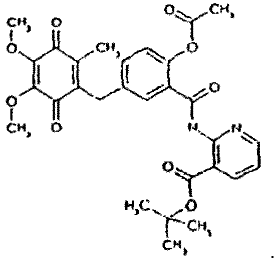

10. Utilisation d'un composé de la formule générale (IIg) dans un procédé selon l'une quelconque des revendications 1 à 4 ; 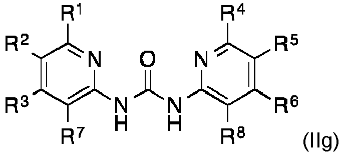 dans laquelle
R¹, R², R³, R⁴, R⁵, R⁶, R⁷ et R⁸ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un thio, un nitro, un halo, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, un alkyle en C₁₋₁₀ hétéroaryle amino, un dihétéroarylamino, un diarylamino, un aryle hétéroarylamino, un arylthio, un hétéroarylthio ; dans lequel ledit aryle, hétéroaryle ou alkyle est optionnellement substitué par un à dix substituants sélectionnés indépendamment à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle, un hétéroarylamino.

11. Composé de formule (IIj) (avec R⁷X étant de préférence un alkyle O-C₁₋₁₀ ou ledit composé ayant la structure selon la formule (IIk)), ou un tautomère, isomère, sel, hydrate ou solvate de celui-ci ; 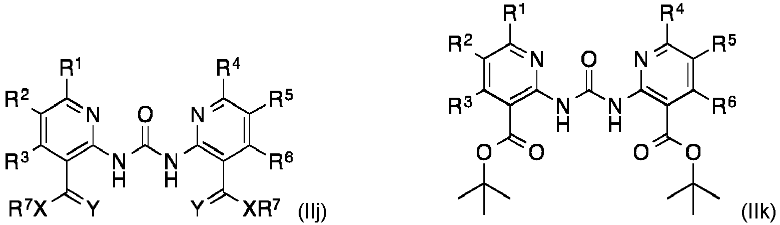 dans laquelle
R¹, R², R³, R⁴, R⁵ et R⁶ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un thio, un nitro, un halo, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio ; dans lequel ledit aryle, hétéroaryle ou alkyle est optionnellement substitué par un à dix substituants sélectionnés indépendamment à partir du groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle, un hétéroarylamino ;
X = O, NR⁸, ou S ;
R⁷ = un hydrogène, un aryle, un hétéroaryle, un hétéroarylalkyle, un arylalkyle, un alkyle en C₁₋₁₀, un alcényle en C₁₋₁₀, un alcynyle en C₁₋₁₀ ; dans lequel ledit aryle, hétéroaryle ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle hétéroarylamino ;
R⁸ = un hydrogène, un aryle, un hétéroaryle, un hétéroarylalkyle, un arylalkyle, un alkyle en C₁₋₁₀, un alcényle en C₁₋₁₀, un alcynyle en C₁₋₁₀ ; dans lequel ledit aryle, hétéroaryle ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle hétéroarylamino ;
Y est O, S ou NR⁹;
R⁹ = un hydrogène, un aryle, un hétéroaryle, un arylalkyle, un hétéroarylalkyle, un alkyle en C₁₋₁₀, un sulfinyle, un sulfonyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino.

12. Utilisation d'un amide *N*-ACDG dans un procédé selon l'une quelconque des revendications 1 à 4 ; dans laquelle l'ACDG est un groupe de direction imidazol-2-yle attaché à l'atome N d'un amide selon la formule (IIIa) ou un tautomère, isomère, sel, hydrate ou solvate de celui-ci ; 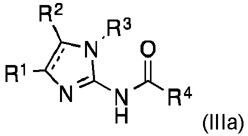 dans laquelle
R¹ et R² sont chacun indépendamment sélectionnés dans la liste comprenant : un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un trifluorométhyle, un cyano, un nitro, un halo, un thio, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio ; dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un arylthio, un hétéroarylthio, un aryloxy, un hétéroaryloxy, un diarylamino, un dihétéroarylamino, un aryle hétéroarylamino ;
R³ est sélectionné dans la liste comprenant un hydrogène, un aryle, un hétéroaryle, un hétéroarylalkyle, un arylalkyle, un alkyle en C₁₋₂₅, un alcényle en C₁₋₁₀, un alcynyle en C₁₋₂₅, un arylalkyle, un hétéroarylalkyle ou ; R³ est préférentiellement XR⁵ dans lequel X est -C=O-, -C=N-R⁶ ou -C=S- ;
R⁵ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylamino, un hétéroarylamino, un arylthio, un hétéroarylthio, un aryloxy, un hétéroaryloxy, un thio, un amino ou un hydroxyle ; dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un arylamino, un hétéroarylamino, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle ;
R⁶ est un hydrogène, un aryle, un hétéroaryle, un arylalkyle, un hétéroarylalkyle, un alkyle en C₁₋₁₀, un sulfinyle, un sulfonyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino.
R⁴ est un hydrogène, un aryle, un hétéroaryle, un hétéroarylalkyle, un arylalkyle, un alkyle en C₁₋₂₅, un alcényle en C₁₋₂₅, un alcynyle en C₁₋₂₅, un arylamino, un hétéroarylamino, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio, un aryloxy, un hétéroaryloxy, un hydroxyle, un amino, un thio ou un sel pharmaceutiquement acceptable de celui-ci, ou un énantiomère, ou une forme stéréoisomérique de celui-ci, ou un solvate de celui-ci, dans lequel ledit aryle, hétéroaryle, alcoxy ou alkyle est optionnellement substitué par un à dix substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino ;
En outre, R³ est le plus préférentiellement un groupe COOR⁸ avec R⁸ sélectionné dans la liste comprenant un hydrogène, un alkyle en C₁₋₁₀, un alcényle en C₁₋₁₀, un alcynyle en C₁₋₁₀, un aryle, un hétéroaryle, un arylalkyle, un hétéroarylalkyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀hétéroarylamino, un arylthio, un hétéroarylthio, un aryloxy ou un hétéroaryloxy, un diarylamino, un dihétéroarylamino, un aryle hétéroarylamino ; et R⁸ étant de préférence un groupe alkyle en C₁₋₁₀, de préférence commençant un groupe *t*Bu.

13. Composé selon la formule (IIId), ou tautomère, isomère, sel, hydrate ou solvate de celui-ci ; 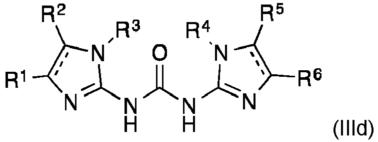 dans laquelle
R¹, R², R⁵ et R⁶ sont chacun sélectionnés dans la liste comprenant : un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un trifluorométhyle, un cyano, un thio, un nitro, un halo, un hydroxyle, un amino, un acylamino, un alcoxy en C₁₋₁₀, un aryloxy, un hétéroaryloxy, un alkylthio en C₁₋₁₀, un alkylamino en C₁₋₁₀, un dialkylamino en C₁₋₁₀, un alcoxycarbonyle en C₁₋₁₀, des esters d'alkyle en C₁₋₁₀, un alkyle en C₁₋₁₀ aryle amino, alkyle en C₁₋₁₀ hétéroaryle amino, un diarylamino, un dihétéroarylamino, un aryle hétéroarylamino, un arylthio, un hétéroarylthio ; dans lequel ledit aryle, hétéroaryle ou alkyle est optionnellement substitué par un à dix substituants sélectionnés indépendamment à partir du groupe composé par un halogène, un aryle, un hétéroaryle, un alkyle en C₁₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino, un alkyle en C₁₋₁₀ aryle amino, un alkyle en C₁₋₁₀ hétéroaryle amino, un diarylamino, un dihétéroarylamino, un aryle hétéroarylamino, un arylthio, un hétéroarylthio, un aryloxy, un hétéroaryloxy ;
R³ et R⁴ sont chacun indépendamment sélectionnés dans la liste comprenant un hydrogène, un aryle, un hétéroaryle, un hétéroarylalkyle, un arylalkyle, un alkyle en C₁₋₂₅, un alcényle en C₁₋₁₀, un alcynyle en C₁₋₂₅, un arylalkyle, un hétéroarylalkyle ou R³ et/ou R⁴ est XR⁷ dans lequel X est -C=O-, -C=N-R⁸ ou -C=S- ;
R⁷ est un hydrogène, un aryle, un hétéroaryle, un hétéroarylalkyle, un arylalkyle, un alkyle en C₁₋₁₀, un alcényle en C₁₋₁₀, un alcynyle en C₁₋₁₀, un alcoxy en C₁₋₁₀, un alkylthio en C₁₋₁₀, un alkylamino en C₁₋₁₀, un dialkylamino en C₁₋₁₀, un alkyle en C₁₋₁₀ aryle amino, un alkyle en C₁₋₁₀ hétéroaryle amino, un diarylamino, un dihétéroarylamino, un aryle hétéroarylamino, un arylamino, un hétéroarylamino, un arylthio, un hétéroarylthio, un aryloxy, un hétéroaryloxy, un thio, un amino ou un hydroxyle ; dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un arylamino, un hétéroarylamino, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle ;
R⁸ est un hydrogène, un aryle, un hétéroaryle, un alkyle en C₁₋₁₀, un sulfinyle, un sulfonyle, dans lequel ledit aryle, hétéroaryle ou alkyle est optionnellement substitué par un à cinq substituants sélectionnés indépendamment dans le groupe composé par un halogène, un aryle, un hétéroaryle, un alkyle en C₁₋₁₀, un trifluorométhyle, un alcoxy en C₁₋₁₀, un alcoxycarbonyle en C₁₋₁₀, un trifluorométhoxy, un alkylthio en C₁₋₁₀, un cyano, un alkylamino en C₁₋₁₀, un nitro, un hydroxyle, un carbonyle, un amino, une oxime, un imino, un azido, une hydrazine, un acyle, un alkylamino en C₁₋₁₀, un dialkylamino en C₁₋₁₀, un acide carboxylique, un acylamino, des esters d'alkyle en C₁₋₁₀, un carbamate, un thioamido, une urée, un sulfonamido, un alkylsulfoxyde, un haloalkyle, un alcényle, un alcynyle, un arylalkyle, un hétéroarylalkyle, un alkylcarbonylaminoalkyle, un aryloxy, un arylcarbonyle, un arylamino, un hétéroarylamino ;
R³ et R⁴ ne sont pas tous deux hydrogène.

14. Utilisation du procédé selon l'une quelconque des revendications 1 à 5 pour la synthèse d'un composé sélectionné dans la liste comprenant : des composés contenant un amide, des esters, des thioesters, des carbonates, des thiocarbonates, des thiocarbamates, des polycarbonates, une polyurée et des polycarbamates.
